(19) Europäisches Patentamt
European Patent Office
Office européen des brevets

(11) Publication number: 0 395 417 B1

# (12) EUROPEAN PATENT SPECIFICATION

(45) Date of publication of patent specification: 08.02.95

(51) Int. Cl.[6]: C07K 14/655, A61K 38/31

(21) Application number: 90304551.6

(22) Date of filing: 26.04.90

## (54) Linear somatostatin analogues.

(30) Priority: 26.04.89 US 343325

(43) Date of publication of application:
31.10.90 Bulletin 90/44

(45) Publication of the grant of the patent:
08.02.95 Bulletin 95/06

(84) Designated Contracting States:
AT BE CH DE DK ES FR GB GR IT LI LU NL SE

(56) References cited:
EP-A- 0 030 920
EP-A- 0 187 622
GB-A- 2 206 352

PEPTIDES STRUCTURE AND FUNCTION, PROCEEDINGS OF THE NINTH AMERICAN PEPTIDESYMPOSIUM, Toronto, Ontario, June 1985, pages 627-630, Pierce Chemical Co., US;R.-Z. CAI et al.: "Synthesis and evaluation of activities of octapeptide

analogs of somatostatin"

(73) Proprietor: The Administrators of The Tulane Educational Fund
1430 Tulane Avenue
New Orleans
Louisiana 70112 (US)

(72) Inventor: Coy, David H.
4319 Perrier Street
New Orleans,
Louisiana 70115 (US)
Inventor: Murphy, William A.
107 North Magnolia Drive
Covington,
Louisiana 70433 (US)

(74) Representative: Sheard, Andrew Gregory et al
Kilburn & Strode
30, John Street
London WC1N 2DD (GB)

EP 0 395 417 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to therapeutic peptides.

A number of somatostatin analogues exhibiting Growth Hormone-releasing-inhibiting activity have been described in the literature, including analogues containing fewer than the naturally occurring fourteen amino acids. For example, Coy *et al.* U.S. Patent No. 4,485,101 describes dodecapeptides having an N-terminal acetyl group, a C-terminal $NH_2$, D-Trp at position 6 and p-Cl-Phe at position 4. (Herein, when no designation of configuration is given, the L-isomer is intended.)

EP-A-0187622 refers to somatostatin derivatives that have modified cysteine residues at positions 2 and 7.

EP-A-0203031 refers to somatostatin derivatives where the bridge C''-C' (from positions 2 to 7) is a Cys-Cys, Lys-Asp or Cys-Lys linkage.

Abbreviations: Nle = norleucine, Nal- naphthylalanine.

According to a first aspect of the present invention there is provided a compound, e.g. an octapeptide, (which is a linear somatostatin analogue) of the general formula:

$$\begin{array}{l} R^1 \\ | \\ A^1 - A^2 - A^3 - \text{D-Trp} - \text{Lys} - A^6 - A^7 - A^8 - R^3 \\ | \\ R^2 \end{array} \qquad (I)$$

wherein:

$A^1$ represents a D-isomer of Ala, pyridyl-Ala, Leu, Ile, Val, Met, Nle, Trp, beta-Nal, Phe, o-X-Phe (wherein X represents H, $CH_3$, Cl, Br, F, OH, $OCH_3$ or $NO_2$), p-X-Phe (wherein X represents H, $CH_3$, Cl, Br, F, OH, $OCH_3$ or $NO_2$), 2,4-dichloro-Phe, pentafluoro-Phe, or L-Phe;

$A^2$ represents Ala, pyridyl-Ala, Leu, Ile, Val, Met, Nle, Trp, beta-Nal, Phe, o-X-Phe (wherein X represents H, $CH_3$, Cl, Br, F, OH, $OCH_3$ or $NO_2$), p-X-Phe (wherein X represents H, $CH_3$, Cl, Br, F, OH, $OCH_3$, $NO_2$), 2,4-dichloro-Phe, or pentafluoro-Phe;

$A^3$ represents Ala, pyridyl-Ala, Leu, Ile, Val, Met, Nle, Trp, Tyr, beta-Nal, Phe, o-X-Phe (wherein X represents H, $CH_3$, Cl, Br, F, OH, $OCH_3$ or $NO_2$), p-X-Phe (wherein X represents H, $CH_3$, Cl, Br, F, OH, $OCH_3$ or $NO_2$), 2,4-dichloro-Phe, or pentafluoro-Phe;

$A^6$ represents Ala, pyridyl-Ala, Leu, Ile, Val, Lys, Met, Nle, Thr, Thr-$R^4$, Trp, Ser-$R^4$, beta-Nal, o-X-Phe (wherein X represents $CH_3$, Cl, Br, F, OH, $OCH_3$ or $NO_2$), p-X-Phe (wherein X represents $CH_3$, Cl, Br, F, OH, $OCH_3$ or $NO_2$), 2,4-dichloro-Phe, or pentafluoro-Phe;

$A^7$ represents Ala, pyridyl-Ala, Leu, Ile, Val, Met, Nle, Trp, beta-Nal, Phe, o-X-Phe (wherein X represents H, $CH_3$, Cl, Br, F, OH, $OCH_3$ or $NO_2$), p-X-Phe (wherein X represents H, $CH_3$, Cl, Br, F, OH, $OCH_3$ or $NO_2$), 2,4-dichlor-Phe, or pentafluoro-Phe;

$A^8$ represents a D- or L-isomer of any of Ala, pyridyl-Ala, Leu, Ile, Ser, Thr, Ser-$R^4$, Thr-$R^4$, Val, Met, Nle, Trp, beta-Nal, o-X-Phe (wherein X represents $CH_3$, Cl, Br, F, OH, $OCH_3$ or $NO_2$), p-X-Phe (wherein X represents $CH_3$, Cl, Br, F, OH, $OCH_3$ or $NO_2$), 2,4-dichloro-Phe, or pentafluoro-Phe;

each of $R^1$ and $R^2$ independently represent H, lower (e.g $C_{6-10}$) acyl, or lower (e.g. $C_{1-6}$) alkyl;

$R^3$ represents H, $NH_2$, or lower (e.g. $C_{1-6}$) alkyl;

provided that at least one of $A^1$ and $A^8$ must be an aromatic amino acid; and further provided that if either $A^2$ or $A^7$ represents an aromatic amino acid, then $A^8$ cannot be an aromatic amino acid;

$R^4$ may be nothing or may be a carbohydrate, e.g., $C_x(H_2O)_y$, where x is 1-18 and y is 1-16, linked through the hydroxyl group of Ser or Thr;

or a pharmaceutically acceptable salt thereof.

The linkage of the carbohydrate group to the serine or threonine hydroxyl group may be an alpha or beta linkage.

$R^4$ may, for example, be a protected glycosyl radical, e.g., a glucofuranosyl or glucopyranosyl radical which is derived from naturally occurring aldetetroses, aldopentoses, aldohexoses, ketopentoses, deoxyaldoses, aminoaldoses and oligosaccharides such as di- and trisaccharides, and stereoisomers thereof. $R^4$ may be derived from natural D- or L-monosaccharides which occur in microorganisms, plants, animals or humans, such as ribose, arabinose, xylose, lyxose, allose, altrose, glucose, mannose, gulose, idose,

glactose, talose, erythose, threose, psicose, fructrose, sorbose, tagatose, xylulose, fucose, rhamnose, olivose, oliose, mycarose, rhodosamine, N-acetyl-glucosamine, N-acetylgalacrosamine, n-acetyl-m-annosamine, or disaccharides such as maltose, lactose, cellobiose, gentiobiose, N-acetyl-lactosamine, chitobiose, beta-galactopyranosyl-(1,3)-N-acetylgalactosamine and beta-galactopyranosyl(1,4)-N-acetylglucosamine or a synthetic derivative thereof, such as a 2-deoxy-, 2-amino, 2-acetamido- or 2-halogeno-(especially bromo-and iodo-) sugar.

Protective groups may be, for example, the ($C_6$-$C_{10}$)-acyl groups, such as ($C_1$-$C_6$)-alkanoyl (e.g., acetyl, trichloroacetyl, trifluoroacetyl), benzoyl or p-nitrobenzoyl, and optionally modified methyl, methyloxymethyl, benzyl, tetrahydropyranyl, benzylidene, isopropylidene or trityl group, or the acyl protective groups, e.g., acetyl.

Preferably, of $A^1$ and $A^2$, only one is an aromatic amino acid; and of $A^7$ and $A^8$, only one is an aromatic amino acid.

In a preferred embodiment $A^1$ represents a D-isomer of Trp, beta-Nal, o-X-Phe (wherein X represents $CH_3$ or $OCH_3$), p-X-Phe (wherein X represents $CH_3$ or $OCH_3$) and $A^8$ represents a D- or L-isomer of Ala, pyridyl-Ala, Leu, Ile, Ser, Thr, Val, Met, Nle, o-X-Phe (wherein X represents Cl, Br, F, OH, $NO_2$), p-X-Phe (wherein X represents Cl, Br, F, OH, $NO_2$), 2,4-dichloro-Phe, or pentafluoro-Phe,

Preferably $A^1$ represents a D-isomer of o-X-Phe (wherein X represents H, Cl, Br, F, OH, $NO_2$), p-X-Phe (wherein X represents H, Cl, Br, F, OH, $NO_2$), 2,4-dichloro-Phe, or pentafluoro-Phe, or L-Phe, and $A^8$ represents a D- or L-isomer of Ala, pyridyl-Ala, Leu, Ile, Thr, Val, Met, Nle, Trp, beta-Nal, 0-X-Phe (wherein X represents $CH_3$ or $OCH_3$), or p-X-Phe (wherein X represents $CH_3$ or $OCH_3$).

In another preferred embodiment $A^8$ represents a D- or L-isomer of Thr, Trp, beta-Nal, o-X-Phe (wherein X represents $CH_3$ or $OCH_3$), or p-X-Phe (wherein X represents $CH_3$ or $OCH_3$) and $A^1$ represents Phe or a D-isomer of Ala, pyridyl-Ala, Leu, Ile, Val, Met, Nle, o-X-Phe (wherein X represents H, Cl, Br, F, OH, $NO_2$), p-X-Phe (wherein X represents H, Cl, Br, F, OH, $NO_2$), 2,4-dichloro-Phe, or pentafluoro-Phe.

Advantageously $A^8$ represents a D- or L-isomer of Ser, Thr, o-X-Phe (wherein X represents Cl, Br, F, OH, $NO_2$), p-X-Phe (wherein X represents Cl, Br, F, OH, $NO_2$), 2,4-dichloro-Phe, or pentafluoro-Phe; and $A^1$ represents a D- isomer of Ala, pyridyl-Ala, Leu, Ile, Val, Met, Nle, Trp, beta-Nal, o-X-Phe (wherein X represents $CH_3$ or $OCH_3$), p-X-Phe (wherein X represents $CH_3$ or $OCH_3$).

More preferably, $A^1$ represents beta-D-Nal or D-Phe; $A^2$ represents Ala, Phe, or p-chloro-Phe; $A^3$ represents Tyr or Phe; $A^6$ represents Val, Lys, Thr; $A^7$ represents Ala or Phe; $A^8$ represents Thr or D-beta-Nal.

Preferred compounds of the invention include:

D-Phe-p-chloro-Phe-Tyr-D-Trp-Lys-Val-Phe-Thr-$NH_2$ [SEQ.ID NO1];
D-Phe-Phe-Tyr-D-Trp-Lys-Val-Phe-Thr-$NH_2$ [SEQ.ID NO 2];
D-Phe-Phe-Phe-D-Trp-Lys-Thr-Phe-Thr-$NH_2$ [SEQ.ID NO 3]; and
D-Phe-Ala-Tyr-D-Trp-Lys-Val-Ala-beta-D-Nal-$NH_2$ [SEQ.ID NO 4].

The compounds of the invention have the general formula recited above. They are all octapeptide analogues of somatostatin which have D-Trp at the fourth position and Lys at the fifth position. It has been found that p-chloro-phenylalanine at position $A^2$ and threonine at position $A^8$ are modifications which particularly enhance activity. However, compounds containing an aromatic amino acid at position $A^8$ are inactive if there is an aromatic amino acid at either or both positions $A^2$ and $A^7$.

The compounds can be provided in the form of pharmaceutically acceptable salts. Examples of preferred salts are those with therapeutically acceptable organic acids, e.g., acetic, lactic, maleic, citric, malic, ascorbic, succinic, benzoic, salicylic, methanesulphonic, toluenesulphonic, or pamoic acid, as well as polymeric acids such as tannic acid or carboxymethyl cellulose and salts with inorganic acids such as the hydrohalic acids, e.g., hycrochloric acid, sulphuric acid or phosphoric acid.

A second aspect of the present invention relates to a compound of the first aspect for use in medicine.

A third aspect of the present invention encompasses a process for the preparation of a compound of the first aspect, the process comprising coupling successive amino acid residues together.

The compounds of the first aspect may be synthesized by any known and convenient method known in the art. However, a particularly preferred process will be described which involves the preparation of novel and valuable intermediates.

Thus a fourth aspect of the present invention relates to a compound having the general formula:

$$\begin{array}{l} \quad R^1 \\ \quad | \\ R^6 - A^1 - A^2 - A^3 - \text{D-Trp} - \text{N-benzyloxycarboxyl} - \text{Lys} - \\ \quad | \\ \quad R^2 \qquad\qquad\qquad\qquad A^6 - A^7 - A^8 - R^7 \qquad (II) \end{array}$$

wherein

$A^1$ represents a D-isomer of Ala, pyridyl-Ala, Leu, Ile, Val, Met, Nle, Trp, beta-Nal, Phe, o-X-Phe (wherein X represents $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, pentafluoro-Phe, or L-Phe;

$A^2$ represents Ala, pyridyl-Ala, Leu, Ile, Val, Met, Nle, Trp, beta-Nal, Phe, o-X-Phe (wherein X represents $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, or pentafluoro-Phe;

$A^3$ represents Ala, pyridyl-Ala, Leu, Ile, Val, Met, Nle, Trp, o-benzyl-Tyr, beta-Nal, Phe, o-X-Phe (wherein X represents H, $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, or pentafluoro-Phe;

$A^6$ represents Ala, pyridyl-Ala, Leu, Ile, Val, N-benzyloxycarbonyl-Lys, Met, Nle, Thr-$R^4$, Trp, Ser-$R^4$, beta-Nal, o-X-Phe (wherein X represents $CH_3$, Cl, Br, F, OH, $OCH_3$ or $NO_2$), p-X-Phe (wherein X = $CH_3$, Cl, Br, F, OH, $OCH_3$ or $NO_2$), 2,4-dichloro-Phe, or pentafluoro-Phe;

$A^7$ represents Ala, pyridyl-Ala, Leu, Ile, Val, Met, Nle, Trp, beta-Nal, Phe, o-X-Phe (wherein X represents $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, or pentafluoro-Phe;

$A^8$ represents a D- or L-isomer of Ala, pyridyl-Ala, Leu, Ile, Ser-$R^4$, Thr-$R^4$, Val, Met, Nle, Trp, beta-Nal, o-X-Phe (wherein X represents $CH_3$, Cl, Br, F, OH, $OCH_3$ or $NO_2$), p-X-Phe (wherein X = $CH_3$, Cl, Br, F, OH, $OCH_3$ or $NO_2$), 2,4-dichloro-Phe, or pentafluoro-Phe;

each of $R^1$ and $R^2$ individually represent a hydrogen atom, lower acyl or lower alkyl;

provided that at least one of $A^1$ and $A^8$ must be an aromatic amino acid; and further provided that if either $A^2$ or $A^7$ represents aromatic amino acid, then $A^8$ cannot be an aromatic amino acid;

$R^4$ represents nothing, a protected glycosyl residue, or an o-benzyl group or a carbohydrate;

$R^6$ represents a protective group, e.g. Boc;

$R^7$ represents a resin;

or a pharmaceutically acceptable salt thereof.

Preferably $R^4$ represents an o-benzyl group and/or the resin is a polystyrene resin, e.g. benzhydrylamine polystyrene resin.

A fifth aspect of the present invention relates to a process for the preparation of a compound of the fourth aspect of Formula II, the process comprising:

(a) reacting a resin with an amino acid residue with a side chain $A^8$ and a protecting group;

(b) reacting the resulting resin-bound compound with an amino acid residue with a side chain represented by $A^7$;

(c) repeating (b) with amino acids with side chains N-benzyloxycarbonyl-Lys, D-Trp, $A^3$, $A^2$ and $A^1$ consecutively; and

(d) where and when necessary, reacting the compound prepared, or one of the above residues with a glycosyl residue, a carbohydrate, an acylating agent or an alkylating agent.

The protecting group is preferably Boc.

In a sixth aspect of the present invention there is provided a process for the preparation of a compound of the first aspect, the process comprising reacting a compound of the fourth aspect with:

cresol;

dithiothreitol; and

hydrogen fluoride

and where necessary, removing the protecting group.

A seventh aspect of the present invention relates to the use of a compound of the first aspect in the preparation of an agent in the treatment or prophylaxis of cancer, acromegaly, hyposecretory endocrine

disorders, diabetes, cirrhosis, ulcers, pancreatitis, diarrhoea, hepatitis, Alzheimer,s disease, mushroom poisoning or retinopathy.

The invention can also be realised in the use of a compound of the first aspect in the preparation of an agent for inhibiting the release of human growth hormone, somatedins, insulin, glucagon, autoparacrine growth factors or pancreatic exocrine secretion.

An eighth aspect of the present invention relates to a pharmaceutical composition comprising a compound of the first aspect and a pharmaceutically acceptable carrier.

A ninth aspect of the present invention relates to a process for the preparation of a pharmaceutical composition, the process comprising admixing a compound of the first aspect and a pharmaceutically acceptable carrier.

In a preferred embodiment, a therapeutically effective amount of the therapeutic compound and a pharmaceutically acceptable carrier substance, e.g. magnesium carbonate, lactose, or a phospholipid with which the therepeutic compound can form a micelle, together form a therapeutic composition, e.g. a pill, tablet, capsule, or liquid for oral administration to a human patient, a (spreadable) cream, gel, lotion, or ointment to be applied topically or to be iontorphoretially forced through the skin of a human patient in need of the compound, a liquid capable of being administered nasally as drops or spray, or a liquid adapted for intravenous, parenteral, subcutaneous, or intraperitoneal administration. The composition, e.g. pill, tablet or capsule can be coated with a substance capable of protecting the composition from the gastric acid in the (human) patient's stomach for a period of time sufficient to allow the composition to pass undisintegrated into the patient's small intestine. The therapeutic composition may be in the form of a biodegradable or nonbiodegradable sustained release composition or formulation for intramuscular administration. For maximum efficacy, zero order release is desired, and can be obtained using an implantable or external pump, e.g. Infusoid™ pump, to administer the therapeutic composition.

The compounds of the invention may be active in inhibiting the release or secretion of growth hormone, somatomedins (e.g., IGF-1), insulin, glucagon and other autoparacrine growth factors or pancreatic growth factors. The compounds of the invention can be acyclic and, therefore can be stable and resistant to oxidation. In addition, such an acyclic nature of the peptide may facilitate synthesis and purification, and may also improve efficiency and reduce manufacturing costs.

It is to be understood that preferred features and characteristics of one aspect of the present invention apply to another aspect mutatatis mutandis.

The invention will be described by way of example with reference to the accompanying drawings, in which:

Fig. 1 is a graph showing the effects of linear analogues of the present invention on growth hormone secretion by rat pituitary cells.

Fig. 2 is a graph showing the effects of linear analogues of the present invention on growth hormone secretion by rat pituitary cells.

The invention will now be described by way of example, with reference to the accompanying Examples which are provided by way of illustration and are not intended as being limiting.

Example 1

The synthesis of one therapeutic peptide follows. Other peptides can be prepared by making appropriate modifications, within the ability of someone of ordinary skill in this field, of the following synthetic method.

The first step in the preparation of the peptide

D-Phe-Phe-Phe-D-Trp-Lys-Thr-Phe-Thr-$NH_2$ [SEQ.ID NO 3]

is the preparation of an intermediate:

Boc-D-Phe-Phe-Phe-D-Trp-N-benzyloxycarbonyl-Lys-O-benzyl-Thr-Phe-O-benzyl-Thr-benzhydrylamine resin, as follows.

Benzhydrylamine-polystyrene resin (Advanced ChemTech, Inc. (1.2g, 0.5 mmole) in the chloride ion form is placed in the reaction vessel of an Advanced ChemTech peptide synthesizer. programmed to perform the following reaction cycle: (a) methylene chloride; (b) 33% trifluoroacetic acid in methylene chloride (2 times for 1 and 25 min each); (c) methylene chloride; (d) ethanol; (e) methylene chloride; and (f) 10% triethylamine in chloroform.

The neutralized resin was stirred with Boc-O-benzyl-threonine and diisopropylcarbodiimide (1.5 mmole each) in methylene chloride for 1 hr and the resulting amino acid resin is then cycled through steps (a) to (f) in the above wash program. The following amino acids (1.5 mmole) are then coupled successively by the same procedure:

Boc-Phe, Boc-O-benzyl-Thr, Boc-N-benzyloxycarbonyllysine, Boc-D-Trp, Boc-Phe, and Boc-Phe and Boc-D-Phe. After washing and drying, the completed resin weighed 1.70 g.

The resin (1.70 g, 0.5 mmole) is then mixed with cresol (5 ml), dithiothreitol (100 mg) and anhydrous hydrogen fluoride (35 ml) at 0 °C and stirred for 45 min. Excess hydrogen fluoride is evaporated rapidly under a stream of dry nitrogen, and free peptide precipitated and washed with ether. The crude peptide is then dissolved in a minimum volume of 50% acetic acid and eluted on a colum (2.5 x 100 cm) of Sephadex G-25 using the same solvent. Fractions containing a major component by UV absorption and thin layer chromatography are then pooled, evaporated to a small volume and applied to a column (2.5 x 50 cm) of Vydac octadecylsilane silica (10-15 µM).

The column was eluted with a linear gradient of 10-45% acetonitrile in 0.1% trifluoroacetic acid in water. Fractions are examined by thin layer chromatography and analytical high performance liquid chromatography and pooled to give maximum purity. Repeated lyophilization of the solution from water gives 65 mg of the product as a white, fluffy powder.

The product was found to be homogeneous by hplc and tlc. Amino acid analysis of an acid hydrolysate confirms the composition of the octapeptide.

Other peptides of the invention may be prepared in an analogous fashion to those described above.

Example 2

Effects of linear somatostatin analogs on growth hormone secretion in cultured rat pituitary cell dispersion

Octapeptides of the invention are tested for inhibtion of growth hormone-releasing-activity using rat pituitary cells, as follows.

Anterior pituitaries from adult Charles River CD male rats (Wilmington, MA) weighing 200-250 g and housed under controlled conditions (lights on from 0500-1900 h), were dispersed and cultured using aseptic technique by modification of previously described methods (Hoefer et al., 1984, Mol. Cell. Endocrinol. 35:229; Ben-Jonathan et al., 1983, Methods Enzymol. 103:249; Heiman et al., 1985, Endocrinology 116:410). Pituitaries were removed from decapitated rats, sectioned, and then placed into a siliconized, liquid scintillation vial containing 2 ml 0.2% trypsin (Worthington Biochemicals, Freehold, NJ) in sterile-filtered Krebs-Ringer bicarbonate buffer supplemented with 1% bovine serum albumin, 14mM glucose, modified Eagle medium (MEM) vitamin solution and MEM amino acids (Gibco Laboratories, Grand Island, NY) (KRBGA). All glassware was siliconized as described by Sayers et al., 1971, Endocrinology 88:1063. The fragments were incubated in a water bath for 35 min at 37 °C with agitation. The vial contents then were poured into a scintillation vial containing 2 ml 0.1% DNase (Sigma Chemical Co., St. Louis, MO) in KRBGA and incubated for 2 min at 37 °C with agitation. After incubation the tissue was decanted back into the centrifuge tube and allowed to settle. Medium was discarded, and pituitary sections were washed 3 times with 1 ml fresh KRBGA. The cells were then dispersed by gently drawing the fragments into and expelling them out of a siliconized, fire-polished Pasteur pipette in 2 ml 0.05% LBI (lima beam trypsin inhibitor, Worthington Biochemicals). Dispersed cells were filtered through a 630µm diameter Nylon mesh (Tetko, Elmsford, NY) into a fresh 15 ml centrifuge tube and harvested by centrifugation at 100 x g for 1 min. The final speed was attained gradually through a centrifugation period of 17 min.

After centrifugation, medium was discarded and the pelleted cells were resuspended in fresh LBI (2 ml) with a Pasteur pipette. The dispersed cells were then diluted with approximately 15 ml sterile-filtered Dulbecco's modified Eagle medium (GIBCO), which was supplemented with 2.5% fetal calf serum (GIBCO), 3% horse serum (GIBCO), 10% fresh rat serum (stored on ice for no longer than 1 h) from the pituitary donors, 1% MEM nonessential amino acids (GIBCO), gentamycin (10 ng/ml; Sigma) and nyatatin (10,000 U/ml; GIBCO). The cells were poured into a 50 ml round-bottomed glass extraction flask with a large diameter opening and were counted with a lemacytometer (approximately 2,000,000 cells per pituitary) and randomly plated at a density of 200,000 cells per well (Co-star cluster 24; Rochester Scientific Co., Rochester, NY). The plated cells were maintained in the above Dulbecco's medium in a humidified atmosphere of 95% air and 5% $CO_2$ at 37 °C for 96 h.

In preparation for a hormone challenge, the cells were washed 3x with medium 199 (GIBCO) to remove old medium and floating cells. Each dose of analog (diluted in normal saline in siliconized test tubes) was tested in the presence of 1 nM GRF(1-29)$NH_2$ (growth hormone releasing factor) in quadruplicate wells in a total volume of 1 ml medium 199 containing 1% BSA (fraction V; Sigma). After 3 h. at 37 °C in an air/carbon dioxide atmosphere (95/5%), the medium was removed and stored at -20 °C until assayed for hormone content. Growth hormone was measured in a conventional radioimmunoassay using anti-growth hormone antibody.

The effect of 9 different peptides on the release of growth hormone in cultured rat pituitary cells is shown in Figs. 1 and 2. The peptides DC-25-4 (Figure 1) and DC-25-24 (Figure 2) are most active in inhibiting the release of growth hormone. Both DC-25-4 and DC-25-24 contain an electron withdrawing group near one end of the molecule and an electron donating group near the opposite end of the molecule. Peptides DC-23-85 (Figure 1) and DC-25-16 (Figure 2), which are not within the present invention, show essentially no activity.

Example 3

Inhibition of $I^{125}$ Somatotropin-release-inhibiting factor (SRIF-14) binding by linear somatostatin analogs

Crude membrane preparations were obtained from rat pancreas, cerebral cortex, or human small cell lung carcinoma (NCI-H69) cells by homogenizing (Polytron, setting 6, 15 sec) the tissues or cells in ice-cold 50 mM Tris-HCl and centrifuging twice at 39,000 x g (10 min), with an intermediate resuspension in fresh buffer. The final pellets were resuspended in 10 mM Tris-HCl for assay. Aliquots of the membrane preparation were incubated for 25 min at 30 °C with labeled somatotropin-release-inhibiting factor, [$^{125}$I-Tyr$^{11}$] SRIF-14 (2000 Ci/mmol, Amersham Corp.), in 50 mM HEPES (pH 7.4) containing bovine serum albumin (10 mg/ml; fraction V, Sigma Chem.), $MgCl_2$ (5mM), Trasylol (200 KIU/ml), bacitracin (0.02 mg/ml), and phenylmethylsulphonyl fluoride (0.02 mg/ml). The final assay volume was 0.3 ml. The incubations were terminated by rapid filtration through Whatman GF/C filters (pre-soaked in 0.3% polyethylenimine) under reduced pressure. Each tube and filter were then washed three times with 5 ml aliquots of ice-cold buffer. Specific binding was defined as the total [$^{125}$I]SRIF-14 bound minus that bound in the presence of 200 nM unlabelled SRIF-14.

Table 1 gives results of inhibition of [$^{125}$I]SRIF-14 binding by linear peptides of the invention. The concentration of [$^{125}$I]SRIF-14 was approximately 0.05 nM. (Values in parenthesis indicate the number of independent determinations.) The $IC_{50}$ (concentration of analog resulting is 50% competitive inhibition) in nM values are indicated for pancreas, small cell lung carcinoma (SCLC), and brain. The results show that analogs DC-25-4 and DC-23-99 are particularly effective in inhibiting the binding of $I^{125}$ SRIF-14. Peptide DC-23-85, which is not within the invention, inhibits the binding of $I^{125}$SRIF-14 only poorly.

Use

When administered to mammals, particularly humans, (e.g. orally, topically, intravenously, parenterally in a sustained release, biodegradable or nonbiodegradable form, nasally, or by suppository), the compounds can be effective to inhibit growth hormone release as well as to inhibit somatomedins (e.g., IGF-1), insulin, glucagon, other autoparacrine growth factors or pancreatic exocrine secretion, and to therapeutically affect the central nervous system.

The compounds can be administered to a mammal, e.g. a human, in the dosages used for somatostatin or, because of their greater potency, in smaller dosages. The compounds of the invention can be used for the treatment of cancer, particularly growth hormone-dependent cancer (e.g., bone, cartilage, pancreas (endocrine and exocrine), prostate, or breast), acromegaly and related hypersecretory endocrine states, or of bleeding ulcers in emergency patients and in those suffering from pancreatitis or diarrhea. The compounds can also be used in the management of diabetes and to protect the liver of patients suffering from cirrhosis and hepatitis. The compounds can also be used to treat Alzheimer's disease, as analgesics to treat pain by acting specifically on certain opiate receptors, and as gastric cytoprotective compounds for ulcer therapy. The compounds can also be used to treat certain types of mushroom poisoning.

The compounds can also be used to treat diabetes-related retinopathy. The anti-cancer activity of the compounds may be related to their ability to antagonize cancer-related growth factors such as epidermal growth factor.

The compounds can be administered to a mammal, e.g., a human, in a dosage of 0.01 to 1000 mcg/kg/day, preferably 0.1 to 100 mcg/kg/day.

Mechanism

The activity of previously described analogs of somatostatin is dependent on the presense of a disulfide linkage between cysteine residues located at or near the ends of the peptide, see, e.g., Coy et al, U.S. Patent No. 4,485,101, hereby incorporated by reference. The disulfide linkage results in a cyclic conformation necessary for activity.

The inclusion of a disulfide linkage is an undesirable feature in these synthetic peptides in that the step favoring synthesis of the disulfide linkage imposes a dramatic decrease in the overall yield of the synthesis. Furthermore, the disulfide linkages are subject to oxidation and thus result in a less stable product.

The instant invention may avoid the use of disulfide linkages and their attendant drawbacks. The octapeptides of the instant invention may utilize non-covalent interactions between the side chains of critically positioned constituent amino acid residues to confer a hairpin or quasi-cyclic conformation on the peptides.

The side chains and substituted side chains of the amino acid residues of the instant invention may be subject to two types of interactions that tend to confer the desired tertiary structure on the peptide. The first type of interaction occurs when amino acids bearing hydrophobic side chains are located at or near both ends of the peptide. Peptides of this structure exploit the tendency of hydrophobic moieties to avoid contact with polar substances. Interactions between the hydrophobic groups at each end of the peptide, favored over interactions between these groups and the polar solvents of physiological environments, confer a hairpin or quasi-cyclic configuration on the peptide.

The second type of interaction arises as a result of the interaction of electron-donating and electron-withdrawing moieties of amino acids at opposite ends of the peptide. The invention features peptides in which an amino acid possessing an electron-donating group resides in one end region of the peptide while an amino acid possessing an electron-withdrawing group resides in the other end region of the peptide. The attraction between the electron-donating group, at one end of the peptide, and the electron-withdrawing group, at the other end of the peptide, acts to confer a hairpin or quasi-cyclic structure on the peptide. Both hydrophobic-hydrophobic interactions and electron donor-electron withdrawer interactions may be active in a given peptide.

Other embodiments are within the following claims.

Table I

Table 1

Inhibition of $I^{125}$ SRIF-14 binding by linear analogs of somatostatin

| | $IC_{50}$ (nM) | | |
|---|---|---|---|
| Analog | Pancreas | SCLC | Brain |
| Somatostatin | 0.53 (5) | 4.2 (5) | 0.53 (3) |
| BIM-23053/DC-25-4 | 2.8 (2) | 2.2 (1) | 109 |
| BIM-23052/DC-23-99 | 9.4 (1) | 1.2 (1) | 7.3 (1) |
| BIM-23049/DC-23-76 | 9.2 (3) | 2.1 (1) | >10,000 (1) |
| BIM-23051/DC-23-89 | 34 (2) | 15 (1) | >10,000 (1) |
| BIM-23050/DC-23-85 | 264 (1) | --- | 2,189 (2) |

Results are expressed as the concentration in nM of analogue that gives 50% inhibition of $I^{125}$ SRIF-14 binding ($IC_{50}$). The numbers in parantheses indicate the number of trials. The structure of the analogues is as follows: BIM-23049/DC-23-76--beta-D-Nal-Ala-Tyr-D-Trp-Lys-Val-Ala-Thr-$NH_2$ [SEQ.ID NO 5]; BIM-23050/DC-23-85--n-methyl-D-Ala-Tyr-D-Trp-Lys-Val-Phe-$NH_2$ [SEQ.ID NO 6]; BIM-23051/DC-23-89--D-Phe-Ala-Phe-D-Trp-Lys-Thr-Ala-Thr-$NH_2$ [SEQ.ID NO 7]; BIM-23052/DC-23-99--D-Phe-Phe-Phe-D-Trp-Lys-Thr-Phe-Thr-$NH_2$ [SEQ.ID NO 3]; BIM-23053/DC-25-4--D-Phe-Ala-Tyr-D-Trp-Lys-Val-Ala-beta-D-Nal-$NH_2$ [SEQ.ID NO 4]. The structure of SRIF-14 is: Ala-Gly-Cys-Lys-Asn-Phe-Phe-Trp-Lys-Thr-Phe-Ser-OH.

| Key | SEQ.ID NO |
|---|---|
| BIM-23049/DC-23-76 | 5 |
| BIM-23050/DC-23-85 | 6 |
| BIM-23051/DC-23-89 | 7 |
| BIM-23052/DC-23-99 | 3 |
| BIM-23053/DC-25-4 | 4 |

**SEQUENCE LISTING:**

**SEQ ID NO:** 1
**SEQUENCE TYPE:** Amino acid
**SEQUENCE LENGTH:** 8

**FEATURES:**
**LOCATION OF FEATURES:** Position 4: D-Trp
Position 2: p-Cl-Phe
Position 1: D-Phe

**PROPERTIES:** Octapeptide analogue of somatostatin

**SEQUENCE:**

```
Xaa Xaa Tyr Xaa Lys Val Phe Thr
 1              5
```

---

**SEQ ID NO:** 2
**SEQUENCE TYPE:** Amino acid
**SEQUENCE LENGTH:** 8

**FEATURES:**
**LOCATION OF FEATURES:** Position 4: D-Trp
Position 1: D-Phe

**PROPERTIES:** Octapeptide analogue of somatostatin

**SEQUENCE:**

```
Xaa Phe Tyr Xaa Lys Val Phe Thr
 1              5
```

---

**SEQ ID NO:** 3
**SEQUENCE TYPE:** Amino acid
**SEQUENCE LENGTH:** 8

**FEATURES:**
**LOCATION OF FEATURES:** Position 4: D-Trp
Position 1: D-Phe

**PROPERTIES:** Octapeptide analogue of somatostatin

**SEQUENCE:**

```
Xaa Phe Phe Xaa Lys Thr Phe Thr
  1               5
```

---

**SEQ ID NO:** 4
**SEQUENCE TYPE:** Amino acid
**SEQUENCE LENGTH:** 8

**FEATURES:**
**LOCATION OF FEATURES:** Position 8: beta-D-Nal
Position 4: D-Trp
Position 1: D-Phe

**PROPERTIES:** Octapeptide analogue of somatostatin

**SEQUENCE:**

```
Xaa Ala Tyr Xaa Lys Val Ala Xaa
  1               5
```

---

**SEQ ID NO:** 5
**SEQUENCE TYPE:** Amino acid
**SEQUENCE LENGTH:** 8

**FEATURES:**
**LOCATION OF FEATURES:** Position 4: D-Trp
Position 1: beta-D-Nal

**PROPERTIES:** Octapeptide analogue of somatostatin

**SEQUENCE:**

```
Xaa Ala Tyr Xaa Lys Val Ala Thr
 1               5
```

---

**SEQ ID NO:** 6
**SEQUENCE TYPE:** Amino acid
**SEQUENCE LENGTH:** 6

**FEATURES:**
**LOCATION OF FEATURES:** Position 3: D-Trp
Position 1: N-methyl-D-Ala

**PROPERTIES:** Hexapeptide analogue of somatostatin

**SEQUENCE:**

```
Xaa Tyr Xaa Lys Val Phe
 1               5
```

---

**SEQ ID NO:** 7
**SEQUENCE TYPE:** Amino acid
**SEQUENCE LENGTH:** 8

**FEATURES:**
**LOCATION OF FEATURES:** Position 4: D-Trp
Position 1: D-Phe

**PROPERTIES:** Octapeptide analogue of somatostatin

**SEQUENCE:**

```
Xaa Ala Phe Xaa Lys Thr Ala Thr
 1              5
```

## Claims

### Claims for the following Contracting States : AT, BE, CH, DE, DK, FR, GB, IT, LI, LU, NL, SE

**1.** A compound having the general formula:

$$\begin{array}{l} R^1 \\ | \\ A^1 - A^2 - A^3 - \text{D-Trp} - \text{Lys} - A^6 - A^7 - A^8 - R^3 \\ | \\ R^2 \end{array}$$

wherein
$A^1$ represents a D-isomer of Ala, pyridyl-Ala, Leu, Ile, Val, Met, Nle, Trp, beta-Nal, Phe, o-X-Phe (wherein X represents $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, pentafluoro-Phe, or L-Phe;
$A^2$ represents Ala, pyridyl-Ala, Leu, Ile, Val, Met, Nle, Trp, beta-Nal, Phe, o-X-Phe (wherein X represents $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, or pentafluoro-Phe;
$A^3$ represents Ala, pyridyl-Ala, Leu, Ile, Val, Met, Nle, Trp, Tyr, beta-Nal, Phe, o-X-Phe (wherein X represents H, $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, or pentafluoro-Phe;
$A^6$ represents Ala, pyridyl-Ala, Leu, Ile, Val, Lys, Met, Nle, Thr-$R^4$, Trp, Ser-$R^4$, beta-Nal, o-X-Phe (wherein X represents $CH_3$, Cl, Br, F, OH, $OCH_3$ or $NO_2$), p-X-Phe (wherein X = $CH_3$, Cl, Br, F, OH, $OCH_3$ or $NO_2$), 2,4-dichloro-Phe, or pentafluoro-Phe;
$A^7$ represents Ala, pyridyl-Ala, Leu, Ile, Val, Met, Nle, Trp, beta-Nal, Phe, o-X-Phe (wherein X represents $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, or pentafluoro-Phe;
$A^8$ represents a D- or L-isomer of any of Ala, pyridyl-Ala, Leu, Ile, Ser-$R^4$, Thr-$R^4$, Val, Met, Nle, Trp, beta-Nal, o-X-Phe (wherein X represents $CH_3$, Cl, Br, F, OH, $OCH_3$ or $NO_2$), p-X-Phe (wherein X = $CH_3$, Cl, Br, F, OH, $OCH_3$ or $NO_2$), 2,4-dichloro-Phe, or pentafluoro-Phe;
each of $R^1$ and $R^2$ independently represent a hydrogen atom, lower acyl or lower alkyl;

$R^3$ represents a hydrogen atom, an $-NH_2$ or a loweralkyl group;
provided that at least one of $A^1$ and $A^8$ must be an aromatic amino acid; and further provided that if either $A^2$ or $A^7$ represents an aromatic amino acid, then $A^8$ cannot be an aromatic amino acid;
$R^4$ represents nothing, a protected glycosyl residue, or a carbohydrate;
or a pharmaceutically acceptable salt thereof.

**2.** A compound as claimed in claim 1, wherein one but not both $A^1$ and $A^2$ represent an aromatic amino acid; and wherein one but not both of $A^7$ and $A^8$ represent an aromatic amino acid.

**3.** A compound as claimed in claim 1 or 2 wherein $A^1$ represents a D-isomer of Trp, beta-Nal, o-X-Phe (wherein X represents $CH_3$ or $OCH_3$), p-X-Phe (wherein X represents $CH_3$ of $OCH_3$); and
$A^8$ represents a D- or L-isomer of Ala, pyridyl-Ala, Leu, Ile, Ser, Thr, Val, Met, Nle, o-X-Phe (wherein X represents Cl, Br, F, OH or $NO_2$), p-X-Phe (wherein X represents Cl, Br, F, OH, or $NO_2$), 2,4-dichloro-Phe, or pentafluoro-Phe.

**4.** A compound as claimed in any of claims 1 to 3, which has one, some or all of the following characteristics:
$A^1$ represents beta-D-Nal or D-Phe;
$A^2$ represents Ala, Phe or p-chloro-Phe;
$A^3$ represents Tyr or Phe;
$A^6$ represents Val, Lys or Thr;
$A^7$ represents Ala or Phe; and/or
$A^8$ represents Thr or beta-D-Nal.

**5.** A compound as claimed in any of claims 1 to 4 which is:
D-Phe-Phe-Phe-D-Trp-Lys-Thr-Phe-Thr-$NH_2$ [SEQ.ID NO 3];
D-Phe-Phe-Tyr-D-Trp-Lys-Val-Phe-Thr-$NH_2$ [SEQ.ID NO 2];
D-Phe-p-chloro-Phe-Tyr-D-Trp-Lys-Val-Phe-Thr-$NH_2$ [SEQ.ID NO 1]; or
D-Phe-Ala-Tyr-D-Trp-Lys-Val-Ala-beta-D-Nal-$NH_2$ [SEQ.ID NO 4].

**6.** A compound having the general formula:

$$\begin{array}{l} R^1 \\ | \\ A^1 - A^2 - A^3 - \text{D-Trp} - \text{Lys} - A^6 - A^7 - A^8 - R^3 \\ | \\ R^2 \end{array}$$

wherein
$A^1$ represents a D-isomer of Ala, pyridyl-Ala, Leu, Ile, Val, Met, Nle, Trp, beta-Nal, Phe, o-X-Phe (wherein X represents $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, pentafluoro-Phe, or L-Phe;
$A^2$ represents Ala, pyridyl-Ala, Leu, Ile, Val, Met, Nle, Trp, beta-Nal, Phe, o-X-Phe (wherein X represents $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, or pentafluoro-Phe;
$A^3$ represents Ala, pyridyl-Ala, Leu, Ile, Val, Met, Nle, Trp, Tyr, beta-Nal, Phe, o-X-Phe (wherein X represents H, $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, or pentafluoro-Phe;
$A^6$ represents Ala, pyridyl-Ala, Leu, Ile, Val, Lys, Met, Nle, Thr-$R^4$, Trp, Ser-$R^4$, beta-Nal, o-X-Phe (wherein X represents $CH_3$, Cl, Br, F, OH, $OCH_3$ or $NO_2$), p-X-Phe (wherein X = $CH_3$, Cl, Br, F, OH, $OCH_3$ or $NO_2$), 2,4-dichloro-Phe, or pentafluoro-Phe;
$A^7$ represents Ala, pyridyl-Ala, Leu, Ile, Val, Met, Nle, Trp, beta-Nal, Phe, o-X-Phe (wherein X represents $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, or pentafluoro-Phe;

$A^8$ represents a D- or L-isomer of any of Ala, pyridyl-Ala, Leu, Ile, Ser-$R^4$, Thr-$R^4$, Val, Met, Nle, Trp, beta-Nal, o-X-Phe (wherein X represents $CH_3$, Cl, Br, F, OH, $OCH_3$ or $NO_2$), p-X-Phe (wherein X = $CH_3$, Cl, Br, F, OH, $OCH_3$ or $NO_2$), 2,4-dichloro-Phe, or pentafluoro-Phe;
each of $R^1$ and $R^2$ independently represent a hydrogen atom, lower acyl or lower alkyl;
$R^3$ represents a hydrogen atom, -$NH_2$ or loweralkyl group;
provided that at least one of $A^1$ and $A^8$ must be an aromatic amino acid; and further provided that if either $A^2$ or $A^7$ represents an aromatic amino acid, then $A^8$ cannot be an aromatic amino acid;
$R^4$ represents nothing, a protected glycosyl residue, or a carbohydrate;
or a pharmaceutically acceptable salt thereof; for use in medicine.

**7.** A pharmaceutical composition comprising a compound having the general formula:

$R^1$
|
$A^1$ - $A^2$ - $A^3$ - D-Trp - Lys - $A^6$ - $A^7$ - $A^8$ - $R^3$
|
$R^2$

wherein
$A^1$ represents a D-isomer of Ala, pyridyl-Ala, Leu, Ile, Val, Met, Nle, Trp, beta-Nal, Phe, o-X-Phe (wherein X represents $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, pentafluoro-Phe, or L-Phe;
$A^2$ represents Ala, pyridyl-Ala, Leu, Ile, Val, Met, Nle, Trp, beta-Nal, Phe, o-X-Phe (wherein X represents $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, or pentafluoro-Phe;
$A^3$ represents Ala, pyridyl-Ala, Leu, Ile, Val, Met, Nle, Trp, Tyr, beta-Nal, Phe, o-X-Phe (wherein X represents H, $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, or pentafluoro-Phe;
$A^6$ represents Ala, pyridyl-Ala, Leu, Ile, Val, Lys, Met, Nle, Thr-$R^4$, Trp, Ser-$R^4$, beta-Nal, o-X-Phe (wherein X represents $CH_3$, Cl, Br, F, OH, $OCH_3$ or $NO_2$), p-X-Phe (wherein X = $CH_3$, Cl, Br, F, OH, $OCH_3$ or $NO_2$), 2,4-dichloro-Phe, or pentafluoro-Phe;
$A^7$ represents Ala, pyridyl-Ala, Leu, Ile, Val, Met, Nle, Trp, beta-Nal, Phe, o-X-Phe (wherein X represents $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, or pentafluoro-Phe;
$A^8$ represents a D- or L-isomer of any of Ala, pyridyl-Ala, Leu, Ile, Ser-$R^4$, Thr-$R^4$, Val, Met, Nle, Trp, beta-Nal, o-X-Phe (wherein X represents $CH_3$, Cl, Br, F, OH, $OCH_3$ or $NO_2$), p-X-Phe (wherein X = $CH_3$, Cl, Br, F, OH, $OCH_3$ or $NO_2$), 2,4-dichloro-Phe, or pentafluoro-Phe;
each of $R^1$ and $R^2$ independently represent a hydrogen atom, lower acyl or lower alkyl;
$R^3$ represents a hydrogen atom, -$NH_2$ or loweralkyl group;
provided that at least one of $A^1$ and $A^8$ must be an aromatic amino acid; and further provided that if either $A^2$ or $A^7$ represents an aromatic amino acid, then $A^8$ cannot be an aromatic amino acid;
$R^4$ represents nothing, a protected glycosyl residue, or a carbohydrate;
or a pharmaceutically acceptable salt thereof, and a pharmaceutically acceptable carrier.

**8.** A compound as claimed in claim 7 which is coated with a substance capable of protecting the composition from the gastric acid in the stomach for a period of time sufficient to allow the composition to pass undisintegrated into the small intestine.

**9.** A process for the preparation of a compound of the general formula:

$R^1$
|
$A^1 - A^2 - A^3 - \text{D-Trp} - \text{Lys} - A^6 - A^7 - A^8 - R^3$
|
$R^2$

wherein
$A^1$ represents a D-isomer of Ala, pyridyl-Ala, Leu, Ile, Val, Met, Nle, Trp, beta-Nal, Phe, o-X-Phe (wherein X represents $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, pentafluoro-Phe, or L-Phe;
$A^2$ represents Ala, pyridyl-Ala, Leu, Ile, Val, Met, Nle, Trp, beta-Nal, Phe, o-X-Phe (wherein X represents $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, or pentafluoro-Phe;
$A^3$ represents Ala, pyridyl-Ala, Leu, Ile, Val, Met, Nle, Trp, Tyr, beta-Nal, Phe, o-X-Phe (wherein X represents H, $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, or pentafluoro-Phe;
$A^6$ represents Ala, pyridyl-Ala, Leu, Ile, Val, Lys, Met, Nle, Thr-$R^4$, Trp, Ser-$R^4$, beta-Nal, o-X-Phe (wherein X represents $CH_3$, Cl, Br, F, OH, $OCH_3$ or $NO_2$), p-X-Phe (wherein X = $CH_3$, Cl, Br, F, OH, $OCH_3$ or $NO_2$), 2,4-dichloro-Phe, or pentafluoro-Phe;
$A^7$ represents Ala, pyridyl-Ala, Leu, Ile, Val, Met, Nle, Trp, beta-Nal, Phe, o-X-Phe (wherein X represents $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, or pentafluoro-Phe;
$A^8$ represents a D- or L-isomer of any of Ala, pyridyl-Ala, Leu, Ile, Ser-$R^4$, Thr-$R^4$, Val, Met, Nle, Trp, beta-Nal, o-X-Phe (wherein X represents $CH_3$, Cl, Br, F, OH, $OCH_3$ or $NO_2$), p-X-Phe (wherein X = $CH_3$, Cl, Br, F, OH, $OCH_3$ or $NO_2$), 2,4-dichloro-Phe, or pentafluoro-Phe;
each of $R^1$ and $R^2$ independently represent a hydrogen atom, lower acyl or lower alkyl;
$R^3$ represents a hydrogen atom, $-NH_2$ or loweralkyl group;
provided that at least one of $A^1$ and $A^8$ must be an aromatic amino acid; and further provided that if either $A^2$ or $A^7$ represents aromatic amino acid, then $A^8$ cannot be an aromatic amino acid;
$R^4$ represents nothing, a protected glycosyl residue, or or a carbohydrate;
or a pharmaceutically acceptable salt thereof;
the process comprising reacting a compound of the general formula:

$R^1$
|
$R^6 - A^1 - A^2 - A^3 - \text{D-Trp} - \text{N-benzyloxycarboxyl} - \text{Lys} -$
|
$R^2$
$A^6 - A^7 - A^8 - R^7$

wherein:
$R^1$, $R^2$, $A^1$, $A^2$, $A^7$ and $A^8$ are as defined before;
$A^3$ is as defined before except that Tyr is replaced by o-benzyl-Tyr;
$A^6$ is as defined before except that Lys is replaced by N-benzyloxycarbonyl-Lys;
$R^4$ can additionally represent an o-benzyl group;
$R^6$ represents a protective group; and
$R^7$ represents a resin;
with:

cresol;
dithiothreitol; and

hydrogen fluoride.

**10.** A compound having the general formula:

$$
\begin{array}{l}
\quad\;\; R^1 \\
\quad\;\; | \\
R^6 - A^1 - A^2 - A^3 - \text{D-Trp} - \text{N-benzyloxycarboxyl} - \text{Lys} - \\
\quad\;\; | \\
\quad\;\; R^2 \qquad\qquad\qquad\qquad\qquad\qquad A^6 - A^7 - A^8 - R^7
\end{array}
$$

wherein
$A^1$ represents a D-isomer of Ala, pyridyl-Ala, Leu, Ile, Val, Met, Nle, Trp, beta-Nal, Phe, o-X-Phe (wherein X represents $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, pentafluoro-Phe, or L-Phe;
$A^2$ represents Ala, pyridyl-Ala, Leu, Ile, Val, Met, Nle, Trp, beta-Nal, Phe, o-X-Phe (wherein X represents $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, or pentafluoro-Phe;
$A^3$ represents Ala, pyridyl-Ala, Leu, Ile, Val, Met, Nle, Trp, o-benzyl-Tyr, beta-Nal, Phe, o-X-Phe (wherein X represents H, $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, or pentafluoro-Phe;
$A^6$ represents Ala, pyridyl-Ala, Leu, Ile, Val, N-benzyloxycarbonyl-Lys, Met, Nle, Thr-$R^4$, Trp, Ser-$R^4$, beta-Nal, o-X-Phe (wherein X represents $CH_3$, Cl, Br, F, OH, $OCH_3$ or $NO_2$), p-X-Phe (wherein X = $CH_3$, Cl, Br, F, OH, $OCH_3$ or $NO_2$), 2,4-dichloro-Phe, or pentafluoro-Phe;
$A^7$ represents Ala, pyridyl-Ala, Leu, Ile, Val, Met, Nle, Trp, beta-Nal, Phe, o-X-Phe (wherein X represents $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, or pentafluoro-Phe;
$A^8$ represents a D- or L-isomer of Ala, pyridyl-Ala, Leu, Ile, Ser-$R^4$, Thr-$R^4$, Val, Met, Nle, Trp, beta-Nal, o-X-Phe (wherein X represents $CH_3$, Cl, Br, F, OH, $OCH_3$ or $NO_2$), p-X-Phe (wherein X = $CH_3$, Cl, Br, F, OH, $OCH_3$ or $NO_2$), 2,4-dichloro-Phe, or pentafluoro-Phe;
each of $R^1$ and $R^2$ individually represent a hydrogen atom, lower acyl or lower alkyl;
provided that at least one of $A^1$ and $A^8$ must be an aromatic amino acid; and further provided that if either $A^2$ or $A^7$ represents an aromatic amino acid, then $A^8$ cannot be an aromatic amino acid;
$R^4$ represents nothing, a protected glycosyl residue, or an o-benzyl group or a carbohydrate;
$R^6$ represents a protective group, e.g. Boc;
$R^7$ represents a resin;
or a pharmaceutically acceptable salt thereof.

**11.** A process for the preparation of a compound of the general formula:

$$
\begin{array}{l}
\quad\;\; R^1 \\
\quad\;\; | \\
R^6 - A^1 - A^2 - A^3 - \text{D-Trp} - \text{N-benzyloxycarboxyl} - \text{Lys} - \\
\quad\;\; | \\
\quad\;\; R^2 \qquad\qquad\qquad\qquad\qquad\qquad A^6 - A^7 - A^8 - R^7
\end{array}
$$

wherein:
$A^1$ represents a D-isomer of Ala, pyridyl-Ala, Leu, Ile, Val, Met, Nle, Trp, beta-Nal, Phe, o-X-Phe (wherein X represents $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, pentafluoro-Phe, or L-Phe;
$A^2$ represents Ala, pyridyl-Ala, Leu, Ile, Val, Met, Nle, Trp, beta-Nal, Phe, o-X-Phe (wherein X

represents $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, or pentafluoro-Phe;

$A^3$ represents Ala, pyridyl-Ala, Leu, Ile, Val, Met, Nle, Trp, o-benzyl-Tyr, beta-Nal, Phe, o-X-Phe (wherein X represents H, $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, or pentafluoro-Phe;

$A^6$ represents Ala, pyridyl-Ala, Leu, Ile, Val, N-benzyloxycarbonyl-Lys, Met, Nle, Thr-$R^4$, Trp, Ser-$R^4$, beta-Nal, o-X-Phe (wherein X represents $CH_3$, Cl, Br, F, OH, $OCH_3$ or $NO_2$), p-X-Phe (wherein X = $CH_3$, Cl, Br, F, OH, $OCH_3$ or $NO_2$), 2,4-dichloro-Phe, or pentafluoro-Phe;

$A^7$ represents Ala, pyridyl-Ala, Leu, Ile, Val, Met, Nle, Trp, beta-Nal, Phe, o-X-Phe (wherein X represents $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, or pentafluoro-Phe;

$A^8$ represents a D- or L-isomer of Ala, pyridyl-Ala, Leu, Ile, Ser-$R^4$, Thr-$R^4$, Val, Met, Nle, Trp, beta-Nal, o-X-Phe (wherein X represents $CH_3$, Cl, Br, F, OH, $OCH_3$ or $NO_2$), p-X-Phe (wherein X = $CH_3$, Cl, Br, F, OH, $OCH_3$ or $NO_2$), 2,4-dichloro-Phe, or pentafluoro-Phe;

each of $R^1$ and $R^2$ individually represent a hydrogen atom, lower acyl or lower alkyl;

provided that at least one of $A^1$ and $A^8$ must be an aromatic amino acid; and further provided that if either $A^2$ or $A^7$ represents an aromatic amino acid, then $A^8$ cannot be an aromatic amino acid;

$R^4$ represents nothing, a protected glycosyl residue, or an o-benzyl group or a carbohydrate;

$R^6$ represents a protective group, e.g. Boc;

$R^7$ represents a resin;

or a pharmaceutically acceptable salt thereof;

the process comprising:

(a) reacting a resin with an amino acid residue with a side chain $A^8$ and a protecting group;

(b) reacting the resulting resin-bound compound with an amino acid residue with a side chain represented by $A^7$;

(c) repeating (b) with amino acids with side chains N-benzyloxycarbonyl-Lys, D-Trp, $A^3$, $A^2$ and $A^1$ consecutively; and

(d) where and when necessary, reacting the compound prepared, or one of the above residues with a glycosyl residue, a carbohydrate, an acylating agent or an alkylating agent.

**12.** The use of a compound having the general formula:

$$\begin{array}{l} R^1 \\ | \\ A^1 - A^2 - A^3 - \text{D-Trp} - \text{Lys} - A^6 - A^7 - A^8 - R^3 \\ | \\ R^2 \end{array}$$

wherein

$A^1$ represents a D-isomer of Ala, pyridyl-Ala, Leu, Ile, Val, Met, Nle, Trp, beta-Nal, Phe, o-X-Phe (wherein X represents $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, pentafluoro-Phe, or L-Phe;

$A^2$ represents Ala, pyridyl-Ala, Leu, Ile, Val, Met, Nle, Trp, beta-Nal, Phe, o-X-Phe (wherein X represents $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, or pentafluoro-Phe;

$A^3$ represents Ala, pyridyl-Ala, Leu, Ile, Val, Met, Nle, Trp, Tyr, beta-Nal, Phe, o-X-Phe (wherein X represents H, $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, or pentafluoro-Phe;

$A^6$ represents Ala, pyridyl-Ala, Leu, Ile, Val, Lys, Met, Nle, Thr-$R^4$, Trp, Ser-$R^4$, beta-Nal, o-X-Phe (wherein X represents $CH_3$, Cl, Br, F, OH, $OCH_3$ or $NO_2$), p-X-Phe (wherein X = $CH_3$, Cl, Br, F, OH, $OCH_3$ or $NO_2$), 2,4-dichloro-Phe, or pentafluoro-Phe;

$A^7$ represents Ala, pyridyl-Ala, Leu, Ile, Val, Met, Nle, Trp, beta-Nal, Phe, o-X-Phe (wherein X represents $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, or pentafluoro-Phe;

$A^8$ represents a D- or L-isomer of Ala, pyridyl-Ala, Leu, Ile, Ser-$R^4$, Thr-$R^4$, Val, Met, Nle, Trp, beta-Nal, o-X-Phe (wherein X represents $CH_3$, Cl, Br, F, OH, $OCH_3$ or $NO_2$), p-X-Phe (wherein X = $CH_3$, Cl, Br, F, OH, $OCH_3$ or $NO_2$), 2,4-dichloro-Phe, or pentafluoro-Phe;
each of $R^1$ and $R^2$ independently represent a hydrogen atom, lower acyl or lower alkyl;
$R^3$ represents a hydrogen atom, $-NH_2$ or loweralkyl group;
provided that at least one of $A^1$ and $A^8$ must be an aromatic amino acid; and further provided that if either $A^2$ or $A^7$ represents an aromatic amino acid, then $A^8$ cannot be an aromatic amino acid;
$R^4$ represents nothing, a protected glycosyl residue, or a carbohydrate;
or a pharmaceutically acceptable salt thereof;
in the preparation of an agent in the treatment or prophylaxis of cancer, acromegaly, hyposecretory endocrine disorders, diabetes, cirrhosis, ulcers, pancreatitis, diarrheoa, hepatitis, Alzheimer's disease, mushroom poisoning or retinopathy.

**Claims for the following Contracting State : ES**

**1.** A process for the preparation of a compound having the general formula:

$$\begin{array}{l} R^1 \\ | \\ A^1 - A^2 - A^3 - \text{D-Trp} - \text{Lys} - A^6 - A^7 - A^8 - R^3 \\ | \\ R^2 \end{array}$$

wherein
$A^1$ represents a D-isomer of Ala, pyridyl-Ala, Leu, Ile, Val, Met, Nle, Trp, beta-Nal, Phe, o-X-Phe (wherein X represents $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, pentafluoro-Phe, or L-Phe;
$A^2$ represents Ala, pyridyl-Ala, Leu, Ile, Val, Met, Nle, Trp, beta-Nal, Phe, o-X-Phe (wherein X represents $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, or pentafluoro-Phe;
$A^3$ represents Ala, pyridyl-Ala, Leu, Ile, Val, Met, Nle, Trp, Tyr, beta-Nal, Phe, o-X-Phe (wherein X represents H, $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, or pentafluoro-Phe;
$A^6$ represents Ala, pyridyl-Ala, Leu, Ile, Val, Lys, Met, Nle, Thr-$R^4$, Trp, Ser-$R^4$, beta-Nal, o-X-Phe (wherein X represents $CH_3$, Cl, Br, F, OH, $OCH_3$ or $NO_2$), p-X-Phe (wherein X = $CH_3$, Cl, Br, F, OH, $OCH_3$ or $NO_2$), 2,4-dichloro-Phe, or pentafluoro-Phe;
$A^7$ represents Ala, pyridyl-Ala, Leu, Ile, Val, Met, Nle, Trp, beta-Nal, Phe, o-X-Phe (wherein X represents $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, or pentafluoro-Phe;
$A^8$ represents a D- or L-isomer of any of Ala, pyridyl-Ala, Leu, Ile, Ser-$R^4$, Thr-$R^4$, Val, Met, Nle, Trp, beta-Nal, o-X-Phe (wherein X represents $CH_3$, Cl, Br, F, OH, $OCH_3$ or $NO_2$), p-X-Phe (wherein X = $CH_3$, Cl, Br, F, OH, $OCH_3$ or $NO_2$), 2,4-dichloro-Phe, or pentafluoro-Phe;
each of $R^1$ and $R^2$ independently represent a hydrogen atom, lower acyl or lower alkyl;
$R^3$ represents a hydrogen atom, $-NH_2$ or loweralkyl group;
provided that at least one of $A^1$ and $A^8$ must be an aromatic amino acid; and further provided that if either $A^2$ or $A^7$ represents aromatic amino acid, then $A^8$ cannot be an aromatic amino acid;
$R^4$ represents nothing, a protected glycosyl residue, or a carbohydrate;
or a pharmaceutically acceptable salt thereof;
the process comprising coupling successive amino acid residues together.

**2.** A process as claimed in claim 1, wherein one but not both $A^1$ and $A^2$ represent an aromatic amino acid; and wherein one but not both of $A^7$ and $A^8$ represent an aromatic amino acid.

**3.** A process as claimed in claim 1 or 2 wherein $A^1$ represents a D-isomer of Trp, beta-Nal, o-X-Phe (wherein X represents $CH_3$ or $OCH_3$), p-X-Phe (wherein X represents $CH_3$ of $OCH_3$); and

$A^8$ represents a D- or L-isomer of Ala, pyridyl-Ala, Leu, Ile, Ser, Thr, Val, Met, Nle, o-X-Phe (wherein X represents Cl, Br, F, OH or $NO_2$), p-X-Phe (wherein X represents Cl, Br, F, OH, or $NO_2$), 2,4-dichloro-Phe, or pentafluoro-Phe.

**4.** A process as claimed in any of claims 1 to 3, which has one, some or all of the following characteristics:
$A^1$ represents beta-D-Nal or D-Phe;
$A^2$ represents Ala, Phe or p-chloro-Phe;
$A^3$ represents Tyr or Phe;
$A^6$ represents Val, Lys or Thr;
$A^7$ represents Ala or Phe; and/or
$A^8$ represents Thr or beta-D-Nal.

**5.** A process as claimed in any of claims 1 to 4 which is:
D-Phe-Phe-Phe-D-Trp-Lys-Thr-Phe-Thr-$NH_2$ [SEQ.ID NO 3];
D-Phe-Phe-Tyr-D-Trp-Lys-Val-Phe-Thr-$NH_2$ [SEQ.ID NO 2];
D-Phe-p-chloro-Phe-Tyr-D-Trp-Lys-Val-Phe-Thr-$NH_2$ [SEQ.ID NO 1]; or
D-Phe-Ala-Tyr-D-Trp-Lys-Val-Ala-beta-D-Nal-$NH_2$ [SEQ.ID NO 4].

**6.** A process for the preparation of a compound of the general formula:

$$\begin{array}{l} R^1 \\ | \\ A^1 - A^2 - A^3 - D\text{-}Trp - Lys - A^6 - A^7 - A^8 - R^3 \\ | \\ R^2 \end{array}$$

wherein
$A^1$ represents a D-isomer of Ala, pyridyl-Ala, Leu, Ile, Val, Met, Nle, Trp, beta-Nal, Phe, o-X-Phe (wherein X represents $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, pentafluoro-Phe, or L-Phe;
$A^2$ represents Ala, pyridyl-Ala, Leu, Ile, Val, Met, Nle, Trp, beta-Nal, Phe, o-X-Phe (wherein X represents $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, or pentafluoro-Phe;
$A^3$ represents Ala, pyridyl-Ala, Leu, Ile, Val, Met, Nle, Trp, Tyr, beta-Nal, Phe, o-X-Phe (wherein X represents H, $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, or pentafluoro-Phe;
$A^6$ represents Ala, pyridyl-Ala, Leu, Ile, Val, Lys, Met, Nle, Thr-$R^4$, Trp, Ser-$R^4$, beta-Nal, o-X-Phe (wherein X represents $CH_3$, Cl, Br, F, OH, $OCH_3$ or $NO_2$), p-X-Phe (wherein X = $CH_3$, Cl, Br, F, OH, $OCH_3$ or $NO_2$), 2,4-dichloro-Phe, or pentafluoro-Phe;
$A^7$ represents Ala, pyridyl-Ala, Leu, Ile, Val, Met, Nle, Trp, beta-Nal, Phe, o-X-Phe (wherein X represents $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, or pentafluoro-Phe;
$A^8$ represents a D- or L-isomer of Ala, pyridyl-Ala, Leu, Ile, Ser-$R^4$, Thr-$R^4$, Val, Met, Nle, Trp, beta-Nal, o-X-Phe (wherein X represents $CH_3$, Cl, Br, F, OH, $OCH_3$ or $NO_2$), p-X-Phe (wherein X = $CH_3$, Cl, Br, F, OH, $OCH_3$ or $NO_2$), 2,4-dichloro-Phe, or pentafluoro-Phe;
each of $R^1$ and $R^2$ independently represent a hydrogen atom, lower acyl or lower alkyl;
$R^3$ represents a hydrogen atom, -$NH_2$ or loweralkyl group;
provided that at least one of $A^1$ and $A^8$ must be an aromatic amino acid; and further provided that if either $A^2$ or $A^7$ represents an aromatic amino acid, then $A^8$ cannot be an aromatic amino acid;
$R^4$ represents nothing, a protected glycosyl residue, or a carbohydrate;
or a pharmaceutically acceptable salt thereof;
the process comprising reacting a compound of the general formula:

$$\begin{array}{l} \quad R^1 \\ \quad | \\ R^6 - A^1 - A^2 - A^3 - \text{D-Trp} - \text{N-benzyloxycarboxyl} - \text{Lys} - \\ \quad | \\ \quad R^2 \qquad\qquad\qquad\qquad A^6 - A^7 - A^8 - R^7 \end{array}$$

wherein:
$R^1$, $R^2$, $A^1$, $A^2$, $A^7$ and $A^8$ are as defined before;
$A^3$ is as defined before except that Tyr is replaced by o-benzyl-Tyr;
$A^6$ is as defined before except that Lys is replaced by N-benzyloxycarbonyl-Lys;
$R^4$ can additionally represent an o-benzyl group;
$R^6$ represents a protective group; and
$R^7$ represents a resin;
with:
cresol;
dithiothreitol; and
hydrogen fluoride.

**7.** A process for the preparation of a compound of the general formula:

$$\begin{array}{l} \quad R^1 \\ \quad | \\ R^6 - A^1 - A^2 - A^3 - \text{D-Trp} - \text{N-benzyloxycarboxyl} - \text{Lys} - \\ \quad | \\ \quad R^2 \qquad\qquad\qquad\qquad A^6 - A^7 - A^8 - R^7 \end{array}$$

wherein:
$A^1$ represents a D-isomer of Ala, pyridyl-Ala, Leu, Ile, Val, Met, Nle, Trp, beta-Nal, Phe, o-X-Phe (wherein X represents $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, pentafluoro-Phe, or L-Phe;
$A^2$ represents Ala, pyridyl-Ala, Leu, Ile, Val, Met, Nle, Trp, beta-Nal, Phe, o-X-Phe (wherein X represents $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, or pentafluoro-Phe;
$A^3$ represents Ala, pyridyl-Ala, Leu, Ile, Val, Met, Nle, Trp, o-benzyl-Tyr, beta-Nal, Phe, o-X-Phe (wherein X represents H, $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, or pentafluoro-Phe;
$A^6$ represents Ala, pyridyl-Ala, Leu, Ile, Val, N-benzyloxycarbonyl-Lys, Met, Nle, Thr-$R^4$, Trp, Ser-$R^4$, beta-Nal, o-X-Phe (wherein X represents $CH_3$, Cl, Br, F, OH, $OCH_3$ or $NO_2$), p-X-Phe (wherein X = $CH_3$, Cl, Br, F, OH, $OCH_3$ or $NO_2$), 2,4-dichloro-Phe, or pentafluoro-Phe;
$A^7$ represents Ala, pyridyl-Ala, Leu, Ile, Val, Met, Nle, Trp, beta-Nal, Phe, o-X-Phe (wherein X represents $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, or pentafluoro-Phe;
$A^8$ represents a D- or L-isomer of Ala, pyridyl-Ala, Leu, Ile, Ser-$R^4$, Thr-$R^4$, Val, Met, Nle, Trp, beta-Nal, o-X-Phe (wherein X represents $CH_3$, Cl, Br, F, OH, $OCH_3$ or $NO_2$), p-X-Phe (wherein X = $CH_3$, Cl, Br, F, OH, $OCH_3$ or $NO_2$), 2,4-dichloro-Phe, or pentafluoro-Phe;
each of $R^1$ and $R^2$ individually represent a hydrogen atom, lower acyl or lower alkyl;
provided that at least one of $A^1$ and $A^8$ must be an aromatic amino acid; and further provided that if either $A^2$ or $A^7$ represents aromatic amino acid, then $A^8$ cannot be an aromatic amino acid;
$R^4$ represents nothing, a protected glycosyl residue, or an o-benzyl group or a carbohydrate;
$R^6$ represents a protective group, e.g. Boc;

$R^7$ represents a resin;
or a pharmaceutically acceptable salt thereof;
the process comprising:

(a) reacting a resin with an amino acid residue with a side chain $A^8$ and a protecting group;
(b) reacting the resulting resin-bound compound with an amino acid residue with a side chain represented by $A^7$;
(c) repeating (b) with amino acids with side chains $A^7$, N-benzyloxycarbonyl-Lys, D-Trp, $A^3$, $A^2$ and $A^1$; consecutively; and
(d) where and when necessary, reacting the compound prepared, or one of the above residues with a glycosyl residue, a carbohydrate, an acylating agent or an alkylating agent.

**8.** The use of a compound having the general formula:

$$\begin{array}{l} R^1 \\ | \\ A^1 - A^2 - A^3 - \text{D-Trp} - \text{Lys} - A^6 - A^7 - A^8 - R^3 \\ | \\ R^2 \end{array}$$

wherein
$A^1$ represents a D-isomer of Ala, pyridyl-Ala, Leu, Ile, Val, Met, Nle, Trp, beta-Nal, Phe, o-X-Phe (wherein X represents $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, pentafluoro-Phe, or L-Phe;
$A^2$ represents Ala, pyridyl-Ala, Leu, Ile, Val, Met, Nle, Trp, beta-Nal, Phe, o-X-Phe (wherein X represents $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, or pentafluoro-Phe;
$A^3$ represents Ala, pyridyl-Ala, Leu, Ile, Val, Met, Nle, Trp, Tyr, beta-Nal, Phe, o-X-Phe (wherein X represents H, $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, or pentafluoro-Phe;
$A^6$ represents Ala, pyridyl-Ala, Leu, Ile, Val, Lys, Met, Nle, Thr-$R^4$, Trp, Ser-$R^4$, beta-Nal, o-X-Phe (wherein X represents $CH_3$, Cl, Br, F, OH, $OCH_3$ or $NO_2$), p-X-Phe (wherein X = $CH_3$, Cl, Br, F, OH, $OCH_3$ or $NO_2$), 2,4-dichloro-Phe, or pentafluoro-Phe;
$A^7$ represents Ala, pyridyl-Ala, Leu, Ile, Val, Met, Nle, Trp, beta-Nal, Phe, o-X-Phe (wherein X represents $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, or pentafluoro-Phe;
$A^8$ represents a D- or L-isomer of Ala, pyridyl-Ala, Leu, Ile, Ser-$R^4$, Thr-$R^4$, Val, Met, Nle, Trp, beta-Nal, o-X-Phe (wherein X represents $CH_3$, Cl, Br, F, OH, $OCH_3$ or $NO_2$), p-X-Phe (wherein X = $CH_3$, Cl, Br, F, OH, $OCH_3$ or $NO_2$), 2,4-dichloro-Phe, or pentafluoro-Phe;
each of $R^1$ and $R^2$ independently represent a hydrogen atom, lower acyl or lower alkyl;
$R^3$ represents a hydrogen atom, $-NH_2$ or loweralkyl group;
provided that at least one of $A^1$ and $A^8$ must be an aromatic amino acid; and further provided that if either $A^2$ or $A^7$ represents an aromatic amino acid, then $A^8$ cannot be an aromatic amino acid;
$R^4$ represents nothing, a protected glycosyl residue, or a carbohydrate;
or a pharmaceutically acceptable salt thereof;
in the preparation of an agent in the treatment or prophylaxis of cancer, acromegaly, hyposecretory endocrine disorders, diabetes, cirrhosis, ulcers, pancreatitis, diarrhoea, hepatitis, Alzheimer's disease, mushroom poisoning or retinopathy.

**9.** A process for the preparation of a pharmaceutical composition, the process comprising admixing a compound of the general formula:

$$\begin{array}{l} R^1 \\ | \\ A^1 - A^2 - A^3 - \text{D-Trp} - \text{Lys} - A^6 - A^7 - A^8 - R^3 \\ | \\ R^2 \end{array}$$

wherein
$A^1$ represents a D-isomer of Ala, pyridyl-Ala, Leu, Ile, Val, Met, Nle, Trp, beta-Nal, Phe, o-X-Phe (wherein X represents $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, pentafluoro-Phe, or L-Phe;
$A^2$ represents Ala, pyridyl-Ala, Leu, Ile, Val, Met, Nle, Trp, beta-Nal, Phe, o-X-Phe (wherein X represents $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, or pentafluoro-Phe;
$A^3$ represents Ala, pyridyl-Ala, Leu, Ile, Val, Met, Nle, Trp, Tyr, beta-Nal, Phe, o-X-Phe (wherein X represents H, $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, or pentafluoro-Phe;
$A^6$ represents Ala, pyridyl-Ala, Leu, Ile, Val, Lys, Met, Nle, Thr-$R^4$, Trp, Ser-$R^4$, beta-Nal, o-X-Phe (wherein X represents $CH_3$, Cl, Br, F, OH, $OCH_3$ or $NO_2$), p-X-Phe (wherein X = $CH_3$, Cl, Br, F, OH, $OCH_3$ or $NO_2$), 2,4-dichloro-Phe, or pentafluoro-Phe;
$A^7$ represents Ala, pyridyl-Ala, Leu, Ile, Val, Met, Nle, Trp, beta-Nal, Phe, o-X-Phe (wherein X represents $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, or pentafluoro-Phe;
$A^8$ represents a D- or L-isomer of Ala, pyridyl-Ala, Leu, Ile, Ser-$R^4$, Thr-$R^4$, Val, Met, Nle, Trp, beta-Nal, o-X-Phe (wherein X represents $CH_3$, Cl, Br, F, OH, $OCH_3$ or $NO_2$), p-X-Phe (wherein X = $CH_3$, Cl, Br, F, OH, $OCH_3$ or $NO_2$), 2,4-dichloro-Phe, or pentafluoro-Phe;
each of $R^1$ and $R^2$ independently represent a hydrogen atom, lower acyl or lower alkyl;
$R^3$ represents a hydrogen atom, $-NH_2$ or loweralkyl group;
provided that at least one of $A^1$ and $A^8$ must be an aromatic amino acid; and further provided that if either $A^2$ or $A^7$ represents an aromatic amino acid, then $A^8$ cannot be an aromatic amino acid;
$R^4$ represents nothing, a protected glycosyl residue, or a carbohydrate;
or a pharmaceutically acceptable salt thereof;
the process comprising coupling successive amino acid residues together;
and a pharmaceutically acceptable carrier.

**10.** A process as claimed in claim 9 where the composition is coated with a substance capable of protecting the composition from the gastric acid in the stomach for a period of time sufficient to allow the composition to pass undisintegrated into the small intestine.

**Claims for the following Contracting State : GR**

**1.** A process for the preparation of a compound having the general formula:

$$\begin{array}{l} R^1 \\ | \\ A^1 - A^2 - A^3 - \text{D-Trp} - \text{Lys} - A^6 - A^7 - A^8 - R^3 \\ | \\ R^2 \end{array}$$

wherein
$A^1$ represents a D-isomer of Ala, pyridyl-Ala, Leu, Ile, Val, Met, Nle, Trp, beta-Nal, Phe, o-X-Phe

(wherein X represents $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, pentafluoro-Phe, or L-Phe;
$A^2$ represents Ala, pyridyl-Ala, Leu, Ile, Val, Met, Nle, Trp, beta-Nal, Phe, o-X-Phe (wherein X represents $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, or pentafluoro-Phe;
$A^3$ represents Ala, pyridyl-Ala, Leu, Ile, Val, Met, Nle, Trp, Tyr, beta-Nal, Phe, o-X-Phe (wherein X represents H, $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, or pentafluoro-Phe;
$A^6$ represents Ala, pyridyl-Ala, Leu, Ile, Val, Lys, Met, Nle, Thr-$R^4$, Trp, Ser-$R^4$, beta-Nal, o-X-Phe (wherein X represents $CH_3$, Cl, Br, F, OH, $OCH_3$ or $NO_2$), p-X-Phe (wherein X = $CH_3$, Cl, Br, F, OH, $OCH_3$ or $NO_2$), 2,4-dichloro-Phe, or pentafluoro-Phe;
$A^7$ represents Ala, pyridyl-Ala, Leu, Ile, Val, Met, Nle, Trp, beta-Nal, Phe, o-X-Phe (wherein X represents $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, or pentafluoro-Phe;
$A^8$ represents a D- or L-isomer of any of Ala, pyridyl-Ala, Leu, Ile, Ser-$R^4$, Thr-$R^4$, Val, Met, Nle, Trp, beta-Nal, o-X-Phe (wherein X represents $CH_3$, Cl, Br, F, OH, $OCH_3$ or $NO_2$), p-X-Phe (wherein X = $CH_3$, Cl, Br, F, OH, $OCH_3$ or $NO_2$), 2,4-dichloro-Phe, or pentafluoro-Phe;
each of $R^1$ and $R^2$ independently represent a hydrogen atom, lower acyl or lower alkyl;
$R^3$ represents a hydrogen atom, $-NH_2$ or loweralkyl group;
provided that at least one of $A^1$ and $A^8$ must be an aromatic amino acid; and further provided that if either $A^2$ or $A^7$ represents aromatic amino acid, then $A^8$ cannot be an aromatic amino acid;
$R^4$ represents nothing, a protected glycosyl residue, or a carbohydrate;
or a pharmaceutically acceptable salt thereof;
the process comprising coupling successive amino acid residues together.

**2.** A process as claimed in claim 1, wherein one but not both $A^1$ and $A^2$ represent an aromatic amino acid; and wherein one but not both of $A^7$ and $A^8$ represent an aromatic amino acid.

**3.** A process as claimed in claim 1 or 2 wherein $A^1$ represents a D-isomer of Trp, beta-Nal, o-X-Phe (wherein X represents $CH_3$ or $OCH_3$), p-X-Phe (wherein X represents $CH_3$ of $OCH_3$); and
$A^8$ represents a D- or L-isomer of Ala, pyridyl-Ala, Leu, Ile, Ser, Thr, Val, Met, Nle, o-X-Phe (wherein X represents Cl, Br, F, OH or $NO_2$), p-X-Phe (wherein X represents Cl, Br, F, OH, or $NO_2$), 2,4-dichloro-Phe, or pentafluoro-Phe.

**4.** A process as claimed in any of claims 1 to 3, which has one, some or all of the following characteristics:
$A^1$ represents beta-D-Nal or D-Phe;
$A^2$ represents Ala, Phe or p-chloro-Phe;
$A^3$ represents Tyr or Phe;
$A^6$ represents Val, Lys or Thr;
$A^7$ represents Ala or Phe; and/or
$A^8$ represents Thr or beta-D-Nal.

**5.** A process as claimed in any of claims 1 to 4 which is:
D-Phe-Phe-Phe-D-Trp-Lys-Thr-Phe-Thr-$NH_2$ [SEQ.ID NO 3];
D-Phe-Phe-Tyr-D-Trp-Lys-Val-Phe-Thr-$NH_2$ [SEQ.ID NO 2];
D-Phe-p-chloro-Phe-Tyr-D-Trp-Lys-Val-Phe-Thr-$NH_2$ [SEQ.ID NO 1]; or
D-Phe-Ala-Tyr-D-Trp-Lys-Val-Ala-beta-D-Nal-$NH_2$ [SEQ.ID NO 4].

**6.** A process for the preparation of a compound of the general formula:

$$\begin{array}{l} R^1 \\ | \\ A^1 - A^2 - A^3 - \text{D-Trp} - \text{Lys} - A^6 - A^7 - A^8 - R^3 \\ | \\ R^2 \end{array}$$

wherein
$A^1$ represents a D-isomer of Ala, pyridyl-Ala, Leu, Ile, Val, Met, Nle, Trp, beta-Nal, Phe, o-X-Phe (wherein X represents $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, pentafluoro-Phe, or L-Phe;
$A^2$ represents Ala, pyridyl-Ala, Leu, Ile, Val, Met, Nle, Trp, beta-Nal, Phe, o-X-Phe (wherein X represents $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, or pentafluoro-Phe;
$A^3$ represents Ala, pyridyl-Ala, Leu, Ile, Val, Met, Nle, Trp, Tyr, beta-Nal, Phe, o-X-Phe (wherein X represents H, $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, or pentafluoro-Phe;
$A^6$ represents Ala, pyridyl-Ala, Leu, Ile, Val, Lys, Met, Nle, Thr-$R^4$, Trp, Ser-$R^4$, beta-Nal, o-X-Phe (wherein X represents $CH_3$, Cl, Br, F, OH, $OCH_3$ or $NO_2$), p-X-Phe (wherein X = $CH_3$, Cl, Br, F, OH, $OCH_3$ or $NO_2$), 2,4-dichloro-Phe, or pentafluoro-Phe;
$A^7$ represents Ala, pyridyl-Ala, Leu, Ile, Val, Met, Nle, Trp, beta-Nal, Phe, o-X-Phe (wherein X represents $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, or pentafluoro-Phe;
$A^8$ represents a D- or L-isomer of Ala, pyridyl-Ala, Leu, Ile, Ser-$R^4$, Thr-$R^4$, Val, Met, Nle, Trp, beta-Nal, o-X-Phe (wherein X represents $CH_3$, Cl, Br, F, OH, $OCH_3$ or $NO_2$), p-X-Phe (wherein X = $CH_3$, Cl, Br, F, OH, $OCH_3$ or $NO_2$), 2,4-dichloro-Phe, or pentafluoro-Phe;
each of $R^1$ and $R^2$ independently represent a hydrogen atom, lower acyl or lower alkyl;
$R^3$ represents a hydrogen atom, -$NH_2$ or loweralkyl group;
provided that at least one of $A^1$ and $A^8$ must be an aromatic amino acid; and further provided that if either $A^2$ or $A^7$ represents an aromatic amino acid, then $A^8$ cannot be an aromatic amino acid;
$R^4$ represents nothing, a protected glycosyl residue, or a carbohydrate;
or a pharmaceutically acceptable salt thereof;
the process comprising reacting a compound of the general formula:

$$\begin{array}{l} \quad R^1 \\ \quad | \\ R^6 - A^1 - A^2 - A^3 - \text{D-Trp} - \text{N-benzyloxycarboxyl} - \text{Lys} - \\ \quad | \\ \quad R^2 \qquad\qquad\qquad\qquad\qquad A^6 - A^7 - A^8 - R^7 \end{array}$$

wherein:
$R^1$, $R^2$, $A^1$, $A^2$, $A^7$ and $A^8$ are as defined before;
$A^3$ is as defined before except that Tyr is replaced by o-benzyl-Tyr;
$A^6$ is as defined before except that Lys is replaced by N-benzyloxycarbonyl-Lys;
$R^4$ can additionally represent an o-benzyl group;
$R^6$ represents a protective group; and
$R^7$ represents a resin;
with:

cresol;

dithiothreitol; and
hydrogen fluoride.

**7.** A compound having the general formula:

$$\begin{array}{l} \quad R^1 \\ \quad | \\ R^6 - A^1 - A^2 - A^3 - \text{D-Trp} - \text{N-benzyloxycarboxyl} - \text{Lys} - \\ \quad | \\ \quad R^2 \qquad\qquad\qquad\qquad\qquad A^6 - A^7 - A^8 - R^7 \end{array}$$

wherein
$A^1$ represents a D-isomer of Ala, pyridyl-Ala, Leu, Ile, Val, Met, Nle, Trp, beta-Nal, Phe, o-X-Phe (wherein X represents $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, pentafluoro-Phe, or L-Phe;
$A^2$ represents Ala, pyridyl-Ala, Leu, Ile, Val, Met, Nle, Trp, beta-Nal, Phe, o-X-Phe (wherein X represents $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, or pentafluoro-Phe;
$A^3$ represents Ala, pyridyl-Ala, Leu, Ile, Val, Met, Nle, Trp, o-benzyl-Tyr, beta-Nal, Phe, o-X-Phe (wherein X represents H, $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, or pentafluoro-Phe;
$A^6$ represents Ala, pyridyl-Ala, Leu, Ile, Val, N-benzyloxycarbonyl-Lys, Met, Nle, Thr-$R^4$, Trp, Ser-$R^4$, beta-Nal, o-X-Phe (wherein X represents $CH_3$, Cl, Br, F, OH, $OCH_3$ or $NO_2$), p-X-Phe (wherein X = $CH_3$, Cl, Br, F, OH, $OCH_3$ or $NO_2$), 2,4-dichloro-Phe, or pentafluoro-Phe;
$A^7$ represents Ala, pyridyl-Ala, Leu, Ile, Val, Met, Nle, Trp, beta-Nal, Phe, o-X-Phe (wherein X represents $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, or pentafluoro-Phe;
$A^8$ represents a D- or L-isomer of Ala, pyridyl-Ala, Leu, Ile, Ser-$R^4$, Thr-$R^4$, Val, Met, Nle, Trp, beta-Nal, o-X-Phe (wherein X represents $CH_3$, Cl, Br, F, OH, $OCH_3$ or $NO_2$), p-X-Phe (wherein X = $CH_3$, Cl, Br, F, OH, $OCH_3$ or $NO_2$), 2,4-dichloro-Phe, or pentafluoro-Phe;
each of $R^1$ and $R^2$ individually represent a hydrogen atom, lower acyl or lower alkyl;
provided that at least one of $A^1$ and $A^8$ must be an aromatic amino acid; and further provided that if either $A^2$ or $A^7$ represents an aromatic amino acid, then $A^8$ cannot be an aromatic amino acid;
$R^4$ represents nothing, a protected glycosyl residue, or an o-benzyl group or a carbohydrate;
$R^6$ represents a protective group, e.g. Boc;
$R^7$ represents a resin;
or a pharmaceutically acceptable salt thereof.

**8.** A process for the preparation of a compound of the general formula:

$$\begin{array}{l} \quad R^1 \\ \quad | \\ R^6 - A^1 - A^2 - A^3 - \text{D-Trp} - \text{N-benzyloxycarboxyl} - \text{Lys} - \\ \quad | \\ \quad R^2 \qquad\qquad\qquad\qquad\qquad A^6 - A^7 - A^8 - R^7 \end{array}$$

wherein:
$A^1$ represents a D-isomer of Ala, pyridyl-Ala, Leu, Ile, Val, Met, Nle, Trp, beta-Nal, Phe, o-X-Phe (wherein X represents $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, pentafluoro-Phe, or L-Phe;

$A^2$ represents Ala, pyridyl-Ala, Leu, Ile, Val, Met, Nle, Trp, beta-Nal, Phe, o-X-Phe (wherein X represents $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, or pentafluoro-Phe;
$A^3$ represents Ala, pyridyl-Ala, Leu, Ile, Val, Met, Nle, Trp, o-benzyl-Tyr, beta-Nal, Phe, o-X-Phe (wherein X represents H, $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, or pentafluoro-Phe;
$A^6$ represents Ala, pyridyl-Ala, Leu, Ile, Val, N-benzyloxycarbonyl-Lys, Met, Nle, Thr-$R^4$, Trp, Ser-$R^4$, beta-Nal, o-X-Phe (wherein X represents $CH_3$, Cl, Br, F, OH, $OCH_3$ or $NO_2$), p-X-Phe (wherein X = $CH_3$, Cl, Br, F, OH, $OCH_3$ or $NO_2$), 2,4-dichloro-Phe, or pentafluoro-Phe;
$A^7$ represents Ala, pyridyl-Ala, Leu, Ile, Val, Met, Nle, Trp, beta-Nal, Phe, o-X-Phe (wherein X represents $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, or pentafluoro-Phe;
$A^8$ represents a D- or L-isomer of Ala, pyridyl-Ala, Leu, Ile, Ser-$R^4$, Thr-$R^4$, Val, Met, Nle, Trp, beta-Nal, o-X-Phe (wherein X represents $CH_3$, Cl, Br, F, OH, $OCH_3$ or $NO_2$), p-X-Phe (wherein X = $CH_3$, Cl, Br, F, OH, $OCH_3$ or $NO_2$), 2,4-dichloro-Phe, or pentafluoro-Phe;
each of $R^1$ and $R^2$ individually represent a hydrogen atom, lower acyl or lower alkyl;
provided that at least one of $A^1$ and $A^8$ must be an aromatic amino acid; and further provided that if either $A^2$ or $A^7$ represents aromatic amino acid, then $A^8$ cannot be an aromatic amino acid;
$R^4$ represents nothing, a protected glycosyl residue, or an o-benzyl group or a carbohydrate;
$R^6$ represents a protective group, e.g. Boc;
$R^7$ represents a resin;
or a pharmaceutically acceptable salt thereof;
the process comprising:

(a) reacting a resin with an amino acid residue with a side chain $A^8$ and a protecting group;
(b) reacting the resulting resin-bound compound with an amino acid residue with a side chain represented by $A^7$;
(c) repeating (b) with amino acids with side chains $A^7$, N-benzyloxycarbonyl-Lys, D-Trp, $A^3$, $A^2$ and $A^1$; consecutively; and
(d) where and when necessary, reacting the compound prepared, or one of the above residues with a glycosyl residue, a carbohydrate, an acylating agent or an alkylating agent.

**9.** The use of a compound having the general formula:

$$\begin{array}{l} R^1 \\ | \\ A^1 - A^2 - A^3 - \text{D-Trp} - \text{Lys} - A^6 - A^7 - A^8 - R^3 \\ | \\ R^2 \end{array}$$

wherein
$A^1$ represents a D-isomer of Ala, pyridyl-Ala, Leu, Ile, Val, Met, Nle, Trp, beta-Nal, Phe, o-X-Phe (wherein X represents $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, pentafluoro-Phe, or L-Phe;
$A^2$ represents Ala, pyridyl-Ala, Leu, Ile, Val, Met, Nle, Trp, beta-Nal, Phe, o-X-Phe (wherein X represents $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, or pentafluoro-Phe;
$A^3$ represents Ala, pyridyl-Ala, Leu, Ile, Val, Met, Nle, Trp, Tyr, beta-Nal, Phe, o-X-Phe (wherein X represents H, $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, or pentafluoro-Phe;
$A^6$ represents Ala, pyridyl-Ala, Leu, Ile, Val, Lys, Met, Nle, Thr-$R^4$, Trp, Ser-$R^4$, beta-Nal, o-X-Phe (wherein X represents $CH_3$, Cl, Br, F, OH, $OCH_3$ or $NO_2$), p-X-Phe (wherein X = $CH_3$, Cl, Br, F, OH, $OCH_3$ or $NO_2$), 2,4-dichloro-Phe, or pentafluoro-Phe;
$A^7$ represents Ala, pyridyl-Ala, Leu, Ile, Val, Met, Nle, Trp, beta-Nal, Phe, o-X-Phe (wherein X represents $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, or pentafluoro-Phe;

$A^8$ represents a D- or L-isomer of Ala, pyridyl-Ala, Leu, Ile, Ser-$R^4$, Thr-$R^4$, Val, Met, Nle, Trp, beta-Nal, o-X-Phe (wherein X represents $CH_3$, Cl, Br, F, OH, $OCH_3$ or $NO_2$), p-X-Phe (wherein X = $CH_3$, Cl, Br, F, OH, $OCH_3$ or $NO_2$), 2,4-dichloro-Phe, or pentafluoro-Phe;
each of $R^1$ and $R^2$ independently represent a hydrogen atom, lower acyl or lower alkyl;
$R^3$ represents a hydrogen atom, $-NH_2$ or loweralkyl group;
provided that at least one of $A^1$ and $A^8$ must be an aromatic amino acid; and further provided that if either $A^2$ or $A^7$ represents an aromatic amino acid, then $A^8$ cannot be an aromatic amino acid;
$R^4$ represents nothing, a protected glycosyl residue, or a carbohydrate;
or a pharmaceutically acceptable salt thereof;
in the preparation of an agent in the treatment or prophylaxis of cancer, acromegaly, hyposecretory endocrine disorders, diabetes, cirrhosis, ulcers, pancreatitis, diarrhoea, hepatitis, Alzheimer's disease, mushroom poisoning or retinopathy.

**10.** A process for the preparation of a pharmaceutical composition, the process comprising admixing a compound of the general formula:

$$\begin{array}{l} R^1 \\ | \\ A^1 - A^2 - A^3 - \text{D-Trp} - \text{Lys} - A^6 - A^7 - A^8 - R^3 \\ | \\ R^2 \end{array}$$

wherein
$A^1$ represents a D-isomer of Ala, pyridyl-Ala, Leu, Ile, Val, Met, Nle, Trp, beta-Nal, Phe, o-X-Phe (wherein X represents $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, pentafluoro-Phe, or L-Phe;
$A^2$ represents Ala, pyridyl-Ala, Leu, Ile, Val, Met, Nle, Trp, beta-Nal, Phe, o-X-Phe (wherein X represents $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, or pentafluoro-Phe;
$A^3$ represents Ala, pyridyl-Ala, Leu, Ile, Val, Met, Nle, Trp, Tyr, beta-Nal, Phe, o-X-Phe (wherein X represents H, $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, or pentafluoro-Phe;
$A^6$ represents Ala, pyridyl-Ala, Leu, Ile, Val, Lys, Met, Nle, Thr-$R^4$, Trp, Ser-$R^4$, beta-Nal, o-X-Phe (wherein X represents $CH_3$, Cl, Br, F, OH, $OCH_3$ or $NO_2$), p-X-Phe (wherein X = $CH_3$, Cl, Br, F, OH, $OCH_3$ or $NO_2$), 2,4-dichloro-Phe, or pentafluoro-Phe;
$A^7$ represents Ala, pyridyl-Ala, Leu, Ile, Val, Met, Nle, Trp, beta-Nal, Phe, o-X-Phe (wherein X represents $CH_3$, Cl, Br, F, OH, $OCH_3$ or $NO_2$), p-X-Phe (wherein X = H, $CH_3$, Cl, Br, F, OH, $OCH_3$ or $NO_2$), 2,4-dichloro-Phe, or pentafluoro-Phe;
$A^8$ represents a D- or L-isomer of Ala, pyridyl-Ala, Leu, Ile, Ser-$R^4$, Thr-$R^4$, Val, Met, Nle, Trp, beta-Nal, o-X-Phe (wherein X represents $CH_3$, Cl, Br, F, OH, $OCH_3$ or $NO_2$), p-X-Phe (wherein X = $CH_3$, Cl, Br, F, OH, $OCH_3$ or $NO_2$), 2,4-dichloro-Phe, or pentafluoro-Phe;
each of $R^1$ and $R^2$ independently represent a hydrogen atom, lower acyl or lower alkyl;
$R^3$ represents a hydrogen atom, $-NH_2$ or loweralkyl group;
provided that at least one of $A^1$ and $A^8$ must be an aromatic amino acid; and further provided that if either $A^2$ or $A^7$ represents an aromatic amino acid, then $A^8$ cannot be an aromatic amino acid;
$R^4$ represents nothing, a protected glycosyl residue, or a carbohydrate;
or a pharmaceutically acceptable salt thereof;
the process comprising coupling successive amino acid residues together;
and a pharmaceutically acceptable carrier.

**11.** A process as claimed in claim 9 where the composition is coated with a substance capable of protecting the composition from the gastric acid in the stomach for a period of time sufficient to allow the composition to pass undisintegrated into the small intestine.

**Patentansprüche**
**Patentansprüche für folgende Vertragsstaaten : AT, BE, CH, DE, DK, FR, GB, IT, LI, LU, NL, SE**

1. Verbindung mit der allgemeinen Formel:

$$\begin{array}{l} R^1 \\ | \\ A^1 - A^2 - A^3 - \text{D-Trp} - \text{Lys} - A^6 - A^7 - A^8 - R^3 \\ | \\ R^2 \end{array}$$

worin

$A^1$ ein D-Isomer von Ala, Pyridyl-Ala, Leu, Ile, Val, Met, Nle, Trp, Beta-Nal, Phe, o-X-Phe (worin X $CH_3$, Cl, Br, F, OH, $OCH_3$ oder $NO_2$ darstellt), p-X-Phe (worin X = H, $CH_3$, Cl, Br, F, OH, $OCH_3$ oder $NO_2$), 2,4-Dichloro-Phe, Pentafluoro-Phe oder L-Phe darstellt;

$A^2$ Ala, Pyridyl-Ala, Leu, Ile, Val, Met, Nle, Trp, Beta-Nal, Phe, o-X-Phe (worin X $CH_3$, Cl, Br, F, OH, $OCH_3$ oder $NO_2$ darstellt), p-X-Phe (worin X = H, $CH_3$, Cl, Br, F, OH, $OCH_3$ oder $NO_2$), 2,4-Dichloro-Phe oder Pentafluoro-Phe darstellt;

$A^3$ Ala, Pyridyl-Ala, Leu, Ile, Val, Met, Nle, Trp, Tyr, Beta-Nal, Phe, o-X-Phe (worin X H, $CH_3$, Cl, Br, F, OH, $OCH_3$ oder $NO_2$ darstellt), p-X-Phe (worin X = H, $CH_3$, Cl, Br, F, OH, $OCH_3$ oder $NO_2$), 2,4-Dichloro-Phe oder Pentafluoro-Phe darstellt;

$A^6$ Ala, Pyridyl-Ala, Leu, Ile, Val, Lys, Met, Nle, Thr-$R^4$, Trp, Ser-$R^4$, Beta-Nal, o-X-Phe (worin X $CH_3$, Cl, Br, F, OH, $OCH_3$ oder $NO_2$ darstellt), p-X-Phe (worin X = $CH_3$, Cl, Br, F, OH, $OCH_3$ oder $NO_2$), 2,4-Dichloro-Phe oder Pentafluoro-Phe darstellt;

$A^7$ Ala, Pyridyl-Ala, Leu, Ile, Val, Met, Nle, Trp, Beta-Nal, Phe, o-X-Phe (worin X $CH_3$, Cl, Br, F, OH, $OCH_3$ oder $NO_2$ darstellt), p-X-Phe (worin X = H, $CH_3$, Cl, Br, F, OH, $OCH_3$ oder $NO_2$), 2,4-Dichloro-Phe oder Pentafluoro-Phe darstellt;

$A^8$ ein D- oder L-Isomer irgendeines von Ala, Pyridyl-Ala, Leu, Ile, Ser-$R^4$, Thr-$R^4$, Val, Met, Nle, Trp, Beta-Nal, o-X-Phe (worin X $CH_3$, Cl, Br, F, OH, $OCH_3$ oder $NO_2$ darstellt), p-X-Phe (worin X = $CH_3$, Cl, Br, F, OH, $OCH_3$ oder $NO_2$), 2,4-Dichloro-Phe oder Pentafluoro-Phe darstellt;

jedes von $R^1$ und $R^2$ unabhängig ein Wasserstoffatom, niedriges Acyl oder niedriges Alkyl darstellt;

$R^3$ ein Wasserstoffatom, ein -$NH_2$ oder eine niedrige Alkylgruppe darstellt;

vorausgesetzt, daß mindestens eines von $A^1$ und $A^8$ eine aromatische Aminosäure sein muß; und weiters vorausgesetzt, daß, wenn entweder $A^2$ oder $A^7$ eine aromatische Aminosäure darstellt, $A^8$ keine aromatische Aminosäure sein kann;

$R^4$ nichts, einen geschützten Glycosyl-Rest oder ein Kohlenhydrat darstellt;

oder ein pharmazeutisch verträgliches Salz davon.

2. Verbindung nach Anspruch 1, worin eines jedoch nicht beide $A^1$ und $A^2$ eine aromatische Aminosäure darstellen; und worin eines jedoch nicht beide $A^7$ und $A^8$ eine aromatische Aminosäure darstellen.

3. Verbindung nach Anspruch 1 oder 2, worin $A^1$ ein D-Isomer von Trp, Beta-Nal, o-X-Phe (worin X $CH_3$ oder $OCH_3$ darstellt), p-X-Phe (worin X $CH_3$ oder $OCH_3$ darstellt) darstellt; und

$A^8$ ein D- oder L-Isomer von Ala, Pyridyl-Ala, Leu, Ile, Ser, Thr, Val, Met, Nle, o-X-Phe (worin X Cl, Br, F, OH oder $NO_2$ darstellt), p-X-Phe (worin X Cl, Br, F, OH oder $NO_2$ darstellt), 2,4-Dichloro-Phe oder Pentafluoro-Phe darstellt;

4. Verbindung nach einem der Ansprüche 1 bis 3, welche eine, einige oder alle der folgenden Eigenschaften hat:

$A^1$ zeigt Beta-D-Nal oder D-Phe;

$A^2$ zeigt Ala, Phe oder p-Chloro-Phe;

$A^3$ zeigt Tyr oder Phe;

$A^6$ zeigt Val, Lys oder Thr;

$A^7$ zeigt Ala oder Phe; und/oder
$A^8$ zeigt Thr oder Beta-D-Nal.

**5.** Verbindung nach einem der Ansprüche 1 bis 4, welche ist:
D-Phe-Phe-Phe-D-Trp-Lys-Thr-Phe-Thr-$NH_2$ [SEQ.ID NO 3];
D-Phe-Phe-Tyr-D-Trp-Lys-Val-Phe-Thr-$NH_2$ [SEQ.ID NO 2];
D-Phe-p-chloro-Phe-Tyr-D-Trp-Lys-Val-Phe-Thr-$NH_2$ [SEQ.ID NO 1]; oder
D-Phe-Ala-Tyr-D-Trp-Lys-Val-Ala-beta-D-Nal-$NH_2$ [SEQ.ID NO 4].

**6.** Verbindung mit der allgemeinen Formel:

$$\begin{array}{l} R^1 \\ | \\ A^1 - A^2 - A^3 - \text{D-Trp} - \text{Lys} - A^6 - A^7 - A^8 - R^3 \\ | \\ R^2 \end{array}$$

worin

$A^1$ ein D-Isomer von Ala, Pyridyl-Ala, Leu, Ile, Val, Met, Nle, Trp, Beta-Nal, Phe, o-X-Phe (worin X $CH_3$, Cl, Br, F, OH, $OCH_3$ oder $NO_2$ darstellt), p-X-Phe (worin X = H, $CH_3$, Cl, Br, F, OH, $OCH_3$ oder $NO_2$), 2,4-Dichloro-Phe, Pentafluoro-Phe oder L-Phe darstellt;

$A^2$ Ala, Pyridyl-Ala, Leu, Ile, Val, Met, Nle, Trp, Beta-Nal, Phe, o-X-Phe (worin X $CH_3$, Cl, Br, F, OH, $OCH_3$ oder $NO_2$ darstellt), p-X-Phe (worin X = H, $CH_3$, Cl, Br, F, OH, $OCH_3$ oder $NO_2$), 2,4-Dichloro-Phe oder Pentafluoro-Phe darstellt;

$A^3$ Ala, Pyridyl-Ala, Leu, Ile, Val, Met, Nle, Trp, Tyr, Beta-Nal, Phe, o-X-Phe (worin X H, $CH_3$, Cl, Br, F, OH, $OCH_3$ oder $NO_2$ darstellt), p-X-Phe (worin X = H, $CH_3$, Cl, Br, F, OH, $OCH_3$ oder $NO_2$), 2,4-Dichloro-Phe oder Pentafluoro-Phe darstellt;

$A^6$ Ala, Pyridyl-Ala, Leu, Ile, Val, Lys, Met, Nle, Thr-$R^4$, Trp, Ser-$R^4$, Beta-Nal, o-X-Phe (worin X $CH_3$, Cl, Br, F, OH, $OCH_3$ oder $NO_2$ darstellt), p-X-Phe (worin X = $CH_3$, Cl, Br, F, OH, $OCH_3$ oder $NO_2$), 2,4-Dichloro-Phe oder Pentafluoro-Phe darstellt;

$A^7$ Ala, Pyridyl-Ala, Leu, Ile, Val, Met, Nle, Trp, Beta-Nal, Phe, o-X-Phe (worin X $CH_3$, Cl, Br, F, OH, $OCH_3$ oder $NO_2$ darstellt), p-X-Phe (worin X = H, $CH_3$, Cl, Br, F, OH, $OCH_3$ oder $NO_2$), 2,4-Dichloro-Phe oder Pentafluoro-Phe darstellt;

$A^8$ ein D- oder L-Isomer irgendeines von Ala, Pyridyl-Ala, Leu, Ile, Ser-$R^4$, Thr-$R^4$, Val, Met, Nle, Trp, Beta-Nal, o-X-Phe (worin X $CH_3$, Cl, Br, F, OH, $OCH_3$ oder $NO_2$ darstellt), p-X-Phe (worin X = $CH_3$, Cl, Br, F, OH, $OCH_3$ oder $NO_2$), 2,4-Dichloro-Phe oder Pentafluoro-Phe darstellt;

jedes von $R^1$ und $R^2$ unabhängig ein Wasserstoffatom, niedriges Acyl oder niedriges Alkyl darstellt;

$R^3$ ein Wasserstoffatom, -$NH_2$ oder niedrige Alkylgruppe darstellt;

vorausgesetzt, daß mindestens eines von $A^1$ und $A^8$ eine aromatische Aminosäure sein muß; und weiters vorausgesetzt, daß, wenn entweder $A^2$ oder $A^7$ eine aromatische Aminosäure darstellt, $A^8$ keine aromatische Aminosäure sein kann;

$R^4$ nichts, einen geschützten Glycosyl-Rest oder ein Kohlenhydrat darstellt;

oder ein pharmazeutisch verträgliches Salz davon;

zur Verwendung in der Medizin.

**7.** Pharmazeutische Zusammensetzung, umfassend eine Verbindung mit der allgemeinen Formel:

$$\begin{array}{l} R^1 \\ | \\ A^1 - A^2 - A^3 - \text{D-Trp} - \text{Lys} - A^6 - A^7 - A^8 - R^3 \\ | \\ R^2 \end{array}$$

worin

$A^1$ ein D-Isomer von Ala, Pyridyl-Ala, Leu, Ile, Val, Met, Nle, Trp, Beta-Nal, Phe, o-X-Phe (worin X $CH_3$, Cl, Br, F, OH, $OCH_3$ oder $NO_2$ darstellt), p-X-Phe (worin X = H, $CH_3$, Cl, Br, F, OH, $OCH_3$ oder $NO_2$), 2,4-Dichloro-Phe, Pentafluoro-Phe oder L-Phe darstellt;

$A^2$ Ala, Pyridyl-Ala, Leu, Ile, Val, Met, Nle, Trp, Beta-Nal, Phe, o-X-Phe (worin X $CH_3$, Cl, Br, F, OH, $OCH_3$ oder $NO_2$ darstellt), p-X-Phe (worin X = H, $CH_3$, Cl, Br, F, OH, $OCH_3$ oder $NO_2$), 2,4-Dichloro-Phe oder Pentafluoro-Phe darstellt;

$A^3$ Ala, Pyridyl-Ala, Leu, Ile, Val, Met, Nle, Trp, Tyr, Beta-Nal, Phe, o-X-Phe (worin X H, $CH_3$, Cl, Br, F, OH, $OCH_3$ oder $NO_2$ darstellt), p-X-Phe (worin X = H, $CH_3$, Cl, Br, F, OH, $OCH_3$ oder $NO_2$), 2,4-Dichloro-Phe oder Pentafluoro-Phe darstellt;

$A^6$ Ala, Pyridyl-Ala, Leu, Ile, Val, Lys, Met, Nle, Thr-$R^4$, Trp, Ser-$R^4$, Beta-Nal, o-X-Phe (worin X $CH_3$, Cl, Br, F, OH, $OCH_3$ oder $NO_2$ darstellt), p-X-Phe (worin X = $CH_3$, Cl, Br, F, OH, $OCH_3$ oder $NO_2$), 2,4-Dichloro-Phe oder Pentafluoro-Phe darstellt;

$A^7$ Ala, Pyridyl-Ala, Leu, Ile, Val, Met, Nle, Trp, Beta-Nal, Phe, o-X-Phe (worin X $CH_3$, Cl, Br, F, OH, $OCH_3$ oder $NO_2$ darstellt), p-X-Phe (worin X = H, $CH_3$, Cl, Br, F, OH, $OCH_3$ oder $NO_2$), 2,4-Dichloro-Phe oder Pentafluoro-Phe darstellt;

$A^8$ ein D- oder L-Isomer irgendeines von Ala, Pyridyl-Ala, Leu, Ile, Ser-$R^4$, Thr-$R^4$, Val, Met, Nle, Trp, Beta-Nal, o-X-Phe (worin X $CH_3$, Cl, Br, F, OH, $OCH_3$ oder $NO_2$ darstellt), p-X-Phe (worin X = $CH_3$, Cl, Br, F, OH, $OCH_3$ oder $NO_2$), 2,4-Dichloro-Phe oder Pentafluoro-Phe darstellt;

jedes von $R^1$ und $R^2$ unabhängig ein Wasserstoffatom, niedriges Acyl oder niedriges Alkyl darstellt;

$R^3$ ein Wasserstoffatom, -$NH_2$ oder niedrige Alkylgruppe darstellt;

vorausgesetzt, daß mindestens eines von $A^1$ und $A^8$ eine aromatische Aminosäure sein muß; und weiters vorausgesetzt, daß, wenn entweder $A^2$ oder $A^7$ eine aromatische Aminosäure darstellt, $A^8$ keine aromatische Aminosäure sein kann;

$R^4$ nichts, einen geschützten Glycosyl-Rest oder ein Kohlenhydrat darstellt;

oder ein pharmazeutisch verträgliches Salz davon,

und einen pharmazeutisch verträglichen Träger.

**8.** Verbindung nach Anspruch 7, die mit einer Substanz umhüllt ist, welche imstande ist, die Zusammensetzung vor der Magensäure im Magen einen ausreichenden Zeitraum zu schützen, in welchem die Verbindung unaufgelöst in den Dünndarm gelangen kann.

**9.** Verfahren zur Herstellung einer Verbindung mit der allgemeinen Formel:

$$\begin{array}{l} R^1 \\ | \\ A^1 - A^2 - A^3 - \text{D-Trp} - \text{Lys} - A^6 - A^7 - A^8 - R^3 \\ | \\ R^2 \end{array}$$

worin

$A^1$ ein D-Isomer von Ala, Pyridyl-Ala, Leu, Ile, Val, Met, Nle, Trp, Beta-Nal, Phe, o-X-Phe (worin X

$CH_3$, Cl, Br, F, OH, $OCH_3$ oder $NO_2$ darstellt), p-X-Phe (worin X = H, $CH_3$, Cl, Br, F, OH, $OCH_3$ oder $NO_2$), 2,4-Dichloro-Phe, Pentafluoro-Phe oder L-Phe darstellt;

$A^2$ Ala, Pyridyl-Ala, Leu, Ile, Val, Met, Nle, Trp, Beta-Nal, Phe, o-X-Phe (worin X $CH_3$, Cl, Br, F, OH, $OCH_3$ oder $NO_2$ darstellt), p-X-Phe (worin X = H, $CH_3$, Cl, Br, F, OH, $OCH_3$ oder $NO_2$), 2,4-Dichloro-Phe oder Pentafluoro-Phe darstellt;

$A^3$ Ala, Pyridyl-Ala, Leu, Ile, Val, Met, Nle, Trp, Tyr, Beta-Nal, Phe, o-X-Phe (worin X H, $CH_3$, Cl, Br, F, OH, $OCH_3$ oder $NO_2$ darstellt), p-X-Phe (worin X = H, $CH_3$, Cl, Br, F, OH, $OCH_3$ oder $NO_2$), 2,4-Dichloro-Phe oder Pentafluoro-Phe darstellt;

$A^6$ Ala, Pyridyl-Ala, Leu, Ile, Val, Lys, Met, Nle, Thr-$R^4$, Trp, Ser-$R^4$,

Beta-Nal, o-X-Phe (worin X $CH_3$, Cl, Br, F, OH, $OCH_3$ oder $NO_2$ darstellt), p-X-Phe (worin X = $CH_3$, Cl, Br, F, OH, $OCH_3$ oder $NO_2$), 2,4-Dichloro-Phe oder Pentafluoro-Phe darstellt;

$A^7$ Ala, Pyridyl-Ala, Leu, Ile, Val, Met, Nle, Trp, Beta-Nal, Phe, o-X-Phe (worin X $CH_3$, Cl, Br, F, OH, $OCH_3$ oder $NO_2$ darstellt), p-X-Phe (worin X = H, $CH_3$, Cl, Br, F, OH, $OCH_3$ oder $NO_2$), 2,4-Dichloro-Phe oder Pentafluoro-Phe darstellt;

$A^8$ ein D- oder L-Isomer irgendeines von Ala, Pyridyl-Ala, Leu, Ile, Ser-$R^4$, Thr-$R^4$, Val, Met, Nle, Trp, Beta-Nal, o-X-Phe (worin X $CH_3$, Cl, Br, F, OH, $OCH_3$ oder $NO_2$ darstellt), p-X-Phe (worin X = $CH_3$, Cl, Br, F, OH, $OCH_3$ oder $NO_2$), 2,4-Dichloro-Phe oder Pentafluoro-Phe darstellt;

jedes von $R^1$ und $R^2$ unabhängig ein Wasserstoffatom, niedriges Acyl oder niedriges Alkyl darstellt;

$R^3$ ein Wasserstoffatom, $-NH_2$ oder niedrige Alkylgruppe darstellt;

vorausgesetzt, daß mindestens eines von $A^1$ und $A^8$ eine aromatische Aminosäure sein muß; und weiters vorausgesetzt, daß, wenn entweder $A^2$ oder $A^7$ eine aromatische Aminosäure darstellt, $A^8$ keine aromatische Aminosäure sein kann;

$R^4$ nichts, einen geschützten Glycosyl-Rest oder ein Kohlenhydrat darstellt;

oder ein pharmazeutisch verträgliches Salz davon;

welches Verfahren eine Reaktion umfaßt einer Verbindung mit der allgemeinen Formel:

$R^1$
|
$R^6$ - $A^1$ -$A^2$ - $A^3$ - D-Trp - N-Benzyloxycarboxyl - Lys -
|
$R^2$ $A^6$ - $A^7$ - $A^8$ - $R^7$

worin:

$R^1$, $R^2$, $A^1$, $A^2$, $A^7$ und $A^8$ wie vorher definiert sind;

$A^3$ wie vorher definiert ist, außer daß Tyr durch o-Benzyl-Tyr ersetzt ist;

$A^6$ wie vorher definiert ist, außer daß Lys durch N-Benzyloxycarbonyl-Lys ersetzt ist;

$R^4$ zusätzlich eine o-Benzylgruppe darstellen kann;

$R^6$ eine Schutzgruppe darstellt; und

$R^7$ ein Harz darstellt;

mit:

Kresol;

Dithiothreitol; und

Fluorwasserstoff.

**10.** Verbindung mit der allgemeinen Formel:

$$\begin{array}{l} R^1 \\ | \\ R^6 - A^1 - A^2 - A^3 - \text{D-Trp} - \text{N-Benzyloxycarboxyl} - \text{Lys} - \\ | \\ R^2 \qquad\qquad\qquad A^6 - A^7 - A^8 - R^7 \end{array}$$

worin

$A^1$ ein D-Isomer von Ala, Pyridyl-Ala, Leu, Ile, Val, Met, Nle, Trp, Beta-Nal, Phe, o-X-Phe (worin X $CH_3$, Cl, Br, F, OH, $OCH_3$ oder $NO_2$darstellt), p-X-Phe (worin X = H, $CH_3$, Cl, Br, F, OH, $OCH_3$ oder $NO_2$), 2,4-Dichloro-Phe, Pentafluoro-Phe oder L-Phe darstellt;

$A^2$ Ala, Pyridyl-Ala, Leu, Ile, Val, Met, Nle, Trp, Beta-Nal, Phe, o-X-Phe (worin X $CH_3$, Cl, Br, F, OH, $OCH_3$ oder $NO_2$ darstellt), p-X-Phe (worin X = H, $CH_3$, Cl, Br, F, OH, $OCH_3$ oder $NO_2$), 2,4-Dichloro-Phe oder Pentafluoro-Phe darstellt;

$A^3$ Ala, Pyridyl-Ala, Leu, Ile, Val, Met, Nle, Trp, o-Benzyl-Tyr, Beta-Nal, Phe, o-X-Phe (worin X H, $CH_3$, Cl, Br, F, OH, $OCH_3$ oder $NO_2$ darstellt), p-X-Phe (worin X = H, $CH_3$, Cl, Br, F, OH, $OCH_3$ oder $NO_2$), 2,4-Dichloro-Phe oder Pentafluoro-Phe darstellt;

$A^6$ Ala, Pyridyl-Ala, Leu, Ile, Val, N-Benzyloxycarbonyl-Lys, Met, Nle, Thr-$R^4$, Trp, Ser-$R^4$, Beta-Nal, o-X-Phe (worin X $CH_3$, Cl, Br, F, OH, $OCH_3$ oder $NO_2$ darstellt), p-X-Phe (worin X = $CH_3$, Cl, Br, F, OH, $OCH_3$ oder $NO_2$), 2,4-Dichloro-Phe oder Pentafluoro-Phe darstellt;

$A^7$ Ala, Pyridyl-Ala, Leu, Ile, Val, Met, Nle, Trp, Beta-Nal, Phe, o-X-Phe (worin X $CH_3$, Cl, Br, F, OH, $OCH_3$ oder $NO_2$ darstellt), p-X-Phe (worin X = H, $CH_3$, Cl, Br, F, OH, $OCH_3$ oder $NO_2$), 2,4-Dichloro-Phe oder Pentafluoro-Phe darstellt;

$A^8$ ein D- oder L-Isomer von Ala, Pyridyl-Ala, Leu, Ile, Ser-$R^4$, Thr-$R^4$, Val, Met, Nle, Trp, Beta-Nal, o-X-Phe (worin X $CH_3$, Cl, Br, F, OH, $OCH_3$ oder $NO_2$ darstellt), p-X-Phe (worin X = $CH_3$, Cl, Br, F, OH, $OCH_3$ oder $NO_2$), 2,4-Dichloro-Phe oder Pentafluoro-Phe darstellt;

jedes von $R^1$ und $R^2$ einzeln ein Wasserstoffatom, niedriges Acyl oder niedriges Alkyl darstellt;

vorausgesetzt, daß mindestens eines von $A^1$ und $A^8$ eine aromatische Aminosäure sein muß; und weiters vorausgesetzt, daß, wenn entweder $A^2$ oder $A^7$ eine aromatische Aminosäure darstellt, $A^8$ keine aromatische Aminosäure sein kann;

$R^4$ nichts, einen geschützten Glycosyl-Rest, oder eine o-Benzylgruppe oder ein Kohlenhydrat darstellt;

$R^6$ eine Schutzgruppe darstellt, z.B. Boc;

$R^7$ ein Harz darstellt;

oder ein pharmazeutisch verträgliches Salz davon.

**11.** Verfahren zur Herstellung einer Verbindung mit der allgemeinen Formel:

$$\begin{array}{l} R^1 \\ | \\ R^6 - A^1 - A^2 - A^3 - \text{D-Trp} - \text{N-Benzyloxycarboxyl} - \text{Lys} - \\ | \\ R^2 \qquad\qquad\qquad A^6 - A^7 - A^8 - R^7 \end{array}$$

worin

$A^1$ ein D-Isomer von Ala, Pyridyl-Ala, Leu, Ile, Val, Met, Nle, Trp, Beta-Nal, Phe, o-X-Phe (worin X $CH_3$, Cl, Br, F, OH, $OCH_3$ oder $NO_2$ darstellt), p-X-Phe (worin X = H, $CH_3$, Cl, Br, F, OH, $OCH_3$ oder $NO_2$), 2,4-Dichloro-Phe, Pentafluoro-Phe oder L-Phe darstellt;

$A^2$ Ala, Pyridyl-Ala, Leu, Ile, Val, Met, Nle, Trp, Beta-Nal, Phe, o-X-Phe (worin X $CH_3$, Cl, Br, F, OH,

$OCH_3$ oder $NO_2$ darstellt), p-X-Phe (worin X = H, $CH_3$, Cl, Br, F, OH, $OCH_3$ oder $NO_2$), 2,4-Dichloro-Phe oder Pentafluoro-Phe darstellt;

$A^3$ Ala, Pyridyl-Ala, Leu, Ile, Val, Met, Nle, Trp, o-Benzyl-Tyr, Beta-Nal, Phe, o-X-Phe (worin X H, $CH_3$, Cl, Br, F, OH, $OCH_3$ oder $NO_2$ darstellt), p-X-Phe (worin X = H, $CH_3$, Cl, Br, F, OH, $OCH_3$ oder $NO_2$), 2,4-Dichloro-Phe oder Pentafluoro-Phe darstellt;

$A^6$ Ala, Pyridyl-Ala, Leu, Ile, Val, N-Benzyloxycarbonyl-Lys, Met, Nle, Thr-$R^4$, Trp, Ser-$R^4$, Beta-Nal, o-X-Phe (worin X $CH_3$, Cl, Br, F, OH, $OCH_3$ oder $NO_2$ darstellt), p-X-Phe (worin X = $CH_3$, Cl, Br, F, OH, $OCH_3$ oder $NO_2$), 2,4-Dichloro-Phe oder Pentafluoro-Phe darstellt;

$A^7$ Ala, Pyridyl-Ala, Leu, Ile, Val, Met, Nle, Trp, Beta-Nal, Phe, o-X-Phe (worin X $CH_3$, Cl, Br, F, OH, $OCH_3$ oder $NO_2$ darstellt), p-X-Phe (worin X = H, $CH_3$, Cl, Br, F, OH, $OCH_3$ oder $NO_2$), 2,4-Dichloro-Phe oder Pentafluoro-Phe darstellt;

$A^8$ ein D- oder L-Isomer von Ala, Pyridyl-Ala, Leu, Ile, Ser-$R^4$, Thr-$R^4$, Val, Met, Nle, Trp, Beta-Nal, o-X-Phe (worin X $CH_3$, Cl, Br, F, OH, $OCH_3$ oder $NO_2$ darstellt), p-X-Phe (worin X = $CH_3$, Cl, Br, F, OH, $OCH_3$ oder $NO_2$), 2,4-Dichloro-Phe oder Pentafluoro-Phe darstellt;

jedes von $R^1$ und $R^2$ einzeln ein Wasserstoffatom, niedriges Acyl oder niedriges Alkyl darstellt;

vorausgesetzt, daß mindestens eines von $A^1$ und $A^8$ eine aromatische Aminosäure sein muß; und weiters vorausgesetzt, daß, wenn entweder $A^2$ oder $A^7$ eine aromatische Aminosäure darstellt, $A^8$ keine aromatische Aminosäure sein kann;

$R^4$ nichts, einen geschützten Glycosyl-Rest, oder eine o-Benzylgruppe oder ein Kohlenhydrat darstellt;

$R^6$ eine Schutzgruppe darstellt, z.B. Boc;

$R^7$ ein Harz darstellt;

oder ein pharmazeutisch verträgliches Salz davon;

welches Verfahren umfaßt:

(a) Reaktion eines Harzes mit einem Aminosäurerest mit einer Seitenkette $A^8$ und einer Schutzgruppe;

(b) Reaktion der resultierenden harzgebundenen Verbindung mit einem Aminosäurerest mit einer Seitenkette, dargestellt durch $A^7$;

(c) Wiederholung von (b) mit Aminosäuren mit Seitenketten N-Benzyloxycarbonyl-Lys, D-Trp, $A^3$, $A^2$ und $A^1$ nacheinander; und

(d) wo und wenn notwendig, Reaktion der zubereiteten Verbindung oder einer der obengenannten Reste mit einem Glycosyl-Rest, einem Kohlenhydrat, einem Acylierungsmittel oder einem Alkylierungsmittel.

**12.** Verwendung der Verbindung mit der allgemeinen Formel:

$$\begin{array}{l} R^1 \\ | \\ A^1 - A^2 - A^3 - \text{D-Trp} - \text{Lys} - A^6 - A^7 - A^8 - R^3 \\ | \\ R^2 \end{array}$$

worin

$A^1$ ein D-Isomer von Ala, Pyridyl-Ala, Leu, Ile, Val, Met, Nle, Trp, Beta-Nal, Phe, o-X-Phe (worin X $CH_3$, Cl, Br, F, OH, $OCH_3$ oder $NO_2$ darstellt), p-X-Phe (worin X = H, $CH_3$, Cl, Br, F, OH, $OCH_3$ oder $NO_2$), 2,4-Dichloro-Phe, Pentafluoro-Phe oder L-Phe darstellt;

$A^2$ Ala, Pyridyl-Ala, Leu, Ile, Val, Met, Nle, Trp, Beta-Nal, Phe, o-X-Phe (worin X $CH_3$, Cl, Br, F, OH, $OCH_3$ oder $NO_2$ darstellt), p-X-Phe (worin X = H, $CH_3$, Cl, Br, F, OH, $OCH_3$ oder $NO_2$), 2,4-Dichloro-Phe oder Pentafluoro-Phe darstellt;

$A^3$ Ala, Pyridyl-Ala, Leu, Ile, Val, Met, Nle, Trp, Tyr, Beta-Nal, Phe, o-X-Phe (worin X H, $CH_3$, Cl, Br, F, OH, $OCH_3$ oder $NO_2$ darstellt), p-X-Phe (worin X = H, $CH_3$, Cl, Br, F, OH, $OCH_3$ oder $NO_2$), 2,4-Dichloro-Phe oder Pentafluoro-Phe darstellt;

$A^6$ Ala, Pyridyl-Ala, Leu, Ile, Val, Lys, Met, Nle, Thr-$R^4$, Trp, Ser-$R^4$, Beta-Nal, o-X-Phe (worin X $CH_3$, Cl, Br, F, OH, $OCH_3$ oder $NO_2$ darstellt), p-X-Phe (worin X = $CH_3$, Cl, Br, F, OH, $OCH_3$ oder

$NO_2$), 2,4-Dichloro-Phe oder Pentafluoro-Phe darstellt;

$A^7$ Ala, Pyridyl-Ala, Leu, Ile, Val, Met, Nle, Trp, Beta-Nal, Phe, o-X-Phe (worin X $CH_3$, Cl, Br, F, OH, $OCH_3$ oder $NO_2$ darstellt), p-X-Phe (worin X = H, $CH_3$, Cl, Br, F, OH, $OCH_3$ oder $NO_2$), 2,4-Dichloro-Phe oder Pentafluoro-Phe darstellt;

$A^8$ ein D- oder L-Isomer von Ala, Pyridyl-Ala, Leu, Ile, Ser-$R^4$, Thr-$R^4$, Val, Met, Nle, Trp, Beta-Nal, o-X-Phe (worin X $CH_3$, Cl, Br, F, OH, $OCH_3$ oder $NO_2$ darstellt), p-X-Phe (worin X = $CH_3$, Cl, Br, F, OH, $OCH_3$ oder $NO_2$), 2,4-Dichloro-Phe oder Pentafluoro-Phe darstellt;

jedes von $R^1$ und $R^2$ unabhängig ein Wasserstoffatom, niedriges Acyl oder niedriges Alkyl darstellt;

$R^3$ ein Wasserstoffatom, $-NH_2$ oder niedrige Alkylgruppe darstellt;

vorausgesetzt, daß mindestens eines von $A^1$ und $A^8$ eine aromatische Aminosäure sein muß; und weiters vorausgesetzt, daß, wenn entweder $A^2$ oder $A^7$ eine aromatische Aminosäure darstellt, $A^8$ keine aromatische Aminosäure sein kann;

$R^4$ nichts, einen geschützten Glycosyl-Rest oder ein Kohlenhydrat darstellt;

oder ein pharmazeutisch verträgliches Salz davon;

bei der Herstellung eines Mittels zur Behandlung oder Phrophylaxe von Krebs, Akromegalie, hyposekretorischen endokrinen Störungen, Diabetes, Zirrhose, Geschwulsten, Pankreatitis, Diarrhö, Hepatitis, Alzheimer-Krankheit, Pilzvergiftung oder Retinopathie.

**Patentansprüche für folgenden Vertragsstaat : ES**

1. Verfahren zur Herstellung einer Verbindung mit der allgemeinen Formel:

$$\begin{array}{l} R^1 \\ | \\ A^1 - A^2 - A^3 - \text{D-Trp} - \text{Lys} - A^6 - A^7 - A^8 - R^3 \\ | \\ R^2 \end{array}$$

worin

$A^1$ ein D-Isomer von Ala, Pyridyl-Ala, Leu, Ile, Val, Met, Nle, Trp, Beta-Nal, Phe, o-X-Phe (worin X $CH_3$, Cl, Br, F, OH, $OCH_3$ oder $NO_2$ darstellt), p-X-Phe (worin X = H, $CH_3$, Cl, Br, F, OH, $OCH_3$ oder $NO_2$), 2,4-Dichloro-Phe, Pentafluoro-Phe oder L-Phe darstellt;

$A^2$ Ala, Pyridyl-Ala, Leu, Ile, Val, Met, Nle, Trp, Beta-Nal, Phe, o-X-Phe (worin X $CH_3$, Cl, Br, F, OH, $OCH_3$ oder $NO_2$ darstellt), p-X-Phe (worin X = H, $CH_3$, Cl, Br, F, OH, $OCH_3$ oder $NO_2$), 2,4-Dichloro-Phe oder Pentafluoro-Phe darstellt;

$A^3$ Ala, Pyridyl-Ala, Leu, Ile, Val, Met, Nle, Trp, Tyr, Beta-Nal, Phe, o-X-Phe (worin X H, $CH_3$, Cl, Br, F, OH, $OCH_3$ oder $NO_2$ darstellt), p-X-Phe (worin X = H, $CH_3$, Cl, Br, F, OH, $OCH_3$ oder $NO_2$), 2,4-Dichloro-Phe oder Pentafluoro-Phe darstellt;

$A^6$ Ala, Pyridyl-Ala, Leu, Ile, Val, Lys, Met, Nle, Thr-$R^4$, Trp, Ser-$R^4$, Beta-Nal, o-X-Phe (worin X $CH_3$, Cl, Br, F, OH, $OCH_3$ oder $NO_2$ darstellt), p-X-Phe (worin X = $CH_3$, Cl, Br, F, OH, $OCH_3$ oder $NO_2$), 2,4-Dichloro-Phe oder Pentafluoro-Phe darstellt;

$A^7$ Ala, Pyridyl-Ala, Leu, Ile, Val, Met, Nle, Trp, Beta-Nal, Phe, o-X-Phe (worin X $CH_3$, Cl, Br, F, OH, $OCH_3$ oder $NO_2$ darstellt), p-X-Phe (worin X = H, $CH_3$, Cl, Br, F, OH, $OCH_3$ oder $NO_2$), 2,4-Dichloro-Phe oder Pentafluoro-Phe darstellt;

$A^8$ ein D- oder L-Isomer irgendeines von Ala, Pyridyl-Ala, Leu, Ile, Ser-$R^4$, Thr-$R^4$, Val, Met, Nle, Trp, Beta-Nal, o-X-Phe (worin X $CH_3$, Cl, Br, F, OH, $OCH_3$ oder $NO_2$ darstellt), p-X-Phe (worin X = $CH_3$, Cl, Br, F, OH, $OCH_3$ oder $NO_2$), 2,4-Dichloro-Phe oder Pentafluoro-Phe darstellt;

jedes von $R^1$ und $R^2$ unabhängig ein Wasserstoffatom, niedriges Acyl oder niedriges Alkyl darstellt;

$R^3$ ein Wasserstoffatom, ein $-NH_2$ oder eine niedrige Alkylgruppe darstellt;

vorausgesetzt, daß mindestens eines von $A^1$ und $A^8$ eine aromatische Aminosäure sein muß; und weiters vorausgesetzt, daß, wenn entweder $A^2$ oder $A^7$ eine aromatische Aminosäure darstellt, $A^8$ keine aromatische Aminosäure sein kann;

$R^4$ nichts, einen geschützten Glycosyl-Rest oder ein Kohlenhydrat darstellt;

oder ein pharmazeutisch verträgliches Salz davon;

welches Verfahren das sukzessive Verbinden von Aminosäureresten umfaßt.

**2.** Verfahren nach Anspruch 1, worin eines jedoch nicht beide $A^1$ und $A^2$ eine aromatische Aminosäure darstellen; und worin eines jedoch nicht beide $A^7$ und $A^8$ eine aromatische Aminosäure darstellen.

**3.** Verfahren nach Anspruch 1 oder 2, worin $A^1$ ein D-Isomer von Trp, Beta-Nal, o-X-Phe (worin X $CH_3$ oder $OCH_3$ darstellt), p-X-Phe (worin X $CH_3$ oder $OCH_3$ darstellt) darstellt; und

$A^8$ ein D- oder L-Isomer von Ala, Pyridyl-Ala, Leu, Ile, Ser, Thr, Val, Met, Nle, o-X-Phe (worin X Cl, Br, F, OH oder $NO_2$ darstellt), p-X-Phe (worin X Cl, Br, F, OH oder $NO_2$ darstellt), 2,4-Dichloro-Phe oder Pentafluoro-Phe darstellt;

**4.** Verfahren nach einem der Ansprüche 1 bis 3, welches eine, einige oder alle der folgenden Eigenschaften hat:

$A^1$ zeigt Beta-D-Nal oder D-Phe;
$A^2$ zeigt Ala, Phe oder p-Chloro-Phe;
$A^3$ zeigt Tyr oder Phe;
$A^6$ zeigt Val, Lys oder Thr;
$A^7$ zeigt Ala oder Phe; und/oder
$A^8$ zeigt Thr oder Beta-D-Nal.

**5.** Verfahren nach einem der Ansprüche 1 bis 4, welches ist:

D-Phe-Phe-Phe-D-Trp-Lys-Thr-Phe-Thr-$NH_2$ [SEQ.ID NO 3];
D-Phe-Phe-Tyr-D-Trp-Lys-Val-Phe-Thr-$NH_2$ [SEQ.ID NO 2];
D-Phe-p-chloro-Phe-Tyr-D-Trp-Lys-Val-Phe-Thr-$NH_2$ [SEQ.ID NO 1]; oder
D-Phe-Ala-Tyr-D-Trp-Lys-Val-Ala-beta-D-Nal-$NH_2$ [SEQ.ID NO 4].

**6.** Verfahren zur Herstellung einer Verbindung mit der allgemeinen Formel:

$R^1$
|
$A^1$ - $A^2$ - $A^3$ - D-Trp - Lys - $A^6$ - $A^7$ - $A^8$ - $R^3$
|
$R^2$

worin

$A^1$ ein D-Isomer von Ala, Pyridyl-Ala, Leu, Ile, Val, Met, Nle, Trp, Beta-Nal, Phe, o-X-Phe (worin X $CH_3$, Cl, Br, F, OH, $OCH_3$ oder $NO_2$ darstellt), p-X-Phe (worin X = H, $CH_3$, Cl, Br, F, OH, $OCH_3$ oder $NO_2$), 2,4-Dichloro-Phe, Pentafluoro-Phe oder L-Phe darstellt;

$A^2$ Ala, Pyridyl-Ala, Leu, Ile, Val, Met, Nle, Trp, Beta-Nal, Phe, o-X-Phe (worin X $CH_3$, Cl, Br, F, OH, $OCH_3$ oder $NO_2$ darstellt), p-X-Phe (worin X = H, $CH_3$, Cl, Br, F, OH, $OCH_3$ oder $NO_2$), 2,4-Dichloro-Phe oder Pentafluoro-Phe darstellt;

$A^3$ Ala, Pyridyl-Ala, Leu, Ile, Val, Met, Nle, Trp, Tyr, Beta-Nal, Phe, o-X-Phe (worin X H, $CH_3$, Cl, Br, F, OH, $OCH_3$ oder $NO_2$ darstellt), p-X-Phe (worin X = H, $CH_3$, Cl, Br, F, OH, $OCH_3$ oder $NO_2$), 2,4-Dichloro-Phe oder Pentafluoro-Phe darstellt;

$A^6$ Ala, Pyridyl-Ala, Leu, Ile, Val, Lys, Met, Nle, Thr-$R^4$, Trp, Ser-$R^4$, Beta-Nal, o-X-Phe (worin X $CH_3$, Cl, Br, F, OH, $OCH_3$ oder $NO_2$ darstellt), p-X-Phe (worin X = $CH_3$, Cl, Br, F, OH, $OCH_3$ oder $NO_2$), 2,4-Dichloro-Phe oder Pentafluoro-Phe darstellt;

$A^7$ Ala, Pyridyl-Ala, Leu, Ile, Val, Met, Nle, Trp, Beta-Nal, Phe, o-X-Phe (worin X $CH_3$, Cl, Br, F, OH, $OCH_3$ oder $NO_2$ darstellt), p-X-Phe (worin X = H, $CH_3$, Cl, Br, F, OH, $OCH_3$ oder $NO_2$), 2,4-Dichloro-Phe oder Pentafluoro-Phe darstellt;

$A^8$ ein D- oder L-Isomer von Ala, Pyridyl-Ala, Leu, Ile, Ser-$R^4$, Thr-$R^4$, Val, Met, Nle, Trp, Beta-Nal, o-X-Phe (worin X $CH_3$, Cl, Br, F, OH, $OCH_3$ oder $NO_2$ darstellt), p-X-Phe (worin X = $CH_3$, Cl, Br, F, OH, $OCH_3$ oder $NO_2$), 2,4-Dichloro-Phe oder Pentafluoro-Phe darstellt;

jedes von $R^1$ und $R^2$ unabhängig ein Wasserstoffatom, niedriges Acyl oder niedriges Alkyl darstellt;

$R^3$ ein Wasserstoffatom, $-NH_2$ oder niedrige Alkylgruppe darstellt;

vorausgesetzt, daß mindestens eines von $A^1$ und $A^8$ eine aromatische Aminosäure sein muß; und weiters vorausgesetzt, daß, wenn entweder $A^2$ oder $A^7$ eine aromatische Aminosäure darstellt, $A^8$ keine aromatische Aminosäure sein kann;

$R^4$ nichts, einen geschützten Glycosyl-Rest oder ein Kohlenhydrat darstellt;

oder ein pharmazeutisch verträgliches Salz davon;

welches Verfahren eine Reaktion umfaßt einer Verbindung mit der allgemeinen Formel:

$R^1$

|

$R^6$ - $A^1$ -$A^2$ - $A^3$ - D-Trp - N-Benzyloxycarboxyl - Lys -

|

$R^2$ $A^6$ - $A^7$ - $A^8$ - $R^7$

worin:

$R^1$, $R^2$, $A^1$, $A^2$, $A^7$ und $A^8$ wie vorher definiert sind;

$A^3$ wie vorher definiert ist, außer daß Tyr durch o-Benzyl-Tyr ersetzt ist;

$A^6$ wie vorher definiert ist, außer daß Lys durch N-Benzyloxycarbonyl-Lys ersetzt ist;

$R^4$ zusätzlich eine o-Benzylgruppe darstellen kann;

$R^6$ eine Schutzgruppe darstellt; und

$R^7$ ein Harz darstellt;

mit:

Kresol;

Dithiothreitol; und

Fluorwasserstoff.

**7.** Verfahren zur Herstellung einer Verbindung mit der allgemeinen Formel:

$R^1$

|

$R^6$ - $A^1$ -$A^2$ - $A^3$ - D-Trp - N-Benzyloxycarboxyl - Lys -

|

$R^2$ $A^6$ - $A^7$ - $A^8$ - $R^7$

worin

$A^1$ ein D-Isomer von Ala, Pyridyl-Ala, Leu, Ile, Val, Met, Nle, Trp, Beta-Nal, Phe, o-X-Phe (worin X $CH_3$, Cl, Br, F, OH, $OCH_3$ oder $NO_2$ darstellt), p-X-Phe (worin X = H, $CH_3$, Cl, Br, F, OH, $OCH_3$ oder $NO_2$), 2,4-Dichloro-Phe, Pentafluoro-Phe oder L-Phe darstellt;

$A^2$ Ala, Pyridyl-Ala, Leu, Ile, Val, Met, Nle, Trp, Beta-Nal, Phe, o-X-Phe (worin X $CH_3$, Cl, Br, F, OH, $OCH_3$ oder $NO_2$ darstellt), p-X-Phe (worin X = H, $CH_3$, Cl, Br, F, OH, $OCH_3$ oder $NO_2$), 2,4-Dichloro-Phe oder Pentafluoro-Phe darstellt;

$A^3$ Ala, Pyridyl-Ala, Leu, Ile, Val, Met, Nle, Trp, o-Benzyl-Tyr, Beta-Nal, Phe, o-X-Phe (worin X H, $CH_3$, Cl, Br, F, OH, $OCH_3$ oder $NO_2$ darstellt), p-X-Phe (worin X = H, $CH_3$, Cl, Br, F, OH, $OCH_3$ oder $NO_2$), 2,4-Dichloro-Phe oder Pentafluoro-Phe darstellt;

$A^6$ Ala, Pyridyl-Ala, Leu, Ile, Val, N-Benzyloxycarbonyl-Lys, Met, Nle, Thr-$R^4$, Trp, Ser-$R^4$, Beta-Nal, o-X-Phe (worin X $CH_3$, Cl, Br, F, OH, $OCH_3$ oder $NO_2$ darstellt), p-X-Phe (worin X = $CH_3$, Cl, Br, F, OH, $OCH_3$ oder $NO_2$), 2,4-Dichloro-Phe oder Pentafluoro-Phe darstellt;

$A^7$ Ala, Pyridyl-Ala, Leu, Ile, Val, Met, Nle, Trp, Beta-Nal, Phe, o-X-Phe (worin X $CH_3$, Cl, Br, F, OH, $OCH_3$ oder $NO_2$ darstellt), p-X-Phe (worin X = H, $CH_3$, Cl, Br, F, OH, $OCH_3$ oder $NO_2$), 2,4-Dichloro-Phe oder Pentafluoro-Phe darstellt;

$A^8$ ein D- oder L-Isomer von Ala, Pyridyl-Ala, Leu, Ile, Ser-$R^4$, Thr-$R^4$, Val, Met, Nle, Trp, Beta-Nal, o-X-Phe (worin X $CH_3$, Cl, Br, F, OH, $OCH_3$ oder $NO_2$ darstellt), p-X-Phe (worin X = $CH_3$, Cl, Br, F, OH, $OCH_3$ oder $NO_2$), 2,4-Dichloro-Phe oder Pentafluoro-Phe darstellt;

jedes von $R^1$ und $R^2$ unabhängig ein Wasserstoffatom, niedriges Acyl oder niedriges Alkyl darstellt;

vorausgesetzt, daß mindestens eines von $A^1$ und $A^8$ eine aromatische Aminosäure sein muß; und weiters vorausgesetzt, daß, wenn entweder $A^2$ oder $A^7$ eine aromatische Aminosäure darstellt, $A^8$ keine aromatische Aminosäure sein kann;

$R^4$ nichts, einen geschützten Glycosyl-Rest, oder einer o-Benzylgruppe oder ein Kohlenhydrat darstellt;

$R^6$ eine Schutzgruppe darstellt, z.B. Boc;

$R^7$ ein Harz darstellt;

oder ein pharmazeutisch verträgliches Salz davon;

welches Verfahren umfaßt:

(a) Reaktion eines Harzes mit einem Aminosäurerest mit einer Seitenkette $A^8$ und einer Schutzgruppe;

(b) Reaktion der resultierenden harzgebundenen Verbindung mit einem Aminosäurerest mit einer Seitenkette, dargestellt durch $A^7$;

(c) Wiederholung von (b) mit Aminosäuren mit Seitenketten $A^7$, N-Benzyloxycarbonyl-Lys, D-Trp, $A^3$, $A^2$ und $A^1$ nacheinander; und

(d) wo und wenn notwendig, Reaktion der zubereiteten Verbindung oder einer der obengenannten Reste mit einem Glycosyl-Rest, einem Kohlenhydrat, einem Acylierungsmittel oder einem Alkylierungsmittel.

**8.** Verwendung einer Verbindung mit der allgemeinen Formel:

$$\begin{array}{l} R^1 \\ | \\ A^1 - A^2 - A^3 - \text{D-Trp} - \text{Lys} - A^6 - A^7 - A^8 - R^3 \\ | \\ R^2 \end{array}$$

worin

$A^1$ ein D-Isomer von Ala, Pyridyl-Ala, Leu, Ile, Val, Met, Nle, Trp, Beta-Nal, Phe, o-X-Phe (worin X $CH_3$, Cl, Br, F, OH, $OCH_3$ oder $NO_2$ darstellt), p-X-Phe (worin X = H, $CH_3$, Cl, Br, F, OH, $OCH_3$ oder $NO_2$), 2,4-Dichloro-Phe, Pentafluoro-Phe oder L-Phe darstellt;

$A^2$ Ala, Pyridyl-Ala, Leu, Ile, Val, Met, Nle, Trp, Beta-Nal, Phe, o-X-Phe (worin X $CH_3$, Cl, Br, F, OH, $OCH_3$ oder $NO_2$ darstellt), p-X-Phe (worin X = H, $CH_3$, Cl, Br, F, OH, $OCH_3$ oder $NO_2$), 2,4-Dichloro-Phe oder Pentafluoro-Phe darstellt;

$A^3$ Ala, Pyridyl-Ala, Leu, Ile, Val, Met, Nle, Trp, Tyr, Beta-Nal, Phe, o-X-Phe (worin X H, $CH_3$, Cl, Br, F, OH, $OCH_3$ oder $NO_2$ darstellt), p-X-Phe (worin X = H, $CH_3$, Cl, Br, F, OH, $OCH_3$ oder $NO_2$), 2,4-Dichloro-Phe oder Pentafluoro-Phe darstellt;

$A^6$ Ala, Pyridyl-Ala, Leu, Ile, Val, Lys, Met, Nle, Thr-$R^4$, Trp, Ser-$R^4$, Beta-Nal, o-X-Phe (worin X $CH_3$, Cl, Br, F, OH, $OCH_3$ oder $NO_2$ darstellt), p-X-Phe (worin X = $CH_3$, Cl, Br, F, OH, $OCH_3$ oder $NO_2$), 2,4-Dichloro-Phe oder Pentafluoro-Phe darstellt;

$A^7$ Ala, Pyridyl-Ala, Leu, Ile, Val, Met, Nle, Trp, Beta-Nal, Phe, o-X-Phe (worin X $CH_3$, Cl, Br, F, OH, $OCH_3$ oder $NO_2$ darstellt), p-X-Phe (worin X = H, $CH_3$, Cl, Br, F, OH, $OCH_3$ oder $NO_2$), 2,4-Dichloro-Phe oder Pentafluoro-Phe darstellt;

$A^8$ ein D- oder L-Isomer von Ala, Pyridyl-Ala, Leu, Ile, Ser-$R^4$, Thr-$R^4$, Val, Met, Nle, Trp, Beta-Nal, o-X-Phe (worin X $CH_3$, Cl, Br, F, OH, $OCH_3$ oder $NO_2$ darstellt), p-X-Phe (worin X = $CH_3$, Cl, Br, F, OH, $OCH_3$ oder $NO_2$), 2,4-Dichloro-Phe oder Pentafluoro-Phe darstellt;

jedes von $R^1$ und $R^2$ unabhängig ein Wasserstoffatom, niedriges Acyl oder niedriges Alkyl darstellt;

$R^3$ ein Wasserstoffatom, -$NH_2$ oder niedrige Alkylgruppe darstellt;

vorausgesetzt, daß mindestens eines von $A^1$ und $A^8$ eine aromatische Aminosäure sein muß; und weiters vorausgesetzt, daß, wenn entweder $A^2$ oder $A^7$ eine aromatische Aminosäure darstellt, $A^8$ keine

aromatische Aminosäure sein kann;

$R^4$ nichts, einen geschützten Glycosyl-Rest oder ein Kohlenhydrat darstellt;

oder ein pharmazeutisch verträgliches Salz davon;

bei der Herstellung eines Mittels zur Behandlung oder Phrophylaxe von Krebs, Akromegalie, hyposekretorischen endokrinen Störungen, Diabetes, Zirrhose, Geschwulsten, Pankreatitis, Diarrhö, Hepatitis, Alzheimer-Krankheit, Pilzvergiftung oder Retinopathie.

**9.** Verfahren zur Herstellung einer pharmazeutischen Zusammensetzung, welches Verfahren das Beimischen umfaßt einer Verbindung mit der allgemeinen Formel:

$$\begin{array}{l} R^1 \\ | \\ A^1 - A^2 - A^3 - \text{D-Trp} - \text{Lys} - A^6 - A^7 - A^8 - R^3 \\ | \\ R^2 \end{array}$$

worin

$A^1$ ein D-Isomer von Ala, Pyridyl-Ala, Leu, Ile, Val, Met, Nle, Trp, Beta-Nal, Phe, o-X-Phe (worin X $CH_3$, Cl, Br, F, OH, $OCH_3$ oder $NO_2$ darstellt), p-X-Phe (worin X = H, $CH_3$, Cl, Br, F, OH, $OCH_3$ oder $NO_2$), 2,4-Dichloro-Phe, Pentafluoro-Phe oder L-Phe darstellt;

$A^2$ Ala, Pyridyl-Ala, Leu, Ile, Val, Met, Nle, Trp, Beta-Nal, Phe, o-X-Phe (worin X $CH_3$, Cl, Br, F, OH, $OCH_3$ oder $NO_2$ darstellt), p-X-Phe (worin X = H, $CH_3$, Cl, Br, F, OH, $OCH_3$ oder $NO_2$), 2,4-Dichloro-Phe oder Pentafluoro-Phe darstellt;

$A^3$ Ala, Pyridyl-Ala, Leu, Ile, Val, Met, Nle, Trp, Tyr, Beta-Nal, Phe, o-X-Phe (worin X H, $CH_3$, Cl, Br, F, OH, $OCH_3$ oder $NO_2$ darstellt), p-X-Phe (worin X = H, $CH_3$, Cl, Br, F, OH, $OCH_3$ oder $NO_2$), 2,4-Dichloro-Phe oder Pentafluoro-Phe darstellt;

$A^6$ Ala, Pyridyl-Ala, Leu, Ile, Val, Lys, Met, Nle, Thr-$R^4$, Trp, Ser-$R^4$, Beta-Nal, o-X-Phe (worin X $CH_3$, Cl, Br, F, OH, $OCH_3$ oder $NO_2$ darstellt), p-X-Phe (worin X = $CH_3$, Cl, Br, F, OH, $OCH_3$ oder $NO_2$), 2,4-Dichloro-Phe oder Pentafluoro-Phe darstellt;

$A^7$ Ala, Pyridyl-Ala, Leu, Ile, Val, Met, Nle, Trp, Beta-Nal, Phe, o-X-Phe (worin X $CH_3$, Cl, Br, F, OH, $OCH_3$ oder $NO_2$ darstellt), p-X-Phe (worin X = H, $CH_3$, Cl, Br, F, OH, $OCH_3$ oder $NO_2$), 2,4-Dichloro-Phe oder Pentafluoro-Phe darstellt;

$A^8$ ein D- oder L-Isomer von Ala, Pyridyl-Ala, Leu, Ile, Ser-$R^4$, Thr-$R^4$, Val, Met, Nle, Trp, Beta-Nal, o-X-Phe (worin X $CH_3$, Cl, Br, F, OH, $OCH_3$ oder $NO_2$ darstellt), p-X-Phe (worin X = $CH_3$, Cl, Br, F, OH, $OCH_3$ oder $NO_2$), 2,4-Dichloro-Phe oder Pentafluoro-Phe darstellt;

jedes von $R^1$ und $R^2$ unabhängig ein Wasserstoffatom, niedriges Acyl oder niedriges Alkyl darstellt;

$R^3$ ein Wasserstoffatom, -$NH_2$ oder niedrige Alkylgruppe darstellt;

vorausgesetzt, daß mindestens eines von $A^1$ und $A^8$ eine aromatische Aminosäure sein muß; und weiters vorausgesetzt, daß, wenn entweder $A^2$ oder $A^7$ eine aromatische Aminosäure darstellt, $A^8$ keine aromatische Aminosäure sein kann;

$R^4$ nichts, einen geschützten Glycosyl-Rest oder ein Kohlenhydrat darstellt;

oder ein pharmazeutisch verträgliches Salz davon;

welches Verfahren das sukzessive Verbinden der Aminosäurereste umfaßt;

zu einem pharmazeutisch verträglichen Träger.

**10.** Verfahren nach Anspruch 9, in welchem die Zusammensetzung mit einer Substanz umhüllt wird, welche imstande ist, die Zusammensetzung vor der Magensäure im Magen einen ausreichenden Zeitraum zu schützen, in welchem die Verbindung unaufgelöst in den Dünndarm gelangen kann.

**Patentansprüche für folgenden Vertragsstaat : GR**

1. Verfahren zur Herstellung einer Verbindung mit der allgemeinen Formel:

$$\begin{array}{l} R^1 \\ | \\ A^1 - A^2 - A^3 - \text{D-Trp} - \text{Lys} - A^6 - A^7 - A^8 - R^3 \\ | \\ R^2 \end{array}$$

worin

$A^1$ ein D-Isomer von Ala, Pyridyl-Ala, Leu, Ile, Val, Met, Nle, Trp, Beta-Nal, Phe, o-X-Phe (worin X $CH_3$, Cl, Br, F, OH, $OCH_3$ oder $NO_2$ darstellt), p-X-Phe (worin X = H, $CH_3$, Cl, Br, F, OH, $OCH_3$ oder $NO_2$), 2,4-Dichloro-Phe, Pentafluoro-Phe oder L-Phe darstellt;

$A^2$ Ala, Pyridyl-Ala, Leu, Ile, Val, Met, Nle, Trp, Beta-Nal, Phe, o-X-Phe (worin X $CH_3$, Cl, Br, F, OH, $OCH_3$ oder $NO_2$ darstellt), p-X-Phe (worin X = H, $CH_3$, Cl, Br, F, OH, $OCH_3$ oder $NO_2$), 2,4-Dichloro-Phe oder Pentafluoro-Phe darstellt;

$A^3$ Ala, Pyridyl-Ala, Leu, Ile, Val, Met, Nle, Trp, Tyr, Beta-Nal, Phe, o-X-Phe (worin X H, $CH_3$, Cl, Br, F, OH, $OCH_3$ oder $NO_2$ darstellt), p-X-Phe (worin X = H, $CH_3$, Cl, Br, F, OH, $OCH_3$ oder $NO_2$), 2,4-Dichloro-Phe oder Pentafluoro-Phe darstellt;

$A^6$ Ala, Pyridyl-Ala, Leu, Ile, Val, Lys, Met, Nle, Thr-$R^4$, Trp, Ser-$R^4$, Beta-Nal, o-X-Phe (worin X $CH_3$, Cl, Br, F, OH, $OCH_3$ oder $NO_2$ darstellt), p-X-Phe (worin X = $CH_3$, Cl, Br, F, OH, $OCH_3$ oder $NO_2$), 2,4-Dichloro-Phe oder Pentafluoro-Phe darstellt;

$A^7$ Ala, Pyridyl-Ala, Leu, Ile, Val, Met, Nle, Trp, Beta-Nal, Phe, o-X-Phe (worin X $CH_3$, Cl, Br, F, OH, $OCH_3$ oder $NO_2$ darstellt), p-X-Phe (worin X = H, $CH_3$, Cl, Br, F, OH, $OCH_3$ oder $NO_2$), 2,4-Dichloro-Phe oder Pentafluoro-Phe darstellt;

$A^8$ ein D- oder L-Isomer irgendeines von Ala, Pyridyl-Ala, Leu, Ile, Ser-$R^4$, Thr-$R^4$, Val, Met, Nle, Trp, Beta-Nal, o-X-Phe (worin X $CH_3$, Cl, Br, F, OH, $OCH_3$ oder $NO_2$ darstellt), p-X-Phe (worin X = $CH_3$, Cl, Br, F, OH, $OCH_3$ oder $NO_2$), 2,4-Dichloro-Phe oder Pentafluoro-Phe darstellt;

jedes von $R^1$ und $R^2$ unabhängig ein Wasserstoffatom, niedriges Acyl oder niedriges Alkyl darstellt;

$R^3$ ein Wasserstoffatom, -$NH_2$ oder niedrige Alkylgruppe darstellt;

vorausgesetzt, daß mindestens eines von $A^1$ und $A^8$ eine aromatische Aminosäure sein muß; und weiters vorausgesetzt, daß, wenn entweder $A^2$ oder $A^7$ eine aromatische Aminosäure darstellt, $A^8$ keine aromatische Aminosäure sein kann;

$R^4$ nichts, einen geschützten Glycosyl-Rest oder ein Kohlenhydrat darstellt;

oder ein pharmazeutisch verträgliches Salz davon;

welches Verfahren das sukzessive Verbinden der Aminosäurereste umfaßt.

2. Verfahren nach Anspruch 1, worin eines jedoch nicht beide $A^1$ und $A^2$ eine aromatische Aminosäure darstellen; und worin eines jedoch nicht beide $A^7$ und $A^8$ eine aromatische Aminosäure darstellen.

3. Verfahren nach Anspruch 1 oder 2, worin $A^1$ ein D-Isomer von Trp, Beta-Nal, o-X-Phe (worin X $CH_3$ oder $OCH_3$ darstellt), p-X-Phe (worin X $CH_3$ oder $OCH_3$ darstellt) darstellt; und

$A^8$ ein D- oder L-Isomer von Ala, Pyridyl-Ala, Leu, Ile, Ser, Thr, Val, Met, Nle, o-X-Phe (worin X Cl, Br, F, OH oder $NO_2$ darstellt), p-X-Phe (worin X Cl, Br, F, OH oder $NO_2$ darstellt), 2,4-Dichloro-Phe oder Pentafluoro-Phe darstellt;

4. Verfahren nach einem der Ansprüche 1 bis 3, welches eine, einige oder alle der folgenden Eigenschaften hat:

$A^1$ zeigt Beta-D-Nal oder D-Phe;

$A^2$ zeigt Ala, Phe oder p-Chloro-Phe;

$A^3$ zeigt Tyr oder Phe;

$A^6$ zeigt Val, Lys oder Thr;

$A^7$ zeigt Ala oder Phe; und/oder
$A^8$ zeigt Thr oder Beta-D-Nal.

**5.** Verfahren nach einem der Ansprüche 1 bis 4, welches ist:
D-Phe-Phe-Phe-D-Trp-Lys-Thr-Phe-Thr-$NH_2$ [SEQ.ID NO 3];
D-Phe-Phe-Tyr-D-Trp-Lys-Val-Phe-Thr-$NH_2$ [SEQ.ID NO 2];
D-Phe-p-chloro-Phe-Tyr-D-Trp-Lys-Val-Phe-Thr-$NH_2$ [SEQ.ID NO 1]; oder
D-Phe-Ala-Tyr-D-Trp-Lys-Val-Ala-beta-D-Nal-$NH_2$ [SEQ.ID NO 4].

**6.** Verfahren zur Herstellung einer Verbindung mit der allgemeinen Formel:

$$\begin{array}{l} R^1 \\ | \\ A^1 - A^2 - A^3 - \text{D-Trp} - \text{Lys} - A^6 - A^7 - A^8 - R^3 \\ | \\ R^2 \end{array}$$

worin
$A^1$ ein D-Isomer von Ala, Pyridyl-Ala, Leu, Ile, Val, Met, Nle, Trp, Beta-Nal, Phe, o-X-Phe (worin X $CH_3$, Cl, Br, F, OH, $OCH_3$ oder $NO_2$ darstellt), p-X-Phe (worin X = H, $CH_3$, Cl, Br, F, OH, $OCH_3$ oder $NO_2$), 2,4-Dichloro-Phe, Pentafluoro-Phe oder L-Phe darstellt;
$A^2$ Ala, Pyridyl-Ala, Leu, Ile, Val, Met, Nle, Trp, Beta-Nal, Phe, o-X-Phe (worin X $CH_3$, Cl, Br, F, OH, $OCH_3$ oder $NO_2$ darstellt), p-X-Phe (worin X = H, $CH_3$, Cl, Br, F, OH, $OCH_3$ oder $NO_2$), 2,4-Dichloro-Phe oder Pentafluoro-Phe darstellt;
$A^3$ Ala, Pyridyl-Ala, Leu, Ile, Val, Met, Nle, Trp, Tyr, Beta-Nal, Phe, o-X-Phe (worin X H, $CH_3$, Cl, Br, F, OH, $OCH_3$ oder $NO_2$ darstellt), p-X-Phe (worin X = H, $CH_3$, Cl, Br, F, OH, $OCH_3$ oder $NO_2$), 2,4-Dichloro-Phe oder Pentafluoro-Phe darstellt;
$A^6$ Ala, Pyridyl-Ala, Leu, Ile, Val, Lys, Met, Nle, Thr-$R^4$, Trp, Ser-$R^4$, Beta-Nal, o-X-Phe (worin X $CH_3$, Cl, Br, F, OH, $OCH_3$ oder $NO_2$ darstellt), p-X-Phe (worin X = $CH_3$, Cl, Br, F, OH, $OCH_3$ oder $NO_2$), 2,4-Dichloro-Phe oder Pentafluoro-Phe darstellt;
$A^7$ Ala, Pyridyl-Ala, Leu, Ile, Val, Met, Nle, Trp, Beta-Nal, Phe, o-X-Phe (worin X $CH_3$, Cl, Br, F, OH, $OCH_3$ oder $NO_2$ darstellt), p-X-Phe (worin X = H, $CH_3$, Cl, Br, F, OH, $OCH_3$ oder $NO_2$), 2,4-Dichloro-Phe oder Pentafluoro-Phe darstellt;
$A^8$ ein D- oder L-Isomer von Ala, Pyridyl-Ala, Leu, Ile, Ser-$R^4$, Thr-$R^4$, Val, Met, Nle, Trp, Beta-Nal, o-X-Phe (worin X $CH_3$, Cl, Br, F, OH, $OCH_3$ oder $NO_2$ darstellt), p-X-Phe (worin X = $CH_3$, Cl, Br, F, OH, $OCH_3$ oder $NO_2$), 2,4-Dichloro-Phe oder Pentafluoro-Phe darstellt;
jedes von $R^1$ und $R^2$ unabhängig ein Wasserstoffatom, niedriges Acyl oder niedriges Alkyl darstellt;
$R^3$ ein Wasserstoffatom, -$NH_2$ oder niedrige Alkylgruppe darstellt;
vorausgesetzt, daß mindestens eines von $A^1$ und $A^8$ eine aromatische Aminosäure sein muß; und weiters vorausgesetzt, daß, wenn entweder $A^2$ oder $A^7$ eine aromatische Aminosäure darstellt, $A^8$ keine aromatische Aminosäure sein kann;
$R^4$ nichts, einen geschützten Glycosyl-Rest oder ein Kohlenhydrat darstellt;
oder ein pharmazeutisch verträgliches Salz davon;
welches Verfahren eine Reaktion umfaßt einer Verbindung mit der allgemeinen Formel:

$$\begin{array}{l} \quad R^1 \\ \quad | \\ R^6 - A^1 - A^2 - A^3 - \text{D-Trp} - \text{N-Benzyloxycarboxyl} - \text{Lys} - \\ \quad | \\ \quad R^2 \qquad\qquad A^6 - A^7 - A^8 - R^7 \end{array}$$

worin:
$R^1$, $R^2$, $A^1$, $A^2$, $A^7$ und $A^8$ wie vorher definiert sind;
$A^3$ wie vorher definiert ist, außer daß Tyr durch o-Benzyl-Tyr ersetzt ist;
$A^6$ wie vorher definiert ist, außer daß Lys durch N-Benzyloxycarbonyl-Lys ersetzt ist;
$R^4$ zusätzlich eine o-Benzylgruppe darstellen kann;
$R^6$ eine Schutzgruppe darstellt; und
$R^7$ ein Harz darstellt;
mit:
Kresol;
Dithiothreitol; und
Fluorwasserstoff.

**7.** Verbindung mit der allgemeinen Formel:

$R^1$
|
$R^6$ - $A^1$ -$A^2$ - $A^3$ - D-Trp - N-Benzyloxycarboxyl - Lys -
|
$R^2$ $A^6$ - $A^7$ - $A^8$ - $R^7$

worin

$A^1$ ein D-Isomer von Ala, Pyridyl-Ala, Leu, Ile, Val, Met, Nle, Trp, Beta-Nal, Phe, o-X-Phe (worin X $CH_3$, Cl, Br, F, OH, $OCH_3$ oder $NO_2$ darstellt), p-X-Phe (worin X = H, $CH_3$, Cl, Br, F, OH, $OCH_3$ oder $NO_2$), 2,4-Dichloro-Phe, Pentafluoro-Phe oder L-Phe darstellt;

$A^2$ Ala, Pyridyl-Ala, Leu, Ile, Val, Met, Nle, Trp, Beta-Nal, Phe, o-X-Phe (worin X $CH_3$, Cl, Br, F, OH, $OCH_3$ oder $NO_2$ darstellt), p-X-Phe (worin X = H, $CH_3$, Cl, Br, F, OH, $OCH_3$ oder $NO_2$), 2,4-Dichloro-Phe oder Pentafluoro-Phe darstellt;

$A^3$ Ala, Pyridyl-Ala, Leu, Ile, Val, Met, Nle, Trp, o-Benzyl-Tyr, Beta-Nal, Phe, o-X-Phe (worin X H, $CH_3$, Cl, Br, F, OH, $OCH_3$ oder $NO_2$ darstellt), p-X-Phe (worin X = H, $CH_3$, Cl, Br, F, OH, $OCH_3$ oder $NO_2$), 2,4-Dichloro-Phe oder Pentafluoro-Phe darstellt;

$A^6$ Ala, Pyridyl-Ala, Leu, Ile, Val, N-Benzyloxycarbonyl-Lys, Met, Nle, Thr-$R^4$, Trp, Ser-$R^4$, Beta-Nal, o-X-Phe (worin X $CH_3$, Cl, Br, F, OH, $OCH_3$ oder $NO_2$ darstellt), p-X-Phe (worin X = $CH_3$, Cl, Br, F, OH, $OCH_3$ oder $NO_2$), 2,4-Dichloro-Phe oder Pentafluoro-Phe darstellt;

$A^7$ Ala, Pyridyl-Ala, Leu, Ile, Val, Met, Nle, Trp, Beta-Nal, Phe, o-X-Phe (worin X $CH_3$, Cl, Br, F, OH, $OCH_3$ oder $NO_2$ darstellt), p-X-Phe (worin X = H, $CH_3$, Cl, Br, F, OH, $OCH_3$ oder $NO_2$), 2,4-Dichloro-Phe oder Pentafluoro-Phe darstellt;

$A^8$ ein D- oder L-Isomer von Ala, Pyridyl-Ala, Leu, Ile, Ser-$R^4$, Thr-$R^4$, Val, Met, Nle, Trp, Beta-Nal, o-X-Phe (worin X $CH_3$, Cl, Br, F, OH, $OCH_3$ oder $NO_2$ darstellt), p-X-Phe (worin X = $CH_3$, Cl, Br, F, OH, $OCH_3$ oder $NO_2$), 2,4-Dichloro-Phe oder Pentafluoro-Phe darstellt;

jedes von $R^1$ und $R^2$ unabhängig ein Wasserstoffatom, niedriges Acyl oder niedriges Alkyl darstellt;

vorausgesetzt, daß mindestens eines von $A^1$ und $A^8$ eine aromatische Aminosäure sein muß; und weiters vorausgesetzt, daß, wenn entweder $A^2$ oder $A^7$ eine aromatische Aminosäure darstellt, $A^8$ keine aromatische Aminosäure sein kann;

$R^4$ nichts, einen geschützten Glycosyl-Rest, oder eine o-Benzylgruppe oder ein Kohlenhydrat darstellt;

$R^6$ eine Schutzgruppe darstellt, z.B. Boc;

$R^7$ ein Harz darstellt;

oder ein pharmazeutisch verträgliches Salz davon.

**8.** Verfahren zur Herstellung einer Verbindung mit der allgemeinen Formel:

$$
\begin{array}{l}
\quad R^1 \\
\quad | \\
R^6 - A^1 - A^2 - A^3 - \text{D-Trp} - \text{N-Benzyloxycarboxyl} - \text{Lys} - \\
\quad | \\
\quad R^2 \qquad\qquad A^6 - A^7 - A^8 - R^7
\end{array}
$$

worin

$A^1$ ein D-Isomer von Ala, Pyridyl-Ala, Leu, Ile, Val, Met, Nle, Trp, Beta-Nal, Phe, o-X-Phe (worin X $CH_3$, Cl, Br, F, OH, $OCH_3$ oder $NO_2$darstellt), p-X-Phe (worin X = H, $CH_3$, Cl, Br, F, OH, $OCH_3$ oder $NO_2$), 2,4-Dichloro-Phe, Pentafluoro-Phe oder L-Phe darstellt;

$A^2$ Ala, Pyridyl-Ala, Leu, Ile, Val, Met, Nle, Trp, Beta-Nal, Phe, o-X-Phe (worin X $CH_3$, Cl, Br, F, OH, $OCH_3$ oder $NO_2$ darstellt), p-X-Phe (worin X = H, $CH_3$, Cl, Br, F, OH, $OCH_3$ oder $NO_2$), 2,4-Dichloro-Phe oder Pentafluoro-Phe darstellt;

$A^3$ Ala, Pyridyl-Ala, Leu, Ile, Val, Met, Nle, Trp, o-Benzyl-Tyr, Beta-Nal, Phe, o-X-Phe (worin X H, $CH_3$, Cl, Br, F, OH, $OCH_3$ oder $NO_2$ darstellt), p-X-Phe (worin X = H, $CH_3$, Cl, Br, F, OH, $OCH_3$ oder $NO_2$), 2,4-Dichloro-Phe oder Pentafluoro-Phe darstellt;

$A^6$ Ala, Pyridyl-Ala, Leu, Ile, Val, N-Benzyloxycarbonyl-Lys, Met, Nle, Thr-$R^4$, Trp, Ser-$R^4$, Beta-Nal, o-X-Phe (worin X $CH_3$, Cl, Br, F, OH, $OCH_3$ oder $NO_2$ darstellt), p-X-Phe (worin X = $CH_3$, Cl, Br, F, OH, $OCH_3$ oder $NO_2$), 2,4-Dichloro-Phe oder Pentafluoro-Phe darstellt;

$A^7$ Ala, Pyridyl-Ala, Leu, Ile, Val, Met, Nle, Trp, Beta-Nal, Phe, o-X-Phe (worin X $CH_3$, Cl, Br, F, OH, $OCH_3$ oder $NO_2$ darstellt), p-X-Phe (worin X = H, $CH_3$, Cl, Br, F, OH, $OCH_3$ oder $NO_2$), 2,4-Dichloro-Phe oder Pentafluoro-Phe darstellt;

$A^8$ ein D- oder L-Isomer von Ala, Pyridyl-Ala, Leu, Ile, Ser-$R^4$, Thr-$R^4$, Val, Met, Nle, Trp, Beta-Nal, o-X-Phe (worin X $CH_3$, Cl, Br, F, OH, $OCH_3$ oder $NO_2$ darstellt), p-X-Phe (worin X = $CH_3$, Cl, Br, F, OH, $OCH_3$ oder $NO_2$), 2,4-Dichloro-Phe oder Pentafluoro-Phe darstellt;

jedes von $R^1$ und $R^2$ unabhängig ein Wasserstoffatom, niedriges Acyl oder niedriges Alkyl darstellt;

vorausgesetzt, daß mindestens eines von $A^1$ und $A^8$ eine aromatische Aminosäure sein muß; und weiters vorausgesetzt, daß, wenn entweder $A^2$ oder $A^7$ eine aromatische Aminosäure darstellt, $A^8$ keine aromatische Aminosäure sein kann;

$R^4$ nichts, einen geschützten Glycosyl-Rest, oder eine o-Benzylgruppe oder ein Kohlenhydrat darstellt;

$R^6$ eine Schutzgruppe darstellt, z.B. Boc;

$R^7$ ein Harz darstellt;

oder ein pharmazeutisch verträgliches Salz davon;

welches Verfahren umfaßt:

(a) Reaktion eines Harzes mit einem Aminosäurerest mit einer Seitenkette $A^8$ und einer Schutzgruppe;

(b) Reaktion der resultierenden harzgebundenen Verbindung mit einem Aminosäurerest mit einer Seitenkette, dargestellt durch $A^7$;

(c) Wiederholung von (b) mit Aminosäuren mit Seitenketten $A^7$, N-Benzyloxycarbonyl-Lys, D-Trp, $A^3$, $A^2$ und $A^1$ nacheinander; und

(d) wo und wenn notwendig, Reaktion der zubereiteten Verbindung oder einer der obengenannten Reste mit einem Glycosyl-Rest, einem Kohlenhydrat, einem Acylierungsmittel oder einem Alkylierungsmittel.

**9.** Verwendung einer Verbindung mit der allgemeinen Formel:

$$\begin{array}{l} R^1 \\ | \\ A^1 - A^2 - A^3 - D\text{-}Trp - Lys - A^6 - A^7 - A^8 - R^3 \\ | \\ R^2 \end{array}$$

worin

$A^1$ ein D-Isomer von Ala, Pyridyl-Ala, Leu, Ile, Val, Met, Nle, Trp, Beta-Nal, Phe, o-X-Phe (worin X $CH_3$, Cl, Br, F, OH, $OCH_3$ oder $NO_2$ darstellt), p-X-Phe (worin X = H, $CH_3$, Cl, Br, F, OH, $OCH_3$ oder $NO_2$), 2,4-Dichloro-Phe, Pentafluoro-Phe oder L-Phe darstellt;

$A^2$ Ala, Pyridyl-Ala, Leu, Ile, Val, Met, Nle, Trp, Beta-Nal, Phe, o-X-Phe (worin X $CH_3$, Cl, Br, F, OH, $OCH_3$ oder $NO_2$ darstellt), p-X-Phe (worin X = H, $CH_3$, Cl, Br, F, OH, $OCH_3$ oder $NO_2$), 2,4-Dichloro-Phe oder Pentafluoro-Phe darstellt;

$A^3$ Ala, Pyridyl-Ala, Leu, Ile, Val, Met, Nle, Trp, Tyr, Beta-Nal, Phe, o-X-Phe (worin X H, $CH_3$, Cl, Br, F, OH, $OCH_3$ oder $NO_2$ darstellt), p-X-Phe (worin X = H, $CH_3$, Cl, Br, F, OH, $OCH_3$ oder $NO_2$), 2,4-Dichloro-Phe oder Pentafluoro-Phe darstellt;

$A^6$ Ala, Pyridyl-Ala, Leu, Ile, Val, Lys, Met, Nle, Thr-$R^4$, Trp, Ser-$R^4$, Beta-Nal, o-X-Phe (worin X $CH_3$, Cl, Br, F, OH, $OCH_3$ oder $NO_2$ darstellt), p-X-Phe (worin X = $CH_3$, Cl, Br, F, OH, $OCH_3$ oder $NO_2$), 2,4-Dichloro-Phe oder Pentafluoro-Phe darstellt;

$A^7$ Ala, Pyridyl-Ala, Leu, Ile, Val, Met, Nle, Trp, Beta-Nal, Phe, o-X-Phe (worin X $CH_3$, Cl, Br, F, OH, $OCH_3$ oder $NO_2$ darstellt), p-X-Phe (worin X = H, $CH_3$, Cl, Br, F, OH, $OCH_3$ oder $NO_2$), 2,4-Dichloro-Phe oder Pentafluoro-Phe darstellt;

$A^8$ ein D- oder L-Isomer von Ala, Pyridyl-Ala, Leu, Ile, Ser-$R^4$, Thr-$R^4$, Val, Met, Nle, Trp, Beta-Nal, o-X-Phe (worin X $CH_3$, Cl, Br, F, OH, $OCH_3$ oder $NO_2$ darstellt), p-X-Phe (worin X = $CH_3$, Cl, Br, F, OH, $OCH_3$ oder $NO_2$), 2,4-Dichloro-Phe oder Pentafluoro-Phe darstellt;

jedes von $R^1$ und $R^2$ unabhängig ein Wasserstoffatom, niedriges Acyl oder niedriges Alkyl darstellt;

$R^3$ ein Wasserstoffatom, -$NH_2$ oder niedrige Alkylgruppe darstellt;

vorausgesetzt, daß mindestens eines von $A^1$ und $A^8$ eine aromatische Aminosäure sein muß; und weiters vorausgesetzt, daß, wenn entweder $A^2$ oder $A^7$ eine aromatische Aminosäure darstellt, $A^8$ keine aromatische Aminosäure sein kann;

$R^4$ nichts, einen geschützten Glycosyl-Rest oder ein Kohlenhydrat darstellt;

oder ein pharmazeutisch verträgliches Salz davon;

bei der Herstellung eines Mittels zur Behandlung oder Phrophylaxe von Krebs, Akromegalie, hyposekretorischen endokrinen Störungen, Diabetes, Zirrhose, Geschwulsten, Pankreatitis, Diarrhö, Hepatitis, Alzheimer-Krankheit, Pilzvergiftung oder Retinopathie.

**10.** Verfahren zur Herstellung einer pharmazeutischen Zusammensetzung, welches Verfahren das Beimischen umfaßt einer Verbindung mit der allgemeinen Formel:

$$\begin{array}{l} R^1 \\ | \\ A^1 - A^2 - A^3 - D\text{-}Trp - Lys - A^6 - A^7 - A^8 - R^3 \\ | \\ R^2 \end{array}$$

worin

$A^1$ ein D-Isomer von Ala, Pyridyl-Ala, Leu, Ile, Val, Met, Nle, Trp, Beta-Nal, Phe, o-X-Phe (worin X $CH_3$, Cl, Br, F, OH, $OCH_3$ oder $NO_2$ darstellt), p-X-Phe (worin X = H, $CH_3$, Cl, Br, F, OH, $OCH_3$ oder

$NO_2$), 2,4-Dichloro-Phe, Pentafluoro-Phe oder L-Phe darstellt;

$A^2$ Ala, Pyridyl-Ala, Leu, Ile, Val, Met, Nle, Trp, Beta-Nal, Phe, o-X-Phe (worin X $CH_3$, Cl, Br, F, OH, $OCH_3$ oder $NO_2$ darstellt), p-X-Phe (worin X = H, $CH_3$, Cl, Br, F, OH, $OCH_3$ oder $NO_2$), 2,4-Dichloro-Phe oder Pentafluoro-Phe darstellt;

$A^3$ Ala, Pyridyl-Ala, Leu, Ile, Val, Met, Nle, Trp, Tyr, Beta-Nal, Phe, o-X-Phe (worin X H, $CH_3$, Cl, Br, F, OH, $OCH_3$ oder $NO_2$ darstellt), p-X-Phe (worin X = H, $CH_3$, Cl, Br, F, OH, $OCH_3$ oder $NO_2$), 2,4-Dichloro-Phe oder Pentafluoro-Phe darstellt;

$A^6$ Ala, Pyridyl-Ala, Leu, Ile, Val, Lys, Met, Nle, Thr-$R^4$, Trp, Ser-$R^4$, Beta-Nal, o-X-Phe (worin X $CH_3$, Cl, Br, F, OH, $OCH_3$ oder $NO_2$ darstellt), p-X-Phe (worin X = $CH_3$, Cl, Br, F, OH, $OCH_3$ oder $NO_2$), 2,4-Dichloro-Phe oder Pentafluoro-Phe darstellt;

$A^7$ Ala, Pyridyl-Ala, Leu, Ile, Val, Met, Nle, Trp, Beta-Nal, Phe, o-X-Phe (worin X $CH_3$, Cl, Br, F, OH, $OCH_3$ oder $NO_2$ darstellt), p-X-Phe (worin X = H, $CH_3$, Cl, Br, F, OH, $OCH_3$ oder $NO_2$), 2,4-Dichloro-Phe oder Pentafluoro-Phe darstellt;

$A^8$ ein D- oder L-Isomer von Ala, Pyridyl-Ala, Leu, Ile, Ser-$R^4$, Thr-$R^4$, Val, Met, Nle, Trp, Beta-Nal, o-X-Phe (worin X $CH_3$, Cl, Br, F, OH, $OCH_3$ oder $NO_2$ darstellt), p-X-Phe (worin X = $CH_3$, Cl, Br, F, OH, $OCH_3$ oder $NO_2$), 2,4-Dichloro-Phe oder Pentafluoro-Phe darstellt;

jedes von $R^1$ und $R^2$ unabhängig ein Wasserstoffatom, niedriges Acyl oder niedriges Alkyl darstellt;

$R^3$ ein Wasserstoffatom, $-NH_2$ oder niedrige Alkylgruppe darstellt;

vorausgesetzt, daß mindestens eines von $A^1$ und $A^8$ eine aromatische Aminosäure sein muß; und weiters vorausgesetzt, daß, wenn entweder $A^2$ oder $A^7$ eine aromatische Aminosäure darstellt, $A^8$ keine aromatische Aminosäure sein kann;

$R^4$ nichts, einen geschützten Glycosyl-Rest oder ein Kohlenhydrat darstellt;

oder ein pharmazeutisch verträgliches Salz davon;

welches Verfahren das sukzessive Verbinden der Aminosäurereste umfaßt;

zu einem pharmazeutisch verträglichen Träger.

**11.** Verfahren nach Anspruch 9, in welchem die Zusammensetzung mit einer Substanz umhüllt wird, welche imstande ist, die Zusammensetzung vor der Magensäure im Magen einen ausreichenden Zeitraum zu schützen, in welchem die Verbindung unaufgelöst in den Dünndarm gelangen kann.

## Revendications

### Revendications pour les Etats contractants suivants : AT, BE, CH, DE, DK, FR, GB, IT, LI, LU, NL, SE

**1.** Composé de formule générale :

$$\begin{array}{l} R^1 \\ | \\ A^1 - A^2 - A^3 - D\text{-}Trp - Lys - A^6 - A^7 - A^8 - R^3 \\ | \\ R^2 \end{array}$$

où :

$A^1$ représente un isomère D de Ala, pyridyl-Ala, Leu, Ile, Val, Met, Nle, Trp, $\beta$-Nal, Phe, o-X-Phe (où X représente $CH_3$, Cl, Br, F, OH, $OCH_3$ ou $NO_2$), p-X-Phe (où X représente H, $CH_3$, Cl, Br, F, OH, $OCH_3$ ou $NO_2$), 2,4-dichloro-Phe, pentafluoro-Phe ou L-Phe;

$A^2$ représente Ala, pyridyl-Ala, Leu, Ile, Val, Met, Nle, Trp, $\beta$-Nal, Phe, o-X-Phe (où X représente $CH_3$, Cl, Br, F, OH, $OCH_3$ ou $NO_2$), p-X-Phe (où X représente H, $CH_3$, Cl, Br, F, OH, $OCH_3$ ou $NO_2$), 2,4-dichloro-Phe ou pentafluoro-Phe;

$A^3$ représente Ala, pyridyl-Ala, Leu, Ile, Val, Met, Nle, Trp, Tyr, $\beta$-Nal, Phe, o-X-Phe (où X représente H, $CH_3$, Cl, Br, F, OH, $OCH_3$ ou $NO_2$), p-X-Phe (où X représente H, $CH_3$, Cl, Br, F, OH, $OCH_3$ ou $NO_2$), 2,4-dichloro-Phe ou pentafluoro-Phe;

$A^6$ représente Ala, pyridyl-Ala, Leu, Ile, Val, Lys, Met, Nle, Thr-$R^4$, Trp, Ser-$R^4$, $\beta$-Nal, o-X-Phe (où X représente $CH_3$, Cl, Br, F, OH, $OCH_3$ ou $NO_2$), p-X-Phe (où X représente $CH_3$, Cl, Br, F, OH, $OCH_3$ ou $NO_2$), 2,4-dichloro-Phe ou pentafluoro-Phe;

$A^7$ représente Ala, pyridyl-Ala, Leu, Ile, Val, Met, Nle, Trp, $\beta$-Nal, Phe, o-X-Phe (où X représente $CH_3$, Cl, Br, F, OH, $OCH_3$ ou $NO_2$), p-X-Phe (où X représente H, $CH_3$, Cl, Br, F, OH, $OCH_3$ ou $NO_2$), 2,4-dichloro-Phe ou pentafluoro-Phe;

$A^8$ représente un isomère D ou L de Ala, pyridyl-Ala, Leu, Ile, Ser-$R^4$, Thr-$R^4$, Val, Met, Nle, Trp, $\beta$-Nal, o-X-Phe (où X représente $CH_3$, Cl, Br, F, OH, $OCH_3$ ou $NO_2$), p-X-Phe (où X représente $CH_3$, Cl, Br, F, OH, $OCH_3$ ou $NO_2$), 2,4-dichloro-Phe ou pentafluoro-Phe;

chaque $R^1$ et $R^2$ représente indépendamment un atome d'hydrogène, un acyle inférieur ou un alcoyle inférieur;

$R^3$ représente un atome d'hydrogène, un $NH_2$ ou un radical alcoyle inférieur;

pourvu qu'au moins un des $A^1$ et $A^8$ soit un acide aminé aromatique et, en outre, pourvu que si $A^2$ ou $A^7$ représente un acide aminé aromatique, alors $A^8$ ne peut pas être un acide aminé aromatique;

$R^4$ représente rien, un résidu glycosyle protégé ou un hydrate de carbone;

ou un sel pharmaceutiquement acceptable de celui-ci.

**2.** Composé suivant la revendication 1, dans lequel un, mais pas les deux, de $A^1$ et $A^2$, représente un acide aminé aromatique, et dans lequel un, mais pas les deux, de $A^7$ et $A^8$, représente un acide aminé aromatique.

**3.** Composé suivant la revendication 1 ou 2, dans lequel :

$A^1$ représente un isomère D de Trp, $\beta$-Nal, o-X-Phe (où X représente $CH_3$ ou $OCH_3$), p-X-Phe (où X représente $CH_3$ ou $OCH_3$), et

$A^8$ représente un isomère D ou L de Ala, pyridyl-Ala, Leu, Ile, Ser, Thr, Val, Met, Nle, o-X-Phe (où X représente Cl, Br, F, OH ou $NO_2$), p-X-Phe (où X représente Cl, Br, F, OH ou $NO_2$), 2,4-dichloro-Phe ou pentafluoro-Phe.

**4.** Composé suivant l'une quelconque des revendications 1 à 3, qui présente une, quelques-unes ou toutes les caractéristiques suivantes :

$A^1$ représente $\beta$-D-Nal ou D-Phe;

$A^2$ représente Ala, Phe ou p-chloro-Phe;

$A^3$ représente Tyr ou Phe;

$A^6$ représente Val, Lys ou Thr;

$A^7$ représente Ala ou Phe, et/ou

$A^8$ représente Thr ou $\beta$-D-Nal.

**5.** Composé suivant l'une quelconque des revendications 1 à 4, qui est :

D-Phe-Phe-Phe-D-Trp-Lys-Thr-Phe-Thr-$NH_2$ [SEQ ID N°3];

D-Phe-Phe-Tyr-D-Trp-Lys-Val-Phe-Thr-$NH_2$ [SEQ ID N°2];

D-Phe-p-chloro-Phe-Tyr-D-Trp-Lys-Val-Phe-Thr-$NH_2$ [SEQ ID N°1], ou

D-Phe-Ala-Tyr-D-Trp-Lys-Val-Ala-$\beta$-D-Nal-$NH_2$ [SEQ ID N°4].

**6.** Composé de formule générale :

$R^1$
|
$A^1$ - $A^2$ - $A^3$ - D-Trp - Lys - $A^6$ - $A^7$ - $A^8$ - $R^3$
|
$R^2$

où :

$A^1$ représente un isomère D de Ala, pyridyl-Ala, Leu, Ile, Val, Met, Nle, Trp, $\beta$-Nal, Phe, o-X-Phe (où X représente $CH_3$, Cl, Br, F, OH, $OCH_3$ ou $NO_2$), p-X-Phe (où X représente H, $CH_3$, Cl, Br, F, OH, $OCH_3$ ou $NO_2$), 2,4-dichloro-Phe, pentafluoro-Phe ou L-Phe;

$A^2$ représente Ala, pyridyl-Ala, Leu, Ile, Val, Met, Nle, Trp, $\beta$-Nal, Phe, o-X-Phe (où X représente $CH_3$, Cl, Br, F, OH, $OCH_3$ ou $NO_2$), p-X-Phe (où X représente H, $CH_3$, Cl, Br, F, OH, $OCH_3$ ou $NO_2$), 2,4-dichloro-Phe ou pentafluoro-Phe;

$A^3$ représente Ala, pyridyl-Ala, Leu, Ile, Val, Met, Nle, Trp, Tyr, $\beta$-Nal, Phe, o-X-Phe (où X représente H, $CH_3$, Cl, Br, F, OH, $OCH_3$ ou $NO_2$), p-X-Phe (où X représente H, $CH_3$, Cl, Br, F, OH, $OCH_3$ ou $NO_2$), 2,4-dichloro-Phe ou pentafluoro-Phe;

$A^6$ représente Ala, pyridyl-Ala, Leu, Ile, Val, Lys, Met, Nle, Thr-$R^4$, Trp, Ser-$R^4$, $\beta$-Nal, o-X-Phe (où X représente $CH_3$, Cl, Br, F, OH, $OCH_3$ ou $NO_2$), p-X-Phe (où X représente $CH_3$, Cl, Br, F, OH, $OCH_3$

ou $NO_2$), 2,4-dichloro-Phe ou pentafluoro-Phe;

$A^7$ représente Ala, pyridyl-Ala, Leu, Ile, Val, Met, Nle, Trp, $\beta$-Nal, Phe, o-X-Phe (où X représente $CH_3$, Cl, Br, F, OH, $OCH_3$ ou $NO_2$), p-X-Phe (où X représente H, $CH_3$, Cl, Br, F, OH, $OCH_3$ ou $NO_2$), 2,4-dichloro-Phe ou pentafluoro-Phe;

$A^8$ représente un isomère D ou L de Ala, pyridyl-Ala, Leu, Ile, Ser-$R^4$, Thr-$R^4$, Val, Met, Nle, Trp, $\beta$-Nal, o-X-Phe (où X représente $CH_3$, Cl, Br, F, OH, $OCH_3$ ou $NO_2$), p-X-Phe (où X représente $CH_3$, Cl, Br, F, OH, $OCH_3$ ou $NO_2$), 2,4-dichloro-Phe ou pentafluoro-Phe;

chaque $R^1$ et $R^2$ représente indépendamment un atome d'hydrogène, un acyle inférieur ou un alcoyle inférieur;

$R^3$ représente un atome d'hydrogène, un $NH_2$ ou un radical alcoyle inférieur;

pourvu qu'au moins un des $A^1$ et $A^8$ soit un acide aminé aromatique et, en outre, pourvu que si $A^2$ ou $A^7$ représente un acide aminé aromatique, alors $A^8$ ne peut pas être un acide aminé aromatique;

$R^4$ représente rien, un résidu glycosyle protégé ou un hydrate de carbone;

ou un sel pharmaceutiquement acceptable de celui-ci, à utiliser en médecine.

**7.** Composition pharmaceutique comprenant un composé de formule générale :

$$\begin{array}{l} R^1 \\ | \\ A^1 - A^2 - A^3 - \text{D-Trp} - \text{Lys} - A^6 - A^7 - A^8 - R^3 \\ | \\ R^2 \end{array}$$

où :

$A^1$ représente un isomère D de Ala, pyridyl-Ala, Leu, Ile, Val, Met, Nle, Trp, $\beta$-Nal, Phe, o-X-Phe (où X représente $CH_3$, Cl, Br, F, OH, $OCH_3$ ou $NO_2$), p-X-Phe (où X représente H, $CH_3$, Cl, Br, F, OH, $OCH_3$ ou $NO_2$), 2,4-dichloro-Phe, pentafluoro-Phe ou L-Phe;

$A^2$ représente Ala, pyridyl-Ala, Leu, Ile, Val, Met, Nle, Trp, $\beta$-Nal, Phe, o-X-Phe (où X représente $CH_3$, Cl, Br, F, OH, $OCH_3$ ou $NO_2$), p-X-Phe (où X représente H, $CH_3$, Cl, Br, F, OH, $OCH_3$ ou $NO_2$), 2,4-dichloro-Phe ou pentafluoro-Phe;

$A^3$ représente Ala, pyridyl-Ala, Leu, Ile, Val, Met, Nle, Trp, Tyr, $\beta$-Nal, Phe, o-X-Phe (où X représente H, $CH_3$, Cl, Br, F, OH, $OCH_3$ ou $NO_2$), p-X-Phe (où X représente H, $CH_3$, Cl, Br, F, OH, $OCH_3$ ou $NO_2$), 2,4-dichloro-Phe ou pentafluoro-Phe;

$A^6$ représente Ala, pyridyl-Ala, Leu, Ile, Val, Lys, Met, Nle, Thr-$R^4$, Trp, Ser-$R^4$, $\beta$-Nal, o-X-Phe (où X représente $CH_3$, Cl, Br, F, OH, $OCH_3$ ou $NO_2$), p-X-Phe (où X représente $CH_3$, Cl, Br, F, OH, $OCH_3$ ou $NO_2$), 2,4-dichloro-Phe ou pentafluoro-Phe;

$A^7$ représente Ala, pyridyl-Ala, Leu, Ile, Val, Met, Nle, Trp, $\beta$-Nal, Phe, o-X-Phe (où X représente $CH_3$, Cl, Br, F, OH, $OCH_3$ ou $NO_2$), p-X-Phe (où X représente H, $CH_3$, Cl, Br, F, OH, $OCH_3$ ou $NO_2$), 2,4-dichloro-Phe ou pentafluoro-Phe;

$A^8$ représente un isomère D ou L de Ala, pyridyl-Ala, Leu, Ile, Ser-$R^4$, Thr-$R^4$, Val, Met, Nle, Trp, $\beta$-Nal, o-X-Phe (où X représente $CH_3$, Cl, Br, F, OH, $OCH_3$ ou $NO_2$), p-X-Phe (où X représente $CH_3$, Cl, Br, F, OH, $OCH_3$ ou $NO_2$), 2,4-dichloro-Phe ou pentafluoro-Phe;

chaque $R^1$ et $R^2$ représente indépendamment un atome d'hydrogène, un acyle inférieur ou un alcoyle inférieur;

$R^3$ représente un atome d'hydrogène, un $NH_2$ ou un radical alcoyle inférieur;

pourvu qu'au moins un des $A^1$ et $A^8$ soit un acide aminé aromatique et, en outre, pourvu que si $A^2$ ou $A^7$ représente un acide aminé aromatique, alors $A^8$ ne peut pas être un acide aminé aromatique;

$R^4$ représente rien, un résidu glycosyle protégé ou un hydrate de carbone;

ou un sel pharmaceutiquement acceptable de celui-ci,

et un support pharmaceutiquement acceptable.

**8.** Composition suivant la revendication 7, qui est enrobée d'une substance capable de protéger la composition de l'acide gastrique dans l'estomac, pendant une période suffisante pour permettre à la composition de passer non désintégrée dans l'intestin grêle.

**9.** Procédé de préparation d'un composé de formule générale :

$$\begin{array}{l} R^1 \\ | \\ A^1 - A^2 - A^3 - \text{D-Trp} - \text{Lys} - A^6 - A^7 - A^8 - R^3 \\ | \\ R^2 \end{array}$$

où :

$A^1$ représente un isomère D de Ala, pyridyl-Ala, Leu, Ile, Val, Met, Nle, Trp, β-Nal, Phe, o-X-Phe (où X représente $CH_3$, Cl, Br, F, OH, $OCH_3$ ou $NO_2$), p-X-Phe (où X représente H, $CH_3$, Cl, Br, F, OH, $OCH_3$ ou $NO_2$), 2,4-dichloro-Phe, pentafluoro-Phe ou L-Phe;

$A^2$ représente Ala, pyridyl-Ala, Leu, Ile, Val, Met, Nle, Trp, β-Nal, Phe, o-X-Phe (où X représente $CH_3$, Cl, Br, F, OH, $OCH_3$ ou $NO_2$), p-X-Phe (où X représente H, $CH_3$, Cl, Br, F, OH, $OCH_3$ ou $NO_2$), 2,4-dichloro-Phe ou pentafluoro-Phe;

$A^3$ représente Ala, pyridyl-Ala, Leu, Ile, Val, Met, Nle, Trp, Tyr, β-Nal, Phe, o-X-Phe (où X représente H, $CH_3$, Cl, Br, F, OH, $OCH_3$ ou $NO_2$), p-X-Phe (où X représente H, $CH_3$, Cl, Br, F, OH, $OCH_3$ ou $NO_2$), 2,4-dichloro-Phe ou pentafluoro-Phe;

$A^6$ représente Ala, pyridyl-Ala, Leu, Ile, Val, Lys, Met, Nle, Thr-$R^4$, Trp, Ser-$R^4$, β-Nal, o-X-Phe (où X représente $CH_3$, Cl, Br, F, OH, $OCH_3$ ou $NO_2$), p-X-Phe (où X représente $CH_3$, Cl, Br, F, OH, $OCH_3$ ou $NO_2$), 2,4-dichloro-Phe ou pentafluoro-Phe;

$A^7$ représente Ala, pyridyl-Ala, Leu, Ile, Val, Met, Nle, Trp, β-Nal, Phe, o-X-Phe (où X représente $CH_3$, Cl, Br, F, OH, $OCH_3$ ou $NO_2$), p-X-Phe (où X représente H, $CH_3$, Cl, Br, F, OH, $OCH_3$ ou $NO_2$), 2,4-dichloro-Phe ou pentafluoro-Phe;

$A^8$ représente un isomère D ou L de Ala, pyridyl-Ala, Leu, Ile, Ser-$R^4$, Thr-$R^4$, Val, Met, Nle, Trp, β-Nal, o-X-Phe (où X représente $CH_3$, Cl, Br, F, OH, $OCH_3$ ou $NO_2$), p-X-Phe (où X représente $CH_3$, Cl, Br, F, OH, $OCH_3$ ou $NO_2$), 2,4-dichloro-Phe ou pentafluoro-Phe;

chaque $R^1$ et $R^2$ représente indépendamment un atome d'hydrogène, un acyle inférieur ou un alcoyle inférieur;

$R^3$ représente un atome d'hydrogène, un $NH_2$ ou un radical alcoyle inférieur;

pourvu qu'au moins un des $A^1$ et $A^8$ soit un acide aminé aromatique et, en outre, pourvu que si $A^2$ ou $A^7$ représente un acide aminé aromatique, alors $A^8$ ne peut pas être un acide aminé aromatique;

$R^4$ représente rien, un résidu glycosyle protégé ou un hydrate de carbone;

ou un sel pharmaceutiquement acceptable de celui-ci,

le procédé comprenant la réaction d'un composé de formule générale :

$$\begin{array}{l} \quad\;\; R^1 \\ \quad\;\; | \\ R^6 - A^1 - A^2 - A^3 - \text{D-Trp} - \text{N-benzyloxycarbonyl-Lys} - A^6 - \\ \quad\;\; | \hspace{22em} A^7 - A^8 - R^7 \\ \quad\;\; R^2 \end{array}$$

où :

$R^1$, $R^2$, $A^1$, $A^2$, $A^7$ et $A^8$ sont tels que définis précédemment;

$A^3$ est tel que défini précédemment sauf que Tyr est remplacé par o-benzyl-Tyr;

$A^6$ est tel que défini précédemment sauf que Lys est remplacé par N-benzyloxycarbonyl-Lys;

$R^4$ peut, en outre, représenter un radical o-benzyle;

$R^6$ représente un groupe protecteur, et

$R^7$ représente une résine,

avec :

du crésol;

du dithiothréitol, et

du fluorure d'hydrogène.

**10.** Composé de formule générale :

$$R^6 - \underset{\substack{| \\ R^2}}{\overset{\substack{R^1 \\ |}}{A^1}} - A^2 - A^3 - \text{D-Trp} - \text{N-benzyloxycarbonyl-Lys} - A^6 - A^7 - A^8 - R^7$$

où :

$A^1$ représente un isomère D de Ala, pyridyl-Ala, Leu, Ile, Val, Met, Nle, Trp, $\beta$-Nal, Phe, o-X-Phe (où X représente $CH_3$, Cl, Br, F, OH, $OCH_3$ ou $NO_2$), p-X-Phe (où X représente H, $CH_3$, Cl, Br, F, OH, $OCH_3$ ou $NO_2$), 2,4-dichloro-Phe, pentafluoro-Phe ou L-Phe;

$A^2$ représente Ala, pyridyl-Ala, Leu, Ile, Val, Met, Nle, Trp, $\beta$-Nal, Phe, o-X-Phe (où X représente $CH_3$, Cl, Br, F, OH, $OCH_3$ ou $NO_2$), p-X-Phe (où X représente H, $CH_3$, Cl, Br, F, OH, $OCH_3$ ou $NO_2$), 2,4-dichloro-Phe ou pentafluoro-Phe;

$A^3$ représente Ala, pyridyl-Ala, Leu, Ile, Val, Met, Nle, Trp, o-benzyl-Tyr, $\beta$-Nal, Phe, o-X-Phe (où X représente H, $CH_3$, Cl, Br, F, OH, $OCH_3$ ou $NO_2$), p-X-Phe (où X représente H, $CH_3$, Cl, Br, F, OH, $OCH_3$ ou $NO_2$), 2,4-dichloro-Phe ou pentafluoro-Phe;

$A^6$ représente Ala, pyridyl-Ala, Leu, Ile, Val, N-benzyloxycarbonyl-Lys, Met, Nle, Thr-$R^4$, Trp, Ser-$R^4$, $\beta$-Nal, o-X-Phe (où X représente $CH_3$, Cl, Br, F, OH, $OCH_3$ ou $NO_2$), p-X-Phe (où X représente $CH_3$, Cl, Br, F, OH, $OCH_3$ ou $NO_2$), 2,4-dichloro-Phe ou pentafluoro-Phe;

$A^7$ représente Ala, pyridyl-Ala, Leu, Ile, Val, Met, Nle, Trp, $\beta$-Nal, Phe, o-X-Phe (où X représente $CH_3$, Cl, Br, F, OH, $OCH_3$ ou $NO_2$), p-X-Phe (où X représente H, $CH_3$, Cl, Br, F, OH, $OCH_3$ ou $NO_2$), 2,4-dichloro-Phe ou pentafluoro-Phe;

$A^8$ représente un isomère D ou L de Ala, pyridyl-Ala, Leu, Ile, Ser-$R^4$, Thr-$R^4$, Val, Met, Nle, Trp, $\beta$-Nal, o-X-Phe (où X représente $CH_3$, Cl, Br, F, OH, $OCH_3$ ou $NO_2$), p-X-Phe (où X représente $CH_3$, Cl, Br, F, OH, $OCH_3$ ou $NO_2$), 2,4-dichloro-Phe ou pentafluoro-Phe;

chaque $R^1$ et $R^2$ représente indépendamment un atome d'hydrogène, un acyle inférieur ou un alcoyle inférieur;

pourvu qu'au moins un des $A^1$ et $A^8$ soit un acide aminé aromatique et, en outre, pourvu que si $A^2$ ou $A^7$ représente un acide aminé aromatique, alors $A^8$ ne peut pas être un acide aminé aromatique;

$R^4$ représente rien, un résidu glycosyle protégé, un radical o-benzyle ou un hydrate de carbone;

$R^6$ représente un groupe protecteur, par exemple, Boc;

$R^7$ représente une résine,

ou un sel pharmaceutiquement acceptable de celui-ci.

**11.** Procédé de préparation d'un composé de formule générale :

$$R^6 - \underset{\substack{| \\ R^2}}{\overset{\substack{R^1 \\ |}}{A^1}} - A^2 - A^3 - \text{D-Trp} - \text{N-benzyloxycarbonyl-Lys} - A^6 - A^7 - A^8 - R^7$$

où :

$A^1$ représente un isomère D de Ala, pyridyl-Ala, Leu, Ile, Val, Met, Nle, Trp, $\beta$-Nal, Phe, o-X-Phe (où X représente $CH_3$, Cl, Br, F, OH, $OCH_3$ ou $NO_2$), p-X-Phe (où X représente H, $CH_3$, Cl, Br, F, OH, $OCH_3$ ou $NO_2$), 2,4-dichloro-Phe, pentafluoro-Phe ou L-Phe;

$A^2$ représente Ala, pyridyl-Ala, Leu, Ile, Val, Met, Nle, Trp, $\beta$-Nal, Phe, o-X-Phe (où X représente $CH_3$, Cl, Br, F, OH, $OCH_3$ ou $NO_2$), p-X-Phe (où X représente H, $CH_3$, Cl, Br, F, OH, $OCH_3$ ou $NO_2$), 2,4-dichloro-Phe ou pentafluoro-Phe;

$A^3$ représente Ala, pyridyl-Ala, Leu, Ile, Val, Met, Nle, Trp, o-benzyl-Tyr, $\beta$-Nal, Phe, o-X-Phe (où X représente H, $CH_3$, Cl, Br, F, OH, $OCH_3$ ou $NO_2$), p-X-Phe (où X représente H, $CH_3$, Cl, Br, F, OH, $OCH_3$ ou $NO_2$), 2,4-dichloro-Phe ou pentafluoro-Phe;

$A^6$ représente Ala, pyridyl-Ala, Leu, Ile, Val, N-benzyloxycarbonyl-Lys, Met, Nle, Thr-$R^4$, Trp, Ser-$R^4$, $\beta$-Nal, o-X-Phe (où X représente $CH_3$, Cl, Br, F, OH, $OCH_3$ ou $NO_2$), p-X-Phe (où X représente

$CH_3$, Cl, Br, F, OH, $OCH_3$ ou $NO_2$), 2,4-dichloro-Phe ou pentafluoro-Phe;

$A^7$ représente Ala, pyridyl-Ala, Leu, Ile, Val, Met, Nle, Trp, $\beta$-Nal, Phe, o-X-Phe (où X représente $CH_3$, Cl, Br, F, OH, $OCH_3$ ou $NO_2$), p-X-Phe (où X représente H, $CH_3$, Cl, Br, F, OH, $OCH_3$ ou $NO_2$), 2,4-dichloro-Phe ou pentafluoro-Phe;

$A^8$ représente un isomère D ou L de Ala, pyridyl-Ala, Leu, Ile, Ser-$R^4$, Thr-$R^4$, Val, Met, Nle, Trp, $\beta$-Nal, o-X-Phe (où X représente $CH_3$, Cl, Br, F, OH, $OCH_3$ ou $NO_2$), p-X-Phe (où X représente $CH_3$, Cl, Br, F, OH, $OCH_3$ ou $NO_2$), 2,4-dichloro-Phe ou pentafluoro-Phe;

chaque $R^1$ et $R^2$ représente indépendamment un atome d'hydrogène, un acyle inférieur ou un alcoyle inférieur;

pourvu qu'au moins un des $A^1$ et $A^8$ soit un acide aminé aromatique et, en outre, pourvu que si $A^2$ ou $A^7$ représente un acide aminé aromatique, alors $A^8$ ne peut pas être un acide aminé aromatique;

$R^4$ représente rien, un résidu glycosyle protégé, un radical o-benzyle ou un hydrate de carbone;

$R^6$ représente un groupe protecteur, par exemple, Boc;

$R^7$ représente une résine,

ou un sel pharmaceutiquement acceptable de celui-ci,

le procédé comprenant :

a) la réaction d'une résine avec un résidu acide aminé ayant une chaîne latérale $A^8$ et un groupe protecteur;

b) la réaction du composé lié à la résine résultant avec un résidu acide aminé de chaîne latérale représentée par $A^7$;

c) la répétition de b) avec les acides aminés de chaîne latérale N-benzyloxycarbonyl-Lys, D-Trp, $A^3$, $A^2$ et $A^1$, de manière consécutive, et

d) si nécessaire et là où il est nécessaire, réaction du composé préparé ou de l'un des résidus ci-dessus avec un résidu glycosyle, un hydrate de carbone, un agent acylant ou un agent alcoylant.

**12.** Utilisation d'un composé de formule générale :

$$\begin{array}{l} R^1 \\ | \\ A^1 - A^2 - A^3 - \text{D-Trp} - \text{Lys} - A^6 - A^7 - A^8 - R^3 \\ | \\ R^2 \end{array}$$

où :

$A^1$ représente un isomère D de Ala, pyridyl-Ala, Leu, Ile, Val, Met, Nle, Trp, $\beta$-Nal, Phe, o-X-Phe (où X représente $CH_3$, Cl, Br, F, OH, $OCH_3$ ou $NO_2$), p-X-Phe (où X représente H, $CH_3$, Cl, Br, F, OH, $OCH_3$ ou $NO_2$), 2,4-dichloro-Phe, pentafluoro-Phe ou L-Phe;

$A^2$ représente Ala, pyridyl-Ala, Leu, Ile, Val, Met, Nle, Trp, $\beta$-Nal, Phe, o-X-Phe (où X représente $CH_3$, Cl, Br, F, OH, $OCH_3$ ou $NO_2$), p-X-Phe (où X représente H, $CH_3$, Cl, Br, F, OH, $OCH_3$ ou $NO_2$), 2,4-dichloro-Phe ou pentafluoro-Phe;

$A^3$ représente Ala, pyridyl-Ala, Leu, Ile, Val, Met, Nle, Trp, Tyr, $\beta$-Nal, Phe, o-X-Phe (où X représente H, $CH_3$, Cl, Br, F, OH, $OCH_3$ ou $NO_2$), p-X-Phe (où X représente H, $CH_3$, Cl, Br, F, OH, $OCH_3$ ou $NO_2$), 2,4-dichloro-Phe ou pentafluoro-Phe;

$A^6$ représente Ala, pyridyl-Ala, Leu, Ile, Val, Lys, Met, Nle, Thr-$R^4$, Trp, Ser-$R^4$, $\beta$-Nal, o-X-Phe (où X représente $CH_3$, Cl, Br, F, OH, $OCH_3$ ou $NO_2$), p-X-Phe (où X représente $CH_3$, Cl, Br, F, OH, $OCH_3$ ou $NO_2$), 2,4-dichloro-Phe ou pentafluoro-Phe;

$A^7$ représente Ala, pyridyl-Ala, Leu, Ile, Val, Met, Nle, Trp, $\beta$-Nal, Phe, o-X-Phe (où X représente $CH_3$, Cl, Br, F, OH, $OCH_3$ ou $NO_2$), p-X-Phe (où X représente H, $CH_3$, Cl, Br, F, OH, $OCH_3$ ou $NO_2$), 2,4-dichloro-Phe ou pentafluoro-Phe;

$A^8$ représente un isomère D ou L de Ala, pyridyl-Ala, Leu, Ile, Ser-$R^4$, Thr-$R^4$, Val, Met, Nle, Trp, $\beta$-Nal, o-X-Phe (où X représente $CH_3$, Cl, Br, F, OH, $OCH_3$ ou $NO_2$), p-X-Phe (où X représente $CH_3$, Cl, Br, F, OH, $OCH_3$ ou $NO_2$), 2,4-dichloro-Phe ou pentafluoro-Phe;

chaque $R^1$ et $R^2$ représente indépendamment un atome d'hydrogène, un acyle inférieur ou un alcoyle inférieur;

$R^3$ représente un atome d'hydrogène, un $NH_2$ ou un radical alcoyle inférieur;

pourvu qu'au moins un des $A^1$ et $A^8$ soit un acide aminé aromatique et, en outre, pourvu que si $A^2$ ou $A^7$ représente un acide aminé aromatique, alors $A^8$ ne peut pas être un acide aminé aromatique;

$R^4$ représente rien, un résidu glycosyle protégé ou un hydrate de carbone;
ou un sel pharmaceutiquement acceptable de celui-ci,
dans la préparation d'un agent dans le traitement ou la prophylaxie du cancer, de l'acromégalie, de désordres endocriniens hyposécrétoires, de diabètes, de cirrhoses, d'ulcères, de pancréatites, de diarrhées, d'hépatites, de maladie d'Alzheimer, d'empoisonnement par un champignon ou de rétinopathie.

**Revendications pour l'Etat contractant suivant : ES**

1. Procédé de préparation d'un composé de formule générale :

$$\begin{array}{l} R^1 \\ | \\ A^1 - A^2 - A^3 - \text{D-Trp} - \text{Lys} - A^6 - A^7 - A^8 - R^3 \\ | \\ R^2 \end{array}$$

où :
$A^1$ représente un isomère D de Ala, pyridyl-Ala, Leu, Ile, Val, Met, Nle, Trp, $\beta$-Nal, Phe, o-X-Phe (où X représente $CH_3$, Cl, Br, F, OH, $OCH_3$ ou $NO_2$), p-X-Phe (où X représente H, $CH_3$, Cl, Br, F, OH, $OCH_3$ ou $NO_2$), 2,4-dichloro-Phe, pentafluoro-Phe ou L-Phe;
$A^2$ représente Ala, pyridyl-Ala, Leu, Ile, Val, Met, Nle, Trp, $\beta$-Nal, Phe, o-X-Phe (où X représente $CH_3$, Cl, Br, F, OH, $OCH_3$ ou $NO_2$), p-X-Phe (où X représente H, $CH_3$, Cl, Br, F, OH, $OCH_3$ ou $NO_2$), 2,4-dichloro-Phe ou pentafluoro-Phe;
$A^3$ représente Ala, pyridyl-Ala, Leu, Ile, Val, Met, Nle, Trp, Tyr, $\beta$-Nal, Phe, o-X-Phe (où X représente H, $CH_3$, Cl, Br, F, OH, $OCH_3$ ou $NO_2$), p-X-Phe (où X représente H, $CH_3$, Cl, Br, F, OH, $OCH_3$ ou $NO_2$), 2,4-dichloro-Phe ou pentafluoro-Phe;
$A^6$ représente Ala, pyridyl-Ala, Leu, Ile, Val, Lys, Met, Nle, Thr-$R^4$, Trp, Ser-$R^4$, $\beta$-Nal, o-X-Phe (où X représente $CH_3$, Cl, Br, F, OH, $OCH_3$ ou $NO_2$), p-X-Phe (où X représente $CH_3$, Cl, Br, F, OH, $OCH_3$ ou $NO_2$), 2,4-dichloro-Phe ou pentafluoro-Phe;
$A^7$ représente Ala, pyridyl-Ala, Leu, Ile, Val, Met, Nle, Trp, $\beta$-Nal, Phe, o-X-Phe (où X représente $CH_3$, Cl, Br, F, OH, $OCH_3$ ou $NO_2$), p-X-Phe (où X représente H, $CH_3$, Cl, Br, F, OH, $OCH_3$ ou $NO_2$), 2,4-dichloro-Phe ou pentafluoro-Phe;
$A^8$ représente un isomère D ou L de Ala, pyridyl-Ala, Leu, Ile, Ser-$R^4$, Thr-$R^4$, Val, Met, Nle, Trp, $\beta$-Nal, o-X-Phe (où X représente $CH_3$, Cl, Br, F, OH, $OCH_3$ ou $NO_2$), p-X-Phe (où X représente $CH_3$, Cl, Br, F, OH, $OCH_3$ ou $NO_2$), 2,4-dichloro-Phe ou pentafluoro-Phe;
chaque $R^1$ et $R^2$ représente indépendamment un atome d'hydrogène, un acyle inférieur ou un alcoyle inférieur;
$R^3$ représente un atome d'hydrogène, un $NH_2$ ou un radical alcoyle inférieur;
pourvu qu'au moins un des $A^1$ et $A^8$ soit un acide aminé aromatique et, en outre, pourvu que si $A^2$ ou $A^7$ représente un acide aminé aromatique, alors $A^8$ ne peut pas être un acide aminé aromatique;
$R^4$ représente rien, un résidu glycosyle protégé ou un hydrate de carbone;
ou un sel pharmaceutiquement acceptable de celui-ci,
le procédé comprenant le couplage successif de résidus acide aminé les uns aux autres.

2. Procédé suivant la revendication 1, dans lequel un, mais pas les deux, de $A^1$ et $A^2$, représente un acide aminé aromatique, et dans lequel un, mais pas les deux, de $A^7$ et $A^8$, représente un acide aminé aromatique.

3. Procédé suivant la revendication 1 ou 2, dans lequel :
$A^1$ représente un isomère D de Trp, $\beta$-Nal, o-X-Phe (où X représente $CH_3$ ou $OCH_3$), p-X-Phe (où X représente $CH_3$ ou $OCH_3$), et
$A^8$ représente un isomère D ou L de Ala, pyridyl-Ala, Leu, Ile, Ser, Thr, Val, Met, Nle, o-X-Phe (où X représente Cl, Br, F, OH ou $NO_2$), p-X-Phe (où X représente Cl, Br, F, OH ou $NO_2$), 2,4-dichloro-Phe ou pentafluoro-Phe.

4. Procédé suivant l'une quelconque des revendications 1 à 3, qui présente une, quelques-unes ou toutes les caractéristiques suivantes :
   - $A^1$ représente $\beta$-D-Nal ou D-Phe;
   - $A^2$ représente Ala, Phe ou p-chloro-Phe;
   - $A^3$ représente Tyr ou Phe;
   - $A^6$ représente Val, Lys ou Thr;
   - $A^7$ représente Ala ou Phe, et/ou
   - $A^8$ représente Thr ou $\beta$-D-Nal.

5. Procédé suivant l'une quelconque des revendications 1 à 4, dans lequel le composé est :
   - D-Phe-Phe-Phe-D-Trp-Lys-Thr-Phe-Thr-$NH_2$ [SEQ ID N°3];
   - D-Phe-Phe-Tyr-D-Trp-Lys-Val-Phe-Thr-$NH_2$ [SEQ ID N°2];
   - D-Phe-p-chloro-Phe-Tyr-D-Trp-Lys-Val-Phe-Thr-$NH_2$ [SEQ ID N°1], ou
   - D-Phe-Ala-Tyr-D-Trp-Lys-Val-Ala-$\beta$-D-Nal-$NH_2$ [SEQ ID N°4].

6. Procédé de préparation d'un composé de formule générale :

$$\begin{array}{l} R^1 \\ | \\ A^1 - A^2 - A^3 - \text{D-Trp} - \text{Lys} - A^6 - A^7 - A^8 - R^3 \\ | \\ R^2 \end{array}$$

où :

$A^1$ représente un isomère D de Ala, pyridyl-Ala, Leu, Ile, Val, Met, Nle, Trp, $\beta$-Nal, Phe, o-X-Phe (où X représente $CH_3$, Cl, Br, F, OH, $OCH_3$ ou $NO_2$), p-X-Phe (où X représente H, $CH_3$, Cl, Br, F, OH, $OCH_3$ ou $NO_2$), 2,4-dichloro-Phe, pentafluoro-Phe ou L-Phe;

$A^2$ représente Ala, pyridyl-Ala, Leu, Ile, Val, Met, Nle, Trp, $\beta$-Nal, Phe, o-X-Phe (où X représente $CH_3$, Cl, Br, F, OH, $OCH_3$ ou $NO_2$), p-X-Phe (où X représente H, $CH_3$, Cl, Br, F, OH, $OCH_3$ ou $NO_2$), 2,4-dichloro-Phe ou pentafluoro-Phe;

$A^3$ représente Ala, pyridyl-Ala, Leu, Ile, Val, Met, Nle, Trp, Tyr, $\beta$-Nal, Phe, o-X-Phe (où X représente H, $CH_3$, Cl, Br, F, OH, $OCH_3$ ou $NO_2$), p-X-Phe (où X représente H, $CH_3$, Cl, Br, F, OH, $OCH_3$ ou $NO_2$), 2,4-dichloro-Phe ou pentafluoro-Phe;

$A^6$ représente Ala, pyridyl-Ala, Leu, Ile, Val, Lys, Met, Nle, Thr-$R^4$, Trp, Ser-$R^4$, $\beta$-Nal, o-X-Phe (où X représente $CH_3$, Cl, Br, F, OH, $OCH_3$ ou $NO_2$), p-X-Phe (où X représente $CH_3$, Cl, Br, F, OH, $OCH_3$ ou $NO_2$), 2,4-dichloro-Phe ou pentafluoro-Phe;

$A^7$ représente Ala, pyridyl-Ala, Leu, Ile, Val, Met, Nle, Trp, $\beta$-Nal, Phe, o-X-Phe (où X représente $CH_3$, Cl, Br, F, OH, $OCH_3$ ou $NO_2$), p-X-Phe (où X représente H, $CH_3$, Cl, Br, F, OH, $OCH_3$ ou $NO_2$), 2,4-dichloro-Phe ou pentafluoro-Phe;

$A^8$ représente un isomère D ou L de Ala, pyridyl-Ala, Leu, Ile, Ser-$R^4$, Thr-$R^4$, Val, Met, Nle, Trp, $\beta$-Nal, o-X-Phe (où X représente $CH_3$, Cl, Br, F, OH, $OCH_3$ ou $NO_2$), p-X-Phe (où X représente $CH_3$, Cl, Br, F, OH, $OCH_3$ ou $NO_2$), 2,4-dichloro-Phe ou pentafluoro-Phe;

chaque $R^1$ et $R^2$ représente indépendamment un atome d'hydrogène, un acyle inférieur ou un alcoyle inférieur;

$R^3$ représente un atome d'hydrogène, un $NH_2$ ou un radical alcoyle inférieur;

pourvu qu'au moins un des $A^1$ et $A^8$ soit un acide aminé aromatique et, en outre, pourvu que si $A^2$ ou $A^7$ représente un acide aminé aromatique, alors $A^8$ ne peut pas être un acide aminé aromatique;

$R^4$ représente rien, un résidu glycosyle protégé ou un hydrate de carbone;

ou un sel pharmaceutiquement acceptable de celui-ci,

le procédé comprenant la réaction d'un composé de formule générale :

$$\begin{array}{l} \quad\; R^1 \\ \quad\;\; | \\ R^6 - A^1 - A^2 - A^3 - \text{D-Trp} - \text{N-benzyloxycarbonyl-Lys} - A^6 - \\ \quad\;\; | \\ \quad\; R^2 \qquad\qquad\qquad\qquad\qquad\qquad\qquad\qquad\qquad A^7 - A^8 - R^7 \end{array}$$

où :

$R^1$, $R^2$, $A^1$, $A^2$, $A^7$ et $A^8$ sont tels que définis précédemment;

$A^3$ est tel que défini précédemment sauf que Tyr est remplacé par o-benzyl-Tyr;

$A^6$ est tel que défini précédemment sauf que Lys est remplacé par N-benzyloxycarbonyl-Lys;

$R^4$ peut, en outre, représenter un radical o-benzyle;

$R^6$ représente un groupe protecteur, et

$R^7$ représente une résine,

avec :

du crésol;

du dithiothréitol, et

du fluorure d'hydrogène.

**7.** Procédé de préparation d'un composé de formule générale :

$$R^6 - \underset{\substack{| \\ R^2}}{\overset{\substack{R^1 \\ |}}{A^1}} - A^2 - A^3 - \text{D-Trp} - \text{N-benzyloxycarbonyl-Lys} - A^6 - A^7 - A^8 - R^7$$

où :

$A^1$ représente un isomère D de Ala, pyridyl-Ala, Leu, Ile, Val, Met, Nle, Trp, $\beta$-Nal, Phe, o-X-Phe (où X représente $CH_3$, Cl, Br, F, OH, $OCH_3$ ou $NO_2$), p-X-Phe (où X représente H, $CH_3$, Cl, Br, F, OH, $OCH_3$ ou $NO_2$), 2,4-dichloro-Phe, pentafluoro-Phe ou L-Phe;

$A^2$ représente Ala, pyridyl-Ala, Leu, Ile, Val, Met, Nle, Trp, $\beta$-Nal, Phe, o-X-Phe (où X représente $CH_3$, Cl, Br, F, OH, $OCH_3$ ou $NO_2$), p-X-Phe (où X représente H, $CH_3$, Cl, Br, F, OH, $OCH_3$ ou $NO_2$), 2,4-dichloro-Phe ou pentafluoro-Phe;

$A^3$ représente Ala, pyridyl-Ala, Leu, Ile, Val, Met, Nle, Trp, o-benzyl-Tyr, $\beta$-Nal, Phe, o-X-Phe (où X représente H, $CH_3$, Cl, Br, F, OH, $OCH_3$ ou $NO_2$), p-X-Phe (où X représente H, $CH_3$, Cl, Br, F, OH, $OCH_3$ ou $NO_2$), 2,4-dichloro-Phe ou pentafluoro-Phe;

$A^6$ représente Ala, pyridyl-Ala, Leu, Ile, Val, N-benzyloxycarbonyl-Lys, Met, Nle, Thr-$R^4$, Trp, Ser-$R^4$, $\beta$-Nal, o-X-Phe (où X représente $CH_3$, Cl, Br, F, OH, $OCH_3$ ou $NO_2$), p-X-Phe (où X représente $CH_3$, Cl, Br, F, OH, $OCH_3$ ou $NO_2$), 2,4-dichloro-Phe ou pentafluoro-Phe;

$A^7$ représente Ala, pyridyl-Ala, Leu, Ile, Val, Met, Nle, Trp, $\beta$-Nal, Phe, o-X-Phe (où X représente $CH_3$, Cl, Br, F, OH, $OCH_3$ ou $NO_2$), p-X-Phe (où X représente H, $CH_3$, Cl, Br, F, OH, $OCH_3$ ou $NO_2$), 2,4-dichloro-Phe ou pentafluoro-Phe;

$A^8$ représente un isomère D ou L de Ala, pyridyl-Ala, Leu, Ile, Ser-$R^4$, Thr-$R^4$, Val, Met, Nle, Trp, $\beta$-Nal, o-X-Phe (où X représente $CH_3$, Cl, Br, F, OH, $OCH_3$ ou $NO_2$), p-X-Phe (où X représente $CH_3$, Cl, Br, F, OH, $OCH_3$ ou $NO_2$), 2,4-dichloro-Phe ou pentafluoro-Phe;

chaque $R^1$ et $R^2$ représente indépendamment un atome d'hydrogène, un acyle inférieur ou un alcoyle inférieur;

pourvu qu'au moins un des $A^1$ et $A^8$ soit un acide aminé aromatique et, en outre, pourvu que si $A^2$ ou $A^7$ représente un acide aminé aromatique, alors $A^8$ ne peut pas être un acide aminé aromatique;

$R^4$ représente rien, un résidu glycosyle protégé, un radical o-benzyle ou un hydrate de carbone;

$R^6$ représente un groupe protecteur, par exemple, Boc;

$R^7$ représente une résine,

ou un sel pharmaceutiquement acceptable de celui-ci,

le procédé comprenant :

a) la réaction d'une résine avec un résidu acide aminé ayant une chaîne latérale $A^8$ et un groupe protecteur;

b) la réaction du composé lié à la résine résultant avec un résidu acide aminé de chaîne latérale représentée par $A^7$;

c) la répétition de b) avec les acides aminés de chaîne latérale N-benzyloxycarbonyl-Lys, D-Trp, $A^3$, $A^2$ et $A^1$, de manière consécutive, et

d) si nécessaire et là où il est nécessaire, la réaction du composé préparé ou de l'un des résidus ci-dessus avec un résidu glycosyle, un hydrate de carbone, un agent acylant ou un agent alcoylant.

8. Utilisation d'un composé de formule générale :

$$\begin{array}{l} R^1 \\ | \\ A^1 - A^2 - A^3 - \text{D-Trp} - \text{Lys} - A^6 - A^7 - A^8 - R^3 \\ | \\ R^2 \end{array}$$

où :

$A^1$ représente un isomère D de Ala, pyridyl-Ala, Leu, Ile, Val, Met, Nle, Trp, $\beta$-Nal, Phe, o-X-Phe (où X représente $CH_3$, Cl, Br, F, OH, $OCH_3$ ou $NO_2$), p-X-Phe (où X représente H, $CH_3$, Cl, Br, F, OH, $OCH_3$ ou $NO_2$), 2,4-dichloro-Phe, pentafluoro-Phe ou L-Phe;

$A^2$ représente Ala, pyridyl-Ala, Leu, Ile, Val, Met, Nle, Trp, $\beta$-Nal, Phe, o-X-Phe (où X représente $CH_3$, Cl, Br, F, OH, $OCH_3$ ou $NO_2$), p-X-Phe (où X représente H, $CH_3$, Cl, Br, F, OH, $OCH_3$ ou $NO_2$), 2,4-dichloro-Phe ou pentafluoro-Phe;

$A^3$ représente Ala, pyridyl-Ala, Leu, Ile, Val, Met, Nle, Trp, Tyr, $\beta$-Nal, Phe, o-X-Phe (où X représente H, $CH_3$, Cl, Br, F, OH, $OCH_3$ ou $NO_2$), p-X-Phe (où X représente H, $CH_3$, Cl, Br, F, OH, $OCH_3$ ou $NO_2$), 2,4-dichloro-Phe ou pentafluoro-Phe;

$A^6$ représente Ala, pyridyl-Ala, Leu, Ile, Val, Lys, Met, Nle, Thr-$R^4$, Trp, Ser-$R^4$, $\beta$-Nal, o-X-Phe (où X représente $CH_3$, Cl, Br, F, OH, $OCH_3$ ou $NO_2$), p-X-Phe (où X représente $CH_3$, Cl, Br, F, OH, $OCH_3$ ou $NO_2$), 2,4-dichloro-Phe ou pentafluoro-Phe;

$A^7$ représente Ala, pyridyl-Ala, Leu, Ile, Val, Met, Nle, Trp, $\beta$-Nal, Phe, o-X-Phe (où X représente $CH_3$, Cl, Br, F, OH, $OCH_3$ ou $NO_2$), p-X-Phe (où X représente H, $CH_3$, Cl, Br, F, OH, $OCH_3$ ou $NO_2$), 2,4-dichloro-Phe ou pentafluoro-Phe;

$A^8$ représente un isomère D ou L de Ala, pyridyl-Ala, Leu, Ile, Ser-$R^4$, Thr-$R^4$, Val, Met, Nle, Trp, $\beta$-Nal, o-X-Phe (où X représente $CH_3$, Cl, Br, F, OH, $OCH_3$ ou $NO_2$), p-X-Phe (où X représente $CH_3$, Cl, Br, F, OH, $OCH_3$ ou $NO_2$), 2,4-dichloro-Phe ou pentafluoro-Phe;

chaque $R^1$ et $R^2$ représente indépendamment un atome d'hydrogène, un acyle inférieur ou un alcoyle inférieur;

$R^3$ représente un atome d'hydrogène, un $NH_2$ ou un radical alcoyle inférieur;

pourvu qu'au moins un des $A^1$ et $A^8$ soit un acide aminé aromatique et, en outre, pourvu que si $A^2$ ou $A^7$ représente un acide aminé aromatique, alors $A^8$ ne peut pas être un acide aminé aromatique;

$R^4$ représente rien, un résidu glycosyle protégé ou un hydrate de carbone;

ou un sel pharmaceutiquement acceptable de celui-ci,

dans la préparation d'un agent dans le traitement ou la prophylaxie du cancer, de l'acromégalie, de désordres endocriniens hyposécrétoires, de diabètes, de cirrhoses, d'ulcères, de pancréatites, de diarrhées, d'hépatites, de maladie d'Alzheimer, d'empoisonnement par un champignon ou de rétinopathie.

9. Procédé de préparation d'une composition pharmaceutique, le procédé comprenant le mélange d'un composé de formule générale :

$$\begin{array}{l} R^1 \\ | \\ A^1 - A^2 - A^3 - \text{D-Trp} - \text{Lys} - A^6 - A^7 - A^8 - R^3 \\ | \\ R^2 \end{array}$$

où :

$A^1$ représente un isomère D de Ala, pyridyl-Ala, Leu, Ile, Val, Met, Nle, Trp, $\beta$-Nal, Phe, o-X-Phe (où X représente $CH_3$, Cl, Br, F, OH, $OCH_3$ ou $NO_2$), p-X-Phe (où X représente H, $CH_3$, Cl, Br, F, OH, $OCH_3$ ou $NO_2$), 2,4-dichloro-Phe, pentafluoro-Phe ou L-Phe;

$A^2$ représente Ala, pyridyl-Ala, Leu, Ile, Val, Met, Nle, Trp, $\beta$-Nal, Phe, o-X-Phe (où X représente $CH_3$, Cl, Br, F, OH, $OCH_3$ ou $NO_2$), p-X-Phe (où X représente H, $CH_3$, Cl, Br, F, OH, $OCH_3$ ou $NO_2$), 2,4-dichloro-Phe ou pentafluoro-Phe;

$A^3$ représente Ala, pyridyl-Ala, Leu, Ile, Val, Met, Nle, Trp, Tyr, $\beta$-Nal, Phe, o-X-Phe (où X

représente H, $CH_3$, Cl, Br, F, OH, $OCH_3$ ou $NO_2$), p-X-Phe (où X représente H, $CH_3$, Cl, Br, F, OH, $OCH_3$ ou $NO_2$), 2,4-dichloro-Phe ou pentafluoro-Phe;

$A^6$ représente Ala, pyridyl-Ala, Leu, Ile, Val, Lys, Met, Nle, Thr-$R^4$, Trp, Ser-$R^4$, $\beta$-Nal, o-X-Phe (où X représente $CH_3$, Cl, Br, F, OH, $OCH_3$ ou $NO_2$), p-X-Phe (où X représente $CH_3$, Cl, Br, F, OH, $OCH_3$ ou $NO_2$), 2,4-dichloro-Phe ou pentafluoro-Phe;

$A^7$ représente Ala, pyridyl-Ala, Leu, Ile, Val, Met, Nle, Trp, $\beta$-Nal, Phe, o-X-Phe (où X représente $CH_3$, Cl, Br, F, OH, $OCH_3$ ou $NO_2$), p-X-Phe (où X représente H, $CH_3$, Cl, Br, F, OH, $OCH_3$ ou $NO_2$), 2,4-dichloro-Phe ou pentafluoro-Phe;

$A^8$ représente un isomère D ou L de Ala, pyridyl-Ala, Leu, Ile, Ser-$R^4$, Thr-$R^4$, Val, Met, Nle, Trp, $\beta$-Nal, o-X-Phe (où X représente $CH_3$, Cl, Br, F, OH, $OCH_3$ ou $NO_2$), p-X-Phe (où X représente $CH_3$, Cl, Br, F, OH, $OCH_3$ ou $NO_2$), 2,4-dichloro-Phe ou pentafluoro-Phe;

chaque $R^1$ et $R^2$ représente indépendamment un atome d'hydrogène, un acyle inférieur ou un alcoyle inférieur;

$R^3$ représente un atome d'hydrogène, un $NH_2$ ou un radical alcoyle inférieur;

pourvu qu'au moins un des $A^1$ et $A^8$ soit un acide aminé aromatique et, en outre, pourvu que si $A^2$ ou $A^7$ représente un acide aminé aromatique, alors $A^8$ ne peut pas être un acide aminé aromatique;

$R^4$ représente rien, un résidu glycosyle protégé ou un hydrate de carbone;

ou un sel pharmaceutiquement acceptable de celui-ci,

et d'un excipient pharmaceutiquement acceptable.

**10.** Procédé suivant la revendication 9, dans lequel la composition est enrobée d'une substance capable de protéger la composition de l'acide gastrique dans l'estomac, pendant une période suffisante pour permettre à la composition de passer non désintégrée dans l'intestin grêle.

**Revendications pour l'Etat contractant suivant : GR**

**1.** Procédé de préparation d'un composé de formule générale :

$$\begin{array}{l} R^1 \\ | \\ A^1 - A^2 - A^3 - \text{D-Trp} - \text{Lys} - A^6 - A^7 - A^8 - R^3 \\ | \\ R^2 \end{array}$$

où :

$A^1$ représente un isomère D de Ala, pyridyl-Ala, Leu, Ile, Val, Met, Nle, Trp, $\beta$-Nal, Phe, o-X-Phe (où X représente $CH_3$, Cl, Br, F, OH, $OCH_3$ ou $NO_2$), p-X-Phe (où X représente H, $CH_3$, Cl, Br, F, OH, $OCH_3$ ou $NO_2$), 2,4-dichloro-Phe, pentafluoro-Phe ou L-Phe;

$A^2$ représente Ala, pyridyl-Ala, Leu, Ile, Val, Met, Nle, Trp, $\beta$-Nal, Phe, o-X-Phe (où X représente $CH_3$, Cl, Br, F, OH, $OCH_3$ ou $NO_2$), p-X-Phe (où X représente H, $CH_3$, Cl, Br, F, OH, $OCH_3$ ou $NO_2$), 2,4-dichloro-Phe ou pentafluoro-Phe;

$A^3$ représente Ala, pyridyl-Ala, Leu, Ile, Val, Met, Nle, Trp, Tyr, $\beta$-Nal, Phe, o-X-Phe (où X représente H, $CH_3$, Cl, Br, F, OH, $OCH_3$ ou $NO_2$), p-X-Phe (où X représente H, $CH_3$, Cl, Br, F, OH, $OCH_3$ ou $NO_2$), 2,4-dichloro-Phe ou pentafluoro-Phe;

$A^6$ représente Ala, pyridyl-Ala, Leu, Ile, Val, Lys, Met, Nle, Thr-$R^4$, Trp, Ser-$R^4$, $\beta$-Nal, o-X-Phe (où X représente $CH_3$, Cl, Br, F, OH, $OCH_3$ ou $NO_2$), p-X-Phe (où X représente $CH_3$, Cl, Br, F, OH, $OCH_3$ ou $NO_2$), 2,4-dichloro-Phe ou pentafluoro-Phe;

$A^7$ représente Ala, pyridyl-Ala, Leu, Ile, Val, Met, Nle, Trp, $\beta$-Nal, Phe, o-X-Phe (où X représente $CH_3$, Cl, Br, F, OH, $OCH_3$ ou $NO_2$), p-X-Phe (où X représente H, $CH_3$, Cl, Br, F, OH, $OCH_3$ ou $NO_2$), 2,4-dichloro-Phe ou pentafluoro-Phe;

$A^8$ représente un isomère D ou L de Ala, pyridyl-Ala, Leu, Ile, Ser-$R^4$, Thr-$R^4$, Val, Met, Nle, Trp, $\beta$-Nal, o-X-Phe (où X représente $CH_3$, Cl, Br, F, OH, $OCH_3$ ou $NO_2$), p-X-Phe (où X représente $CH_3$, Cl, Br, F, OH, $OCH_3$ ou $NO_2$), 2,4-dichloro-Phe ou pentafluoro-Phe;

chaque $R^1$ et $R^2$ représente indépendamment un atome d'hydrogène, un acyle inférieur ou un alcoyle inférieur;

$R^3$ représente un atome d'hydrogène, un $NH_2$ ou un radical alcoyle inférieur;

pourvu qu'au moins un des $A^1$ et $A^8$ soit un acide aminé aromatique et, en outre, pourvu que si $A^2$

ou $A^7$ représente un acide aminé aromatique, alors $A^8$ ne peut pas être un acide aminé aromatique;
$R^4$ représente rien, un résidu glycosyle protégé ou un hydrate de carbone;
ou un sel pharmaceutiquement acceptable de celui-ci,
le procédé comprenant le couplage successif de résidus acide aminé les uns aux autres.

**2.** Procédé suivant la revendication 1, dans lequel un, mais pas les deux, de $A^1$ et $A^2$, représente un acide aminé aromatique, et dans lequel un, mais pas les deux, de $A^7$ et $A^8$, représente un acide aminé aromatique.

**3.** Procédé suivant la revendication 1 ou 2, dans lequel :
$A^1$ représente un isomère D de Trp, β-Nal, o-X-Phe (où X représente $CH_3$ ou $OCH_3$), p-X-Phe (où X représente $CH_3$ ou $OCH_3$), et
$A^8$ représente un isomère D ou L de Ala, pyridyl-Ala, Leu, Ile, Ser, Thr, Val, Met, Nle, o-X-Phe (où X représente Cl, Br, F, OH ou $NO_2$), p-X-Phe (où X représente Cl, Br, F, OH ou $NO_2$), 2,4-dichloro-Phe ou pentafluoro-Phe.

**4.** Procédé suivant l'une quelconque des revendications 1 à 3, qui présente une, quelques-unes ou toutes les caractéristiques suivantes :
$A^1$ représente β-D-Nal ou D-Phe;
$A^2$ représente Ala, Phe ou p-chloro-Phe;
$A^3$ représente Tyr ou Phe;
$A^6$ représente Val, Lys ou Thr;
$A^7$ représente Ala ou Phe, et/ou
$A^8$ représente Thr ou β-D-Nal.

**5.** Procédé suivant l'une quelconque des revendications 1 à 4, dans lequel le composé est :
D-Phe-Phe-Phe-D-Trp-Lys-Thr-Phe-Thr-$NH_2$ [SEQ ID N°3];
D-Phe-Phe-Tyr-D-Trp-Lys-Val-Phe-Thr-$NH_2$ [SEQ ID N°2];
D-Phe-p-chloro-Phe-Tyr-D-Trp-Lys-Val-Phe-Thr-$NH_2$ [SEQ ID N°1], ou
D-Phe-Ala-Tyr-D-Trp-Lys-Val-Ala-β-D-Nal-$NH_2$ [SEQ ID N°4].

**6.** Procédé de préparation d'un composé de formule générale :

$$\begin{array}{l} R^1 \\ | \\ A^1 - A^2 - A^3 - \text{D-Trp} - \text{Lys} - A^6 - A^7 - A^8 - R^3 \\ | \\ R^2 \end{array}$$

où :
$A^1$ représente un isomère D de Ala, pyridyl-Ala, Leu, Ile, Val, Met, Nle, Trp, β-Nal, Phe, o-X-Phe (où X représente $CH_3$, Cl, Br, F, OH, $OCH_3$ ou $NO_2$), p-X-Phe (où X représente H, $CH_3$, Cl, Br, F, OH, $OCH_3$ ou $NO_2$), 2,4-dichloro-Phe, pentafluoro-Phe ou L-Phe;
$A^2$ représente Ala, pyridyl-Ala, Leu, Ile, Val, Met, Nle, Trp, β-Nal, Phe, o-X-Phe (où X représente $CH_3$, Cl, Br, F, OH, $OCH_3$ ou $NO_2$), p-X-Phe (où X représente H, $CH_3$, Cl, Br, F, OH, $OCH_3$ ou $NO_2$), 2,4-dichloro-Phe ou pentafluoro-Phe;
$A^3$ représente Ala, pyridyl-Ala, Leu, Ile, Val, Met, Nle, Trp, Tyr, β-Nal, Phe, o-X-Phe (où X représente H, $CH_3$, Cl, Br, F, OH, $OCH_3$ ou $NO_2$), p-X-Phe (où X représente H, $CH_3$, Cl, Br, F, OH, $OCH_3$ ou $NO_2$), 2,4-dichloro-Phe ou pentafluoro-Phe;
$A^6$ représente Ala, pyridyl-Ala, Leu, Ile, Val, Lys, Met, Nle, Thr-$R^4$, Trp, Ser-$R^4$, β-Nal, o-X-Phe (où X représente $CH_3$, Cl, Br, F, OH, $OCH_3$ ou $NO_2$), p-X-Phe (où X représente $CH_3$, Cl, Br, F, OH, $OCH_3$ ou $NO_2$), 2,4-dichloro-Phe ou pentafluoro-Phe;
$A^7$ représente Ala, pyridyl-Ala, Leu, Ile, Val, Met, Nle, Trp, β-Nal, Phe, o-X-Phe (où X représente $CH_3$, Cl, Br, F, OH, $OCH_3$ ou $NO_2$), p-X-Phe (où X représente H, $CH_3$, Cl, Br, F, OH, $OCH_3$ ou $NO_2$), 2,4-dichloro-Phe ou pentafluoro-Phe;
$A^8$ représente un isomère D ou L de Ala, pyridyl-Ala, Leu, Ile, Ser-$R^4$, Thr-$R^4$, Val, Met, Nle, Trp, β-Nal, o-X-Phe (où X représente $CH_3$, Cl, Br, F, OH, $OCH_3$ ou $NO_2$), p-X-Phe (où X représente $CH_3$, Cl,

Br, F, OH, $OCH_3$ ou $NO_2$), 2,4-dichloro-Phe ou pentafluoro-Phe;

chaque $R^1$ et $R^2$ représente indépendamment un atome d'hydrogène, un acyle inférieur ou un alcoyle inférieur;

$R^3$ représente un atome d'hydrogène, un $NH_2$ ou un radical alcoyle inférieur;

pourvu qu'au moins un des $A^1$ et $A^8$ soit un acide aminé aromatique et, en outre, pourvu que si $A^2$ ou $A^7$ représente un acide aminé aromatique, alors $A^8$ ne peut pas être un acide aminé aromatique;

$R^4$ représente rien, un résidu glycosyle protégé ou un hydrate de carbone;

ou un sel pharmaceutiquement acceptable de celui-ci,

le procédé comprenant la réaction d'un composé de formule générale :

$$R^6 - \underset{\underset{R^2}{|}}{\overset{\overset{R^1}{|}}{A^1}} - A^2 - A^3 - \text{D-Trp} - \text{N-benzyloxycarbonyl-Lys} - A^6 - A^7 - A^8 - R^7$$

où :

$R^1$, $R^2$, $A^1$, $A^2$, $A^7$ et $A^8$ sont tels que définis précédemment;

$A^3$ est tel que défini précédemment sauf que Tyr est remplacé par o-benzyl-Tyr;

$A^6$ est tel que défini précédemment sauf que Lys est remplacé par N-benzyloxycarbonyl-Lys;

$R^4$ peut, en outre, représenter un radical o-benzyle;

$R^6$ représente un groupe protecteur, et

$R^7$ représente une résine,

avec :

du crésol;

du dithiothréitol, et

du fluorure d'hydrogène.

**7.** Composé de formule générale :

$$R^6 - \underset{\underset{R^2}{|}}{\overset{\overset{R^1}{|}}{A^1}} - A^2 - A^3 - \text{D-Trp} - \text{N-benzyloxycarbonyl-Lys} - A^6 - A^7 - A^8 - R^7$$

où :

$A^1$ représente un isomère D de Ala, pyridyl-Ala, Leu, Ile, Val, Met, Nle, Trp, $\beta$-Nal, Phe, o-X-Phe (où X représente $CH_3$, Cl, Br, F, OH, $OCH_3$ ou $NO_2$), p-X-Phe (où X représente H, $CH_3$, Cl, Br, F, OH, $OCH_3$ ou $NO_2$), 2,4-dichloro-Phe, pentafluoro-Phe ou L-Phe;

$A^2$ représente Ala, pyridyl-Ala, Leu, Ile, Val, Met, Nle, Trp, $\beta$-Nal, Phe, o-X-Phe (où X représente $CH_3$, Cl, Br, F, OH, $OCH_3$ ou $NO_2$), p-X-Phe (où X représente H, $CH_3$, Cl, Br, F, OH, $OCH_3$ ou $NO_2$), 2,4-dichloro-Phe ou pentafluoro-Phe;

$A^3$ représente Ala, pyridyl-Ala, Leu, Ile, Val, Met, Nle, Trp, o-benzyl-Tyr, $\beta$-Nal, Phe, o-X-Phe (où X représente H, $CH_3$, Cl, Br, F, OH, $OCH_3$ ou $NO_2$), p-X-Phe (où X représente H, $CH_3$, Cl, Br, F, OH, $OCH_3$ ou $NO_2$), 2,4-dichloro-Phe ou pentafluoro-Phe;

$A^6$ représente Ala, pyridyl-Ala, Leu, Ile, Val, N-benzyloxycarbonyl-Lys, Met, Nle, Thr-$R^4$, Trp, Ser-$R^4$, $\beta$-Nal, o-X-Phe (où X représente $CH_3$, Cl, Br, F, OH, $OCH_3$ ou $NO_2$), p-X-Phe (où X représente $CH_3$, Cl, Br, F, OH, $OCH_3$ ou $NO_2$), 2,4-dichloro-Phe ou pentafluoro-Phe;

$A^7$ représente Ala, pyridyl-Ala, Leu, Ile, Val, Met, Nle, Trp, $\beta$-Nal, Phe, o-X-Phe (où X représente $CH_3$, Cl, Br, F, OH, $OCH_3$ ou $NO_2$), p-X-Phe (où X représente H, $CH_3$, Cl, Br, F, OH, $OCH_3$ ou $NO_2$), 2,4-dichloro-Phe ou pentafluoro-Phe;

$A^8$ représente un isomère D ou L de Ala, pyridyl-Ala, Leu, Ile, Ser-$R^4$, Thr-$R^4$, Val, Met, Nle, Trp, $\beta$-Nal, o-X-Phe (où X représente $CH_3$, Cl, Br, F, OH, $OCH_3$ ou $NO_2$), p-X-Phe (où X représente $CH_3$, Cl, Br, F, OH, $OCH_3$ ou $NO_2$), 2,4-dichloro-Phe ou pentafluoro-Phe;

chaque $R^1$ et $R^2$ représente indépendamment un atome d'hydrogène, un acyle inférieur ou un alcoyle inférieur;

pourvu qu'au moins un des $A^1$ et $A^8$ soit un acide aminé aromatique et, en outre, pourvu que si $A^2$ ou $A^7$ représente un acide aminé aromatique, alors $A^8$ ne peut pas être un acide aminé aromatique;

$R^4$ représente rien, un résidu glycosyle protégé, un radical o-benzyle ou un hydrate de carbone;

$R^6$ représente un groupe protecteur, par exemple, Boc;

$R^7$ représente une résine,

ou un sel pharmaceutiquement acceptable de celui-ci.

**8.** Procédé de préparation d'un composé de formule générale :

$$R^6 - \underset{\displaystyle R^2}{\overset{\displaystyle R^1}{A^1}} - A^2 - A^3 - \text{D-Trp} - \text{N-benzyloxycarbonyl-Lys} - A^6 - A^7 - A^8 - R^7$$

où :

$A^1$ représente un isomère D de Ala, pyridyl-Ala, Leu, Ile, Val, Met, Nle, Trp, $\beta$-Nal, Phe, o-X-Phe (où X représente $CH_3$, Cl, Br, F, OH, $OCH_3$ ou $NO_2$), p-X-Phe (où X représente H, $CH_3$, Cl, Br, F, OH, $OCH_3$ ou $NO_2$), 2,4-dichloro-Phe, pentafluoro-Phe ou L-Phe;

$A^2$ représente Ala, pyridyl-Ala, Leu, Ile, Val, Met, Nle, Trp, $\beta$-Nal, Phe, o-X-Phe (où X représente $CH_3$, Cl, Br, F, OH, $OCH_3$ ou $NO_2$), p-X-Phe (où X représente H, $CH_3$, Cl, Br, F, OH, $OCH_3$ ou $NO_2$), 2,4-dichloro-Phe ou pentafluoro-Phe;

$A^3$ représente Ala, pyridyl-Ala, Leu, Ile, Val, Met, Nle, Trp, o-benzyl-Tyr, $\beta$-Nal, Phe, o-X-Phe (où X représente H, $CH_3$, Cl, Br, F, OH, $OCH_3$ ou $NO_2$), p-X-Phe (où X représente H, $CH_3$, Cl, Br, F, OH, $OCH_3$ ou $NO_2$), 2,4-dichloro-Phe ou pentafluoro-Phe;

$A^6$ représente Ala, pyridyl-Ala, Leu, Ile, Val, N-benzyloxycarbonyl-Lys, Met, Nle, Thr-$R^4$, Trp, Ser-$R^4$, $\beta$-Nal, o-X-Phe (où X représente $CH_3$, Cl, Br, F, OH, $OCH_3$ ou $NO_2$), p-X-Phe (où X représente $CH_3$, Cl, Br, F, OH, $OCH_3$ ou $NO_2$), 2,4-dichloro-Phe ou pentafluoro-Phe;

$A^7$ représente Ala, pyridyl-Ala, Leu, Ile, Val, Met, Nle, Trp, $\beta$-Nal, Phe, o-X-Phe (où X représente $CH_3$, Cl, Br, F, OH, $OCH_3$ ou $NO_2$), p-X-Phe (où X représente H, $CH_3$, Cl, Br, F, OH, $OCH_3$ ou $NO_2$), 2,4-dichloro-Phe ou pentafluoro-Phe;

$A^8$ représente un isomère D ou L de Ala, pyridyl-Ala, Leu, Ile, Ser-$R^4$, Thr-$R^4$, Val, Met, Nle, Trp, $\beta$-Nal, o-X-Phe (où X représente $CH_3$, Cl, Br, F, OH, $OCH_3$ ou $NO_2$), p-X-Phe (où X représente $CH_3$, Cl, Br, F, OH, $OCH_3$ ou $NO_2$), 2,4-dichloro-Phe ou pentafluoro-Phe;

chaque $R^1$ et $R^2$ représente indépendamment un atome d'hydrogène, un acyle inférieur ou un alcoyle inférieur;

pourvu qu'au moins un des $A^1$ et $A^8$ soit un acide aminé aromatique et, en outre, pourvu que si $A^2$ ou $A^7$ représente un acide aminé aromatique, alors $A^8$ ne peut pas être un acide aminé aromatique;

$R^4$ représente rien, un résidu glycosyle protégé, un radical o-benzyle ou un hydrate de carbone;

$R^6$ représente un groupe protecteur, par exemple, BOC;

$R^7$ représente une résine,

ou un sel pharmaceutiquement acceptable de celui-ci,

le procédé comprenant :

a) la réaction d'une résine avec un résidu acide aminé ayant une chaîne latérale $A^8$ et un groupe protecteur;

b) la réaction du composé lié à la résine résultant avec un résidu acide aminé de chaîne latérale représentée par $A^7$;

c) la répétition de b) avec les acides aminée de chaîne latérale N-benzyloxycarbonyl-Lys, D-Trp, $A^3$, $A^2$ et $A^1$, de manière consécutive, et

d) si nécessaire et là où il est nécessaire, la réaction du composé préparé ou de l'un des résidus ci-dessus avec un résidu glycosyle, un hydrate de carbone, un agent acylant ou un agent alcoylant.

**9.** Utilisation d'un composé de formule générale :

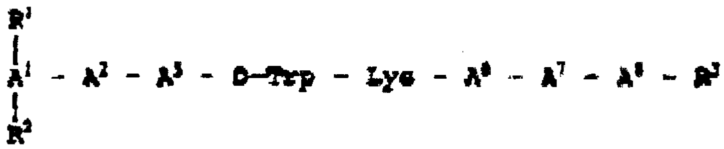

où :

$A^1$ représente un isomère D de Ala, pyridyl-Ala, Leu, Ile, Val, Met, Nle, Trp, β-Nal, Phe, o-X-Phe (où X représente $CH_3$, Cl, Br, F, OH, $OCH_3$ ou $NO_2$), p-X-Phe (où X représente H, $CH_3$, Cl, Br, F, OH, $OCH_3$ ou $NO_2$), 2,4-dichloro-Phe, pentafluoro-Phe ou L-Phe;

$A^2$ représente Ala, pyridyl-Ala, Leu, Ile, Val, Met, Nle, Trp, β-Nal, Phe, o-X-Phe (où X représente $CH_3$, Cl, Br, F, OH, $OCH_3$ ou $NO_2$), p-X-Phe (où X représente H, $CH_3$, Cl, Br, F, OH, $OCH_3$ ou $NO_2$), 2,4-dichloro-Phe ou pentafluoro-Phe;

$A^3$ représente Ala, pyridyl-Ala, Leu, Ile, Val, Met, Nle, Trp, Try, β-Nal, Phe, o-X-Phe (où X représente H, $CH_3$, Cl, Br, F, OH, $OCH_3$, ou $NO_2$), p-X-Phe (où X représente H, $CH_3$, Cl, Br, F, OH, $OCH_3$ ou $NO_2$), 2,4-dichloro-Phe ou pentafluoro-Phe;

$A^6$ représente Ala, pyridyl-Ala, Leu, Ile, Val, Lys, Met, Nle, Thr-$R^4$, Trp, Ser-$R^4$, β-Nal, o-X-Phe (où X représente $CH_3$, Cl, Br, F, OH, $OCH_3$ ou $NO_2$), p-X-Phe (où X représente $CH_3$, Cl, Br, F, OH, $OCH_3$ ou $NO_2$), 2,4-dichloro-Phe ou pentafluoro-Phe;

$A^7$ représente Ala, pyridyl-Ala, Leu, Ile, Val, Met, Nle, Trp, β-Nal, Phe, o-X-Phe (où X représente $CH_3$, Cl, Br, F, OH, $OCH_3$, ou $NO_2$), p-X-Phe (où X représente H, $CH_3$, Cl, Br, F, OH, $OCH_3$ ou $NO_2$), 2,4-dichloro-Phe ou pentafluoro-Phe;

$A^8$ représente un isomère D ou L de Ala, pyridyl-Ala, Leu, Ile, Ser-$R^4$, Thr-$R^4$, Val, Met, Nle, Trp, β-Nal, o-X-Phe (où X représente $CH_3$, Cl, Br, F, OH, $OCH_3$ ou $NO_2$), p-X-Phe (où X représente $CH_3$, Cl, Br, F, OH, $OCH_3$ ou $NO_2$), 2,4-dichloro-Phe ou pentafluoro-Phe;

chaque $R^1$ et $R^2$ représente indépendamment un atome d'hydrogène, un acyle inférieur ou un alcoyle inférieur;

$R^3$ représente un atome d'hydrogène, un $NH_2$ ou un radical alcoyle inférieur;

pourvu qu'au moine un des $A^1$ et $A^8$ soit un acide aminé aromatique et, en outre, pourvu que si $A^2$ ou $A^7$ représente un acide aminé aromatique, alors $A^8$ ne peut pas être un acide aminé aromatique;

$R^4$ représente rien, un résidu glycosyle protégé ou un hydrate de carbone;

ou un sel pharmaceutiquement acceptable de celui-ci,

dans la préparation d'un agent dans le traitement ou la prophylaxie du cancer, de l'acromégalie, de désordres endocriniens hyposécrétoires, de diabètes, de cirrhoses, d'ulcères, de pancréatites, de diarrhées, d'hépatites, de maladie d'Alzheimer, d'un empoisonnement par un champignon ou de rétinopathie.

**10.** Procédé de préparation d'une composition pharmaceutique, le procédé comprenant le mélange d'un composé de formule générale :

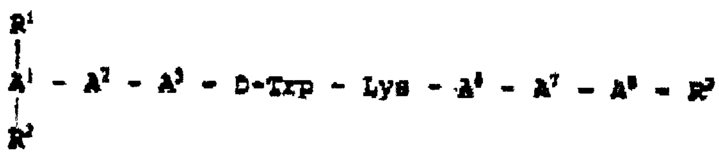

où :

$A^1$ représente un isomère D de Ala, pyridyl-Ala, Leu, Ile, Val, Met, Nle, Trp, β-Nal, Phe, o-X-Phe (où X représente $CH_3$, Cl, Br, F, OH, $OCH_3$, ou $NO_2$), p-X-Phe (où X représente H, $CH_3$, Cl, Br, F, OH, $OCH_3$ ou $NO_2$), 2,4-dichloro-Phe, pentafluoro-Phe ou L-Phe;

$A^2$ représente Ala, pyridyl-Ala, Leu, Ile, Val, Met, Nle, Trp, β-Nal, Phe, o-X-Phe (où X représente $CH_3$, Cl, Br, F, OH, $OCH_3$ ou $NO_2$), p-X-Phe (où X représente H, $CH_3$, Cl, Br, F, OH, $OCH_3$ ou $NO_2$), 2,4-dichloro-Phe ou pentafluoro-Phe;

$A^3$ représente Ala, pyridyl-Ala, Leu, Ile, Val, Met, Nle, Trp, Tyr, β-Nal, Phe, o-X-Phe (où X

représente H, $CH_3$, Cl, Br, F, OH, $OCH_3$ ou $NO_2$), p-X-Phe (où X représente H, $CH_3$, Cl, Br, F, OH, $OCH_3$ ou $NO_2$), 2,4-dichloro-Phe ou pentafluoro-Phe;

$A^6$ représente Ala, pyridyl-Ala, Leu, Ile, Val, Lys, Met, Nle, Thr-$R^4$, Trp, Ser-$R^4$, $\beta$-Nal, o-X-Phe (où X représente $CH_3$, Cl, Br, F, OH, $OCH_3$ ou $NO_2$) p-X-Phe (où X représente $CH_3$, Cl, Br, F, OH, $OCH_3$ ou $NO_2$), 2,4-dichloro-Phe ou pentafluoro-Phe;

$A^7$ représente Ala, pyridyl-Ala, Leu, Ile, Val, Met, Nle, Trp, $\beta$-Nal, Phe, o-X-Phe (où X représente $CH_3$, Cl, Br, F, OH, $OCH_3$ ou $NO_2$), p-X-Phe (où X représente H, $CH_3$, Cl, Br, F, OH, $OCH_3$ ou $NO_2$), 2,4-dichloro-Phe ou pentafluoro-Phe;

$A^8$ représente un isomère D ou L de Ala, pyridyl-Ala, Leu, Ile, Ser-$R^4$, Thr-$R^4$, Val, Met, Nle, Trp, $\beta$-Nal, o-X-Phe (où X représente $CH_3$, Cl, Br, F, OH, $OCH_3$ ou $NO_2$), p-X-Phe (où X représente $CH_3$, Cl, Br, F, OH, $OCH_3$ ou $NO_2$), 2,4-dichloro-Phe ou pentafluoro-Phe;

chaque $R^1$ et $R^2$ représente indépendamment un atome d'hydrogène, un acyle inférieur ou un alcoyle inférieur;

$R^3$ représente un atome d'hydrogène, un $NH_2$ ou un radical alcoyle inférieur;

pourvu qu'au moins un des $A^1$ et $A^8$ soit un acide aminé aromatique et, en outre, pourvu que si $A^2$ ou $A^7$ représente un acide aminé aromatique, alors $A^8$ ne peut pas être un acide aminé aromatique;

$R^4$ représente rien, un résidu glycosyle protégé ou un hydrate de carbone;

ou d'un sel pharmaceutiquement acceptable de celui-ci,

et d'un excipient pharmaceutiquement acceptable.

**11.** Procédé suivant la revendication 10, dans lequel la composition est enrobée d'une substance capable de protéger la composition de l'acide gastrique dans l'estomac, pendant une période suffisante pour permettre à la composition de passer non désintégrée dans l'intestin grêle.

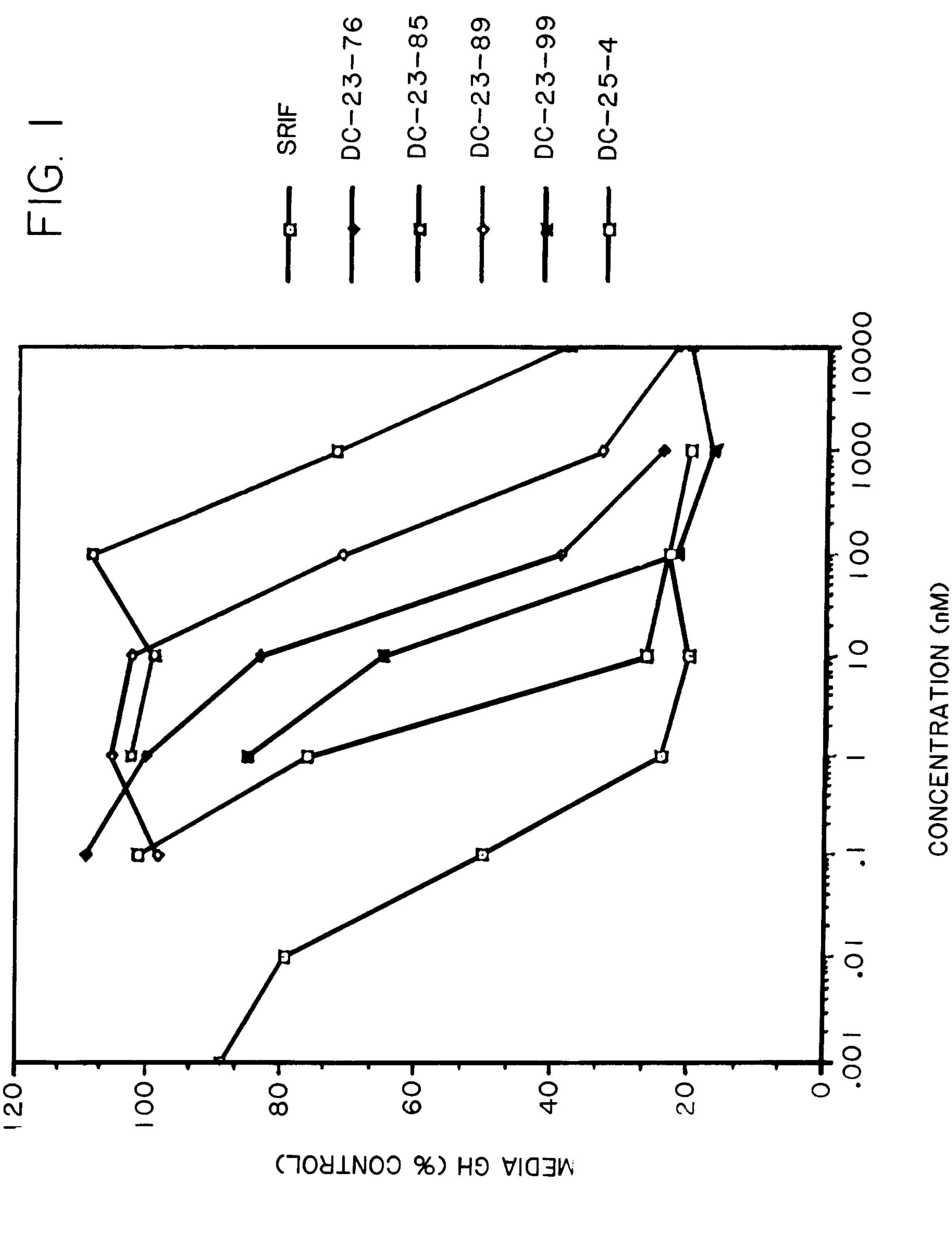
SRIF
DC-23-76
DC-23-85
DC-23-89
DC-23-99
DC-25-4
MEDIA GH (% CONTROL)
0
20
40
60
80
100
120
.001
.01
.1
1
10
100
1000
10000
CONCENTRATION (nM)

FIG. 1

FIG. 2

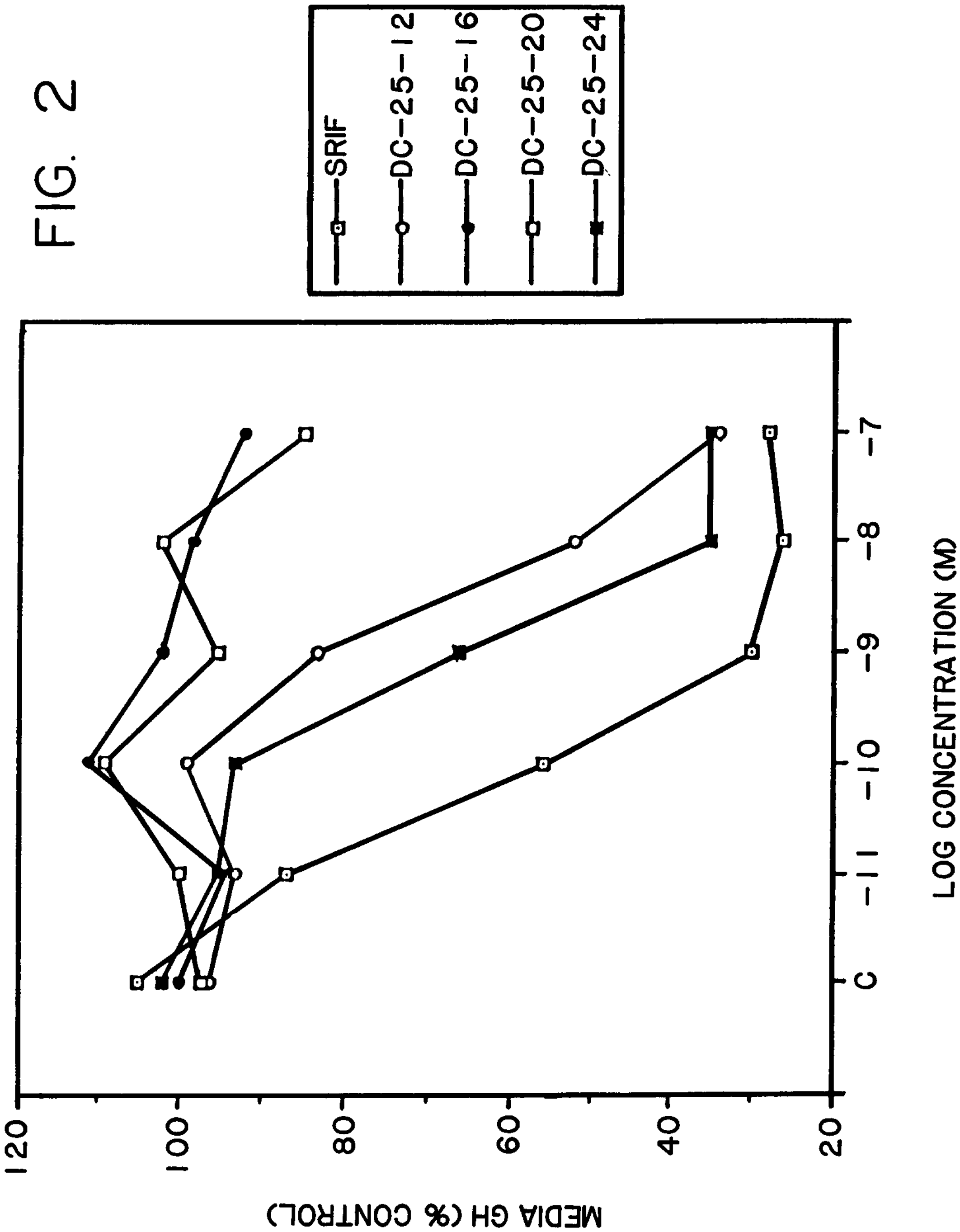
SRIF
DC-25-12
DC-25-16
DC-25-20
DC-25-24
MEDIA GH (% CONTROL)
120
100
80
60
40
20
C
-11
-10
-9
-8
-7
LOG CONCENTRATION (M)